# EUROPEAN PATENT APPLICATION

(11) **EP 4 582 423 A1**
(43) Date of publication of application: **09.07.2025**
(21) Application number: 23877711.4
(22) Date of filing: 12.10.2023
(51) Int. Cl.: C07D 471/04, A61K 31/4745, A61K 31/4375, A61P 35/00, C07D 417/14

(54) **NOVEL TRICYCLIC DERIVATIVE COMPOUND AND USES THEREOF**

(30) Priority: 13.10.2022 KR 20220131761
(71) Applicant: Hanmi Pharm. Co., Ltd., Hwaseong-si, Gyeonggi-do 18536 (KR)
(72) Inventor: HONG, Dong Jin, Hwaseong-si, Gyeonggi-do 18469 (KR); KIM, Seo Hee, Hwaseong-si, Gyeonggi-do 18469 (KR); PARK, So Min, Hwaseong-si, Gyeonggi-do 18469 (KR); KIM, Won Jeoung, Hwaseong-si, Gyeonggi-do 18469 (KR); LEE, Won Jong, Hwaseong-si, Gyeonggi-do 18469 (KR); AHN, Young Gil, Hwaseong-si, Gyeonggi-do 18469 (KR); SUH, Kwee Hyun, Hwaseong-si, Gyeonggi-do 18469 (KR)
(74) Representative: Brevalex
(86) International application number: PCT/KR2023/015752
(87) International publication number: WO 2024/080788

(57) **Abstract**

The present disclosure relates to novel tricycle derivative compounds and uses thereof, and more specifically, to novel tricycle derivative compounds having methionine adenosyltransferase 2A (MAT2A) inhibitory activity and uses thereof.

## Description

### Technical Field

The present disclosure relates to novel tricycle derivative compounds and uses thereof, and more particularly, to novel tricycle derivative compounds with methionine adenosyltransferase 2A (MAT2A) inhibitory activity and uses thereof.

### Background Art

Methionine adenosyltransferase (MAT), also known as S-adenosylmethionine synthase, is a cellular enzyme that catalyzes the synthesis of S-adenosylmethionine (SAM or AdoMet) from methionine and ATP and is thought to be the rate-limiting process in the methionine cycle. SAM is a propylamino donor in polyamine biosynthesis, a major methyl donor for DNA methylation, and is involved in gene transcription and cellular proliferation as well as the production of secondary metabolites.

Methylthioadenosine phosphorylase (MTAP) is an enzyme found in all normal tissues that catalyzes the conversion of methylthioadenosine (MTA) to adenine and 5-methylthiouribose-1-phosphate. Adenine is recovered to produce adenosine monophosphate, and 5-methylthiouribose-1-phosphate is converted to methionine and formate. Because of this recovery pathway, MTA may be provided as an alternative purine source, for example, when new purine synthesis is blocked using an antimetabolite, such as L-alanosine.

Many human and murine malignant cells lack MTAP activity. In addition to MTAP deficiency being found in tissue culture cells, such deficiency is also present in primary leukemia, glioma, melanoma, pancreatic cancer, non-small cell lung cancer (NSCLC), bladder cancer, astrocytoma, osteosarcoma, head and neck cancer, myxoid chondrosarcoma, ovarian cancer, endometrial cancer, breast cancer, soft tissue sarcoma, non-Hodgkin lymphoma, and mesothelioma.

The inventors of the present disclosure have completed the present disclosure by developing novel tricycle derivative compounds with MAT2A inhibitory activity and uses thereof.

### [PRIOR ART DOCUMENTS]

### [PATENT DOCUMENTS]

(Patent Document 1) Korean Public Patent No. 10-2022-0051301
(Patent Document 2) Korean Public Patent No. 10-2022-0050832
(Patent Document 3) Korean Public Patent No. 10-2021-0103498
(Patent Document 4) Korean Public Patent No. 10-2018-0100125
(Patent Document 5) Korean Public Patent No. 10-2019-0046921
(Patent Document 6) Korean Public Patent No. 10-2020-0138769

### Disclosure

### Technical Problem

An objective of the present disclosure is to provide a novel tricycle derivative compound.

Another objective of the present disclosure is to provide a pharmaceutical composition including the aforementioned compound as an active ingredient for treating or preventing a methionine adenosyltransferase 2A (MAT2A)-related disease or disorder.

Other objectives and advantages of the present disclosure will become apparent from the following detailed description in conjunction with the appended claims. Anything not set forth herein may be readily recognized and inferred by one of ordinary skill in the art of the present disclosure or a similar art and is hereby omitted.

### Technical Solution

An embodiment of the present disclosure provides a compound selected from compounds of Formula 1 below and enantiomers, diastereomers, solvates and hydrates thereof, and pharmaceutically acceptable salts thereof.

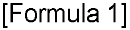

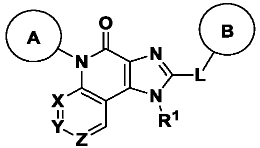

Another embodiment of the present disclosure provides a pharmaceutical composition for treating or preventing a methionine adenosyltransferase 2A (MAT2A)-related disease or disorder, the pharmaceutical composition including, as an active ingredient, the compound selected from the compounds of Formula 1 and enantiomers, diastereomers, solvates and hydrates thereof, and pharmaceutically acceptable salts thereof.

### Advantageous Effects

A novel tricycle derivative compound, which has a structure of Formula 1 according to an embodiment of the present disclosure, and a pharmaceutical composition, which includes the same, are effective against a methionine adenosyltransferase 2A (MAT2A)-related disease or disorder, and may be useful as a therapeutic agent.

### Mode for Invention

The following describes the present disclosure in more detail.

All technical terms used in the present disclosure, unless otherwise defined, are used as commonly understood by one of ordinary skill in the relevant field of the present disclosure. In addition, while suitable methods or samples are described herein, similar or equivalent methods are also included within the scope of the present disclosure.

An embodiment of the present disclosure provides a compound selected from compounds of Formula 1 below and enantiomers, diastereomers, solvates and hydrates thereof, and pharmaceutically acceptable salts thereof. 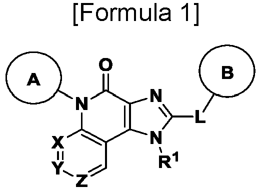
wherein, in Formula 1 above,
X and Z may each independently be N or CH; and
Y may be CR³; and
R³ may be H, alkyl, alkenyl, alkynyl, alkoxy, alkylthio, alkylsulfonyl, hydroxy, halogen, haloalkyl, haloalkoxy, cycloalkyl, cyano, amino, alkylamino, haloalkylamino, dialkylamino, alkylcarbonyl, alkoxycarbonyl, aminocarbonyl, alkylaminocarbonyl, dialkylaminocarbonyl, hydroxyalkyl, hydroxyalkoxy, hydroxyalkylamino, alkoxyalkyl, alkoxyalkoxy, alkoxyalkylamino, aminoalkyl, aminoalkoxy, aminoalkylamino, aryl, heteroaryl, heteroaryloxy, heteroarylamino, heterocyclyl, heterocyclyloxy, heterocyclylamino, heterocyclyloxyalkoxy, or heterocyclyloxyalkylamino,
wherein each of the aryl, heteroaryl, or heterocyclyl, may be
unsubstituted or substituted with 1 to 3 members selected from alkyl, cycloalkyl, haloalkyl, haloalkoxy, alkoxy, hydroxy, halo, cyano, hydroxyalkyl, alkoxyalkyl, and aminoalkyl; 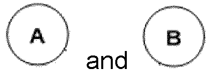
may each independently be H, cycloalkyl, aryl, heteroaryl, or heterocyclyl,
each of the cycloalkyl, aryl, heteroaryl, or heterocyclyl may be monocyclic, bicyclic, or polycyclic,
wherein the cycloalkyl, aryl, heteroaryl, or heterocyclyl may be unsubstituted or substituted with 1 to 3 members independently selected from R² below;
L may be C₁₋₃alkylene, O, NH, N(C₁₋₃alkyl), S, SO, SO₂, or a bond;
R² may be alkyl, cycloalkyl, haloalkyl, haloalkoxy, alkoxy, hydroxy, alkylsulfonyl, halogen, cyano, carboxy, alkoxycarbonyl, hydroxyalkyl, alkoxyalkyl, aminoalkyl, aminosulfonyl, alkylaminosulfonyl, dialkylaminosulfonyl, sulfonylamino, aminocarbonyl, alkylaminocarbonyl, dialkylaminocarbonyl, heterocyclylcarbonyl, or ureido; and
R¹ may be H, alkyl, haloalkyl, hydroxyalkyl, alkoxyalkyl, aminoalkyl, substituted aminoalkyl, aminocarbonylalkyl, or aminosulfonylalkyl.

### DEFINITION

As used herein, the term "alkyl" refers to a straight-chain or branched hydrocarbon residue, which may be substituted or unsubstituted, unless otherwise noted. For example, the alkyl may be a linear saturated monovalent hydrocarbon radical of 1 to 6 carbon atoms or a branched saturated monovalent hydrocarbon radical of 3 to 6 carbon atoms. For example, the alkyl may be, but not limited to, methyl, ethyl, propyl, butyl, pentyl, or isopropyl, isobutyl, or t-butyl, etc. It is recognized by those skilled in the art that the term "alkyl" may include "alkylene".

As used herein, the term "alkylene" refers to a straight-chain or branched hydrocarbon residue which may be substituted or unsubstituted, unless otherwise noted. For example, the alkylene may be a linear saturated divalent hydrocarbon radical of 1 to 6 carbon atoms or a branched saturated divalent hydrocarbon radical of 3 to 6 carbon atoms. For example, the alkylene may be, but not limited to, methylene, ethylene, propylene, 1-methylpropylene, 2-methylpropylene, butylene, pentylene, etc. For example, C₁₋₃alkylene may include a form in which any one hydrogen is substituted or unsubstituted, such as a form in which any one hydrogen is unsubstituted or substituted with C₁₋₃alkyl.

As used herein, the term "alkenyl" refers to an alkyl group containing one or more double bonds, which may be substituted or unsubstituted, unless otherwise noted. For example, the alkenyl may be, but not limited to, prop-1-ene, but-1-ene, but-2-ene, 3-methylbut-1-ene, or pent-1-ene, etc.

As used herein, the term "alkynyl" refers to an alkyl group containing one or more triple bonds, which may be substituted or unsubstituted, unless otherwise noted. For example, the alkynyl may be, but not limited to, ethynyl, propynyl, butynyl, pentynyl, hexynyl, or a branched-chain alkynyl group, etc.

As used herein, the term "alkoxy" refers to a radical that has an oxygen-linked straight-chain or branched hydrocarbon residue, which may be substituted or unsubstituted, unless otherwise noted. For example, the alkoxy may be, but not limited to, methoxy, ethoxy, propoxy, and butoxy, or isopropoxy, isobutoxy, or t-butoxy, etc.

As used herein, the term "alkylthio" refers to a -SR radical wherein R is an alkyl as defined herein, unless otherwise noted. For example, the alkylthio may be, but not limited to, methylthio, ethylthio, etc.

As used herein, the term "alkylsulfonyl" refers to a -SO₂R radical wherein R is an alkyl as defined herein, unless otherwise noted. For example, the alkylsulfonyl may be, but not limited to, methylsulfonyl, ethylsulfonyl, etc.

As used herein, the term "aminosulfonyl" refers to a -SO₂NH₂ radical, unless otherwise noted.

As used herein, the term "alkylaminosulfonyl" refers to a -SO₂NHR radical where R is an alkyl as defined herein, unless otherwise noted. For example, the alkylaminosulfonyl may be, but not limited to, methylaminosulfonyl, ethylaminosulfonyl, etc.

As used herein, the term "dialkylaminosulfonyl" refers to a -SO₂NR'R" radical, wherein R', R" are each alkyl as defined herein, unless otherwise noted.

As used herein, the term "halogen" refers to F, Cl, Br, or I.

As used herein, the term "haloalkyl" refers to including monohaloalkyl and polyhaloalkyl, which may be substituted or unsubstituted, unless otherwise noted. For example, the haloalkyl may be, but not limited to, -CH₂Cl, -CF₃, -CHF₂, -CH₂CF₃, -CF₂CF₃, -CF(CH₃)₂, etc.

As used herein, the term "haloalkoxy" refers to an -OR radical wherein R is a haloalkyl as defined herein, unless otherwise noted. For example, the haloalkoxy may be, but not limited to, -OCF₃, -OCHF₂, etc.

As used herein, the term "haloalkoxyalkyl" refers to an alkyl group substituted with a haloalkoxy as defined above, unless otherwise noted. For example, the haloalkoxyalkyl may be, but not limited to, a haloC₁₋₆alkoxyalkyl including a total of 1 to 6 carbon atoms.

For example, the haloalkoxyalkyl may be, but not limited to, trifluoromethoxymethyl, trifluoroethoxyethyl, etc.

As used herein, the term "cycloalkyl" refers to a saturated or partially unsaturated, monocyclic, bicyclic, or polycyclic hydrocarbon ring, which may be substituted or unsubstituted, unless otherwise noted. For example, the cycloalkyl may be, but not limited to, cyclopropyl, cyclobutyl, cyclopentyl, cyclohexyl, cycloheptyl, indanyl, indenyl, or tetrahydro-naphthalenyl, etc. The cycloalkyl includes bridged cycloalkyl, fused cycloalkyl, or spirocycloalkyl.

As used herein, the term "bridged cycloalkyl" refers to two rings sharing two non-adjacent common ring atoms, unless otherwise noted. The bridged cycloalkyl may be divided into dicyclic, tricyclic, tetracyclic, or a polycyclic bridged cycloalkyl based on the number of rings. For example, the bridged cycloalkyl may be, but not limited to, 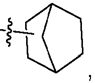 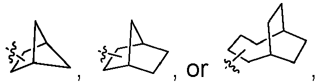 etc.

As used herein, the term "fused cycloalkyl" refers to, unless otherwise noted, that each ring shares an adjacent carbon atom pair with another ring, and one or more rings may share one or more double bonds, but none of these rings has a complete conjugated π-electron system. For example, the fused cycloalkyl may be divided into dicyclic, tricyclic, tetracyclic, or a polycyclic fused cycloalkyl depending on the number of rings. For example, the fused cycloalkyl may be, but not limited to, 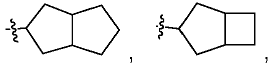 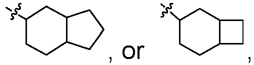 etc.

As used herein, the term "spiro" refers to two rings sharing one atom, and the two rings are not bridged, unless otherwise noted. As used herein, the term "spiro linkage" refers to a linker that share one atom, unless otherwise noted.

As used herein, the term "spirocycloalkyl" refers to, a saturated carbocycle compound including two rings, wherein both rings share only one carbon atom as part of the ring, unless otherwise noted. For example, the spirocycloalkyl may be, but not limited to, 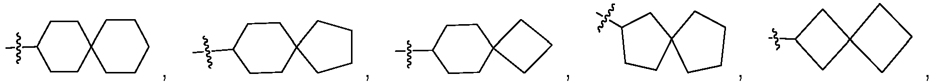 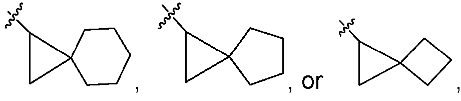 , etc.

As used herein, the term "cyano" refers to a -CN group.

As used herein, the term "ureido" refers to a -NH-CO-NH₂ radical.

As used herein, the term "amino" refers to a -NH₂ group. As used herein, the terms "alkylamino", "haloalkylamino", "dialkylamino", "hydroxyalkylamino", "alkoxyalkylamino", "aminoalkylamino", "sulfonylamino", "heteroarylamino", "heterocyclylamino", and "heterocyclyloxyalkylamino" refers to certain N-substituted organic radicals each bearing alkyl, haloalkyl, dialkyl, hydroxyalkyl, alkoxyalkyl, aminoalkyl, sulfonyl, heteroaryl, heterocyclyl, and heterocyclyloxyalkyl substituent. For example, the alkylamino may be, but not limited to, methylamino, ethylamino, or propylamino, etc. For example, the haloalkylamino may be, but not limited to, trifluoromethylamino or trifluoroethylamino, etc.

As used herein, the term "alkylcarbonyl" refers to a -C(O)R radical wherein R is an alkyl as defined herein, unless otherwise noted. For example, the alkylcarbonyl may include, but not limited to, C₁₋₆ alkylcarbonyl. For example, the alkylcarbonyl may be, but not limited to, methylcarbonyl, ethylcarbonyl, etc.

As used herein, the term "alkoxycarbonyl" refers to a -C(O)OR radical where R is an alkyl as defined herein, unless otherwise noted. For example, the alkoxycarbonyl may include, but not limited to, a C₁₋₆alkoxycarbonyl. For example, the alkoxycarbonyl may be, but not limited to, methoxycarbonyl, ethoxycarbonyl, etc.

As used herein, the term "aminocarbonyl" refers to a -CONH₂ radical, unless otherwise noted.

As used herein, the term "alkylaminocarbonyl" refers to a -CONHR radical where R is an alkyl as defined herein, unless otherwise noted. For example, the alkylaminocarbonyl may include, but not limited to, C₁₋₆ alkylaminocarbonyl. For example, the alkylaminocarbonyl may be, but not limited to, methylaminocarbonyl, ethylaminocarbonyl, etc.

As used herein, the term "dialkylaminocarbonyl" refers to a -CONR'R" radical wherein R' R" is each an alkyl as defined herein, unless otherwise noted. For example, the dialkylaminocarbonyl may include, but not limited to, (C₁₋₆alkyl)₂aminocarbonyl.

As used herein, the term "heterocyclylcarbonyl" refers to a -C(O)R radical where R is a heterocyclyl as defined herein, unless otherwise noted.

As used herein, the term "aminosulfonylalkyl" refers to -alkyl-S(=O)₂-NH₂, wherein alkyl is as defined herein, unless otherwise noted.

As used herein, the term "aminocarbonylalkyl" refers to -alkylene-(C=O)-NH₂, unless otherwise noted.

As used herein, the term "hydroxyalkyl" refers to an alkyl group substituted with an -OH group. For example, the hydroxyalkyl may be, but not limited to, hydroxymethyl, 2-hydroxy-ethyl, 2-hydroxypropyl, 3-hydroxypropyl, 1-(hydroxymethyl)-2-methylpropyl, 3-hydroxybutyl, 4-hydroxybutyl, 2,3-dihydroxypropyl, 1-(hydroxymethyl)-2-hydroxyethyl, 2,3-dihydroxybutyl, or 3,4-dihydroxybutyl, etc.

As used herein, the term "hydroxyalkoxy" refers to an -OR radical wherein R is a hydroxyalkyl as defined herein. For example, the hydroxyalkoxy may be, but not limited to, hydroxyethyloxy, hydroxypropyloxy, etc.

As used herein, the term "alkylamino" refers to a -NHR radical wherein R is an alkyl as defined herein. For example, the alkylamino may be, but not limited to, methylamino, ethylamino, propylamino, or isopropylamino, etc.

As used herein, the term "dialkylamino" refers to a -NR'R" radical wherein R' and R" are each alkyl as defined herein. For example, the dialkylamino may include, but not limited to, a (C₁₋₆alkyl)₂-amino. For example, the dialkylamino may include, but not limited to, dimethylamino, diethylamino, or methylethylamino.

As used herein, the term "hydroxyalkylamino" refers to a -NR'R" radical wherein R' is hydrogen or alkyl as defined herein and R" is hydroxyalkyl as defined herein. For example, the hydroxyalkylamino may be, but not limited to, hydroxyethylamino, hydroxypropylamino, etc.

As used herein, the term "haloC₁₋₆alkylamino" refers to a radical of the formula - NH-C₁₋₆alkyl, where C₁₋₆alkyl is partially or completely substituted by a halogen atom when permitted by available valence, in other words, a radical of the formula -NH-C₁₋₆alkyl-halo, unless otherwise noted.

As used herein, the term "(haloC₁₋₆alkyl)₂amino" refers to a radical of the formula - N-(C₁₋₆alkyl-halo)₂, unless otherwise noted.

As used herein, the term "C₁₋₆alkoxyC₁₋₆alkylamino" refers to a radical of the formula -NH-C₁₋₆alkyl, where C₁₋₆alkyl is partially or completely substituted with C₁₋₆alkoxy, in other words, a radical of the formula -NH-C₁₋₆alkyl-C₁₋₆alkoxy, unless otherwise noted.

As used herein, the term "aminoC₁₋₆alkylamino" refers to a radical of the formula - NH-C₁₋₆alkyl, where C₁₋₆alkyl is partially or completely substituted by amino, in other words, a radical of the formula -NH-C₁₋₆alkyl-amino, unless otherwise noted.

As used herein, the term "sulfonylamino" refers to the radical -NR'S(O)₂R", wherein R' and R" are each hydrogen, alkyl, alkenyl, alkynyl, aryl, cycloalkyl, or heterocyclyl, unless otherwise noted.

As used herein, the term "alkylsulfonylamino" refers to a -NHS(O)₂R radical wherein R is alkyl as defined herein, unless otherwise noted.

As used herein, the term "alkoxyalkyl" refers to an R'-O-R" radical, where R' and R" are each an alkyl as defined herein, unless otherwise noted. For example, the alkoxyalkyl may be, but not limited to, 2-methoxyethyl, 1-, 2-, or 3-methoxypropyl, 2-ethoxyethyl, etc. As used herein, the term "C₁₋₆alkoxyalkyl" refers to an alkyl-O-alkyl ether having a total number of carbons from 1 to 6, unless otherwise noted.

As used herein, the term "alkoxyalkoxy" refers to an -OR radical wherein R is an alkoxyalkyl as defined herein. For example, the alkoxyalkoxy may be, but not limited to, methoxyethyloxy, ethyloxypropyloxy, etc.

As used herein, the term "aminoalkyl" refers to a saturated alkyl group of a straight-chain or branched-chain represented by -NR'R" wherein R', R" are each an alkyl as defined herein, unless otherwise noted.

In an embodiment, R' and R" may be substituted with haloalkyl, hydroxyalkyl, alkoxyalkyl, or alkylcarbonyl.

The aminoalkyl may be, for example, but not limited to, aminomethyl, aminoethyl, methylaminomethyl, etc.

As used herein, the term "aminoalkoxy" refers to an -OR radical wherein R is an aminoalkyl as defined herein. For example, the aminoalkoxy may be, but not limited to, aminoethyloxy, methylaminopropyloxy, dimethylaminoethyloxy, diethylaminopropyloxy, etc.

As used herein, the term "aryl" refers to a monocyclic, bicyclic, or polycyclic aromatic hydrocarbon radical, which may be substituted or unsubstituted, unless otherwise noted. For example, the aryl may include, but not limited to, C₆₋₁₀ aryl. A double bond may alternate (resonate) between adjacent carbon atoms or suitable heteroatoms. For example, the aryl may be, but not limited to, phenyl, biphenyl, naphthyl, toluyl, or naphthalenyl, etc.

As used herein, the term "hetero" refers to an atom selected from B, N, O, S, P(=O), Si, and P.

As used herein, the term "heteroaryl" refers to a monocyclic, bicyclic, or polycyclic aromatic radical, which may be substituted or unsubstituted, including one or more heteroatoms selected from B, N, O, S, P(=O), Si, and P, unless otherwise noted. For example, the heteroaryl may include, but not limited to, a C₄₋₁₀ heteroaryl. For example, the monocyclic heteroaryl may be, but not limited to, pyridinyl, imidazolyl, thiazolyl, oxazolyl, thiophenyl, furanyl, pyrrolyl, isoxazolyl, pyrazolyl, triazolyl, thiadiazolyl, tetrazolyl, oxadiazolyl, pyridazinyl, pyrimidinyl, or pyrazinyl, etc. For example, the bicyclic heteroaryl may be, but not limited to, indolyl, benzothiophenyl, benzofuranyl, benzimidazolyl, benzoxazolyl, benzisoxazolyl, benzthiazolyl, benzthiadiazolyl, benztriazolyl, quinolinyl, isoquinolinyl, purinyl, or furopyridinyl, etc.

As used herein, the term "heteroaryloxy" refers to an -OR radical wherein R is a heteroaryl as defined herein, unless otherwise noted. For example, the heteroaryloxy may include, but not limited to, a C₄₋₁₀heteroaryloxy.

As used herein, the term "heterocyclyl" refers to a saturated or partially unsaturated, monocyclic, bicyclic, or polycyclic carbon atom ring structure radical, which may be substituted or unsubstituted, including one or more heteroatoms selected from B, N, O, S, P(=O), Si, and P, unless otherwise noted. For example, the heterocyclyl may be, but not limited to, 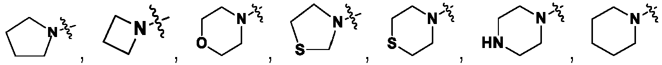 , or a similar group, etc. In addition, for example, the heterocyclyl may be, but not limited to, azetidinyl, pyrrolinyl, pyrrolidinyl, pyrazolinyl, pyrazolidinyl, imidazolinyl, imidazolidinyl, isoxazolinyl, isoxazolidinyl, isothiazolinyl, isothiazolidinyl, oxazolinyl, oxazolidinyl, thiazolinyl, thiazolidinyl, triazolinyl, triazolidinyl, oxadiazolinyl, oxadiazolidinyl, thiadiazolinyl, thiadiazolidinyl, tetrazolinyl, tetrazolidinyl, piperazinyl, piperidinyl, morpholinyl, thiomorpholinyl, dihydro-indazolyl, tetrahydro-indazolyl, dihydro-isoindolyl, dihydrobenzofuranyl, tetrahydro-benzofuranyl, dihydro-benzothienyl, tetrahydro-benzothienyl, dihydro-benzimidazolyl, tetrahydro-benzimidazolyl, dihydro-benzoxazolyl, dihydropyridinyl, dihydroquinolinyl, benzodioxynyl (such as, 1,4-benzodioxane), or benzodioxolyl (such as, 1,3-benzodioxole), etc. The heterocyclyl includes bridged heterocyclyl, fused heterocyclyl, or spiroheterocyclyl. For example, the fused heterocyclyl may be, but not limited to, 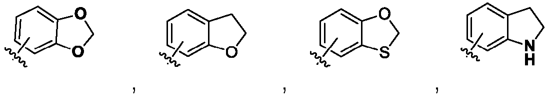 , or 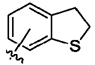 , etc.

As used herein, the term "bridged heterocycloalkyl" refers to a bridged cycloalkyl containing one or more heteroatoms selected from B, N, O, S, P(=O), Si, and P, unless otherwise noted.

As used herein, the term "fused heterocycloalkyl" refers to a fused cycloalkyl containing one or more heteroatoms selected from B, N, O, S, P(=O), Si, and P, unless otherwise noted.

As used herein, the term "heterospirocycloalkyl" refers to a spirocycloalkyl containing one or more hetero atoms selected from B, N, O, S, P(=O), Si, and P, unless otherwise noted. For example, the heterospirocycloalkyl may be, but not limited to, 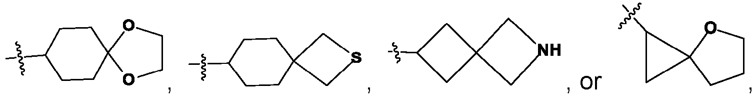 etc.

As used herein, the term "heterocyclyloxy" refers to an -OR radical where R is a heterocyclyl as defined herein, unless otherwise noted. For example, the heterocyclyloxy may be, but not limited to, pyrrole-2-yloxy, pyrrole-3-yloxy, piperidin-2-yloxy, piperidin-3-yloxy, piperazine-2-yloxy, or piperidin-4-yloxy, etc.

As used herein, the term "heterocyclylamino" refers to a -NHR radical wherein R is a heterocyclyl as defined herein, unless otherwise noted. For example, the heterocyclylamino may be, but not limited to, pyrrole-2-ylamino, pyrrole-3-ylamino, piperidin-2-ylamino, piperidin-3-ylamino, piperazine-2-ylamino, or piperidin-4-ylamino, etc.

As used herein, the term "heterocyclyloxyalkoxy" refers to an -OR radical, wherein R is heterocyclyl as defined herein, unless otherwise noted. For example, the heterocyclyloxyalkoxy may be, but not limited to, pyrrole-2-yloxymethoxy, pyrrole-3-yloxyethoxy, piperidin-2-yloxyethoxy, piperidin-3-yloxyethoxy, piperazine-2-yloxymethoxy, or piperidin-4-yloxyethoxy, etc.

As used herein, the term "heterocyclyloxyalkylamino" refers to a -NR-(alkylene)-R' radical wherein R' is hydrogen or an alkyl as defined herein and R" is heterocyclyloxy as defined herein. For example, the heterocyclyloxyalkylamino may be, but not limited to, oxetanyloxyethylamino, piperidinyloxyethylamino, etc.

As used herein, the term "heterocycloalkyl" refers to a monocyclic, which may be substituted or unsubstituted, cyclic alkyl including one or more heteroatoms selected from B, N, O, S, P(=O), Si, and P, unless otherwise noted. For example, the heterocycloalkyl may be, but not limited to, piperidinyl, piperazineyl, morpholinyl, pyrrolidinyl, thiomorpholinyl, imidazolidinyl, or tetrahydrofuryl, etc.

As used herein, the term "stereoisomer" may refer to compounds of the present disclosure or salts thereof that have the same chemical formula or molecular formula but differ optically or sterically, and includes enantiomers or diastereomers.

As used herein, the term "enantiomers" refers to two stereoisomers of a compound that are non-overlapping mirror images of each other.

As used herein, the term "diastereomer" refers to a stereoisomer with two or more chiral centers, the molecules of which are not mirror images of each other.

The compounds of the present disclosure may include asymmetric or chiral centers, and thus may exist in different stereoisomeric forms. All stereoisomeric forms of the compounds of the present disclosure, such as diastereomers, enantiomers, and racemic mixtures, are considered to compose a portion of the present disclosure. A 50:50 mixture of enantiomers is referred to as a racemic mixture or racemate.

As used herein, the term "solvate" may refer to a compound of the present disclosure or a salt thereof including a stoichiometric or non-stoichiometric amount of solvent bound by non-covalent intermolecular forces. Suitable solvents may include those that are volatile, non-toxic, and/or suitable for human administration. A "solvate" may include a molecular complex including the compound and one or more pharmaceutically acceptable solvent molecules, for example ethanol.

As used herein, the term "hydrate" refers to a complex in which the solvent molecule is water.

As used herein, the term "pharmaceutically acceptable salt" refers to a pharmaceutically acceptable organic or inorganic salt, which may be prepared by any suitable method useful to those skilled in the art. For example, when the compound of the present disclosure is a base, the desired pharmaceutically acceptable salt may be prepared by any suitable method useful to those skilled in the art, for example, by treating the free base with an inorganic acid or organic acid.

In an embodiment, in Formula 1 above 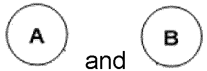 may each independently be H, C₃₋₁₀ cycloalkyl, bridged C₈₋₁₆ cycloalkyl, fused C₃₋₁₀ cycloalkyl, C₈₋₁₆ spirocycloalkyl, C₆₋₁₀ aryl, C₄₋₁₀ heteroaryl, C₁₋₁₀ heterocyclyl, bridged C₆₋₁₄ heterocyclyl, fused C₁₋₁₀ heterocyclyl, or C₆₋₁₄ spiroheterocyclyl.

In an embodiment, in Formula 1 above
X and Z may each independently be N or CH;
Y may be CR^{3a};
R^{3a} may be H, C₁₋₆alkyl, C₂₋₆alkenyl, C₂₋₆alkynyl, C₁₋₆alkoxy, C₁₋₆alkylthio, C₁₋₆alkylsulfonyl, hydroxy, halogen, haloC₁₋₆alkyl, haloC₁₋₆alkoxy, C₃₋₁₀cycloalkyl, cyano, amino, C₁₋₆alkylamino, haloC₁₋₆alkylamino, C₂₋₁₂dialkylamino, C₁₋₆alkylcarbonyl, C₁₋₆alkoxycarbonyl, aminocarbonyl, C₁₋₆alkylaminocarbonyl, C₂₋₁₂dialkylaminocarbonyl, hydroxyC₁₋₆alkyl, hydroxyC₁₋₆alkoxy, hydroxyC₁₋₆alkylamino, C₁₋₆alkoxyC₁₋₆alkyl, C₁₋₆alkoxyC₁₋₆alkoxy, C₁₋₆alkoxyC₁₋₆alkylamino, aminoC₁₋₆alkyl, aminoC₁₋₆alkoxy, aminoC₁₋₆alkylamino, C₁₋₁₀aryl, C₅₋₁₀heteroaryl, C₅₋₁₀heteroaryloxy, C₄₋₁₀heteroarylamino, C₁₋₁₀heterocyclyl, C₁₋₁₀heterocyclyloxy, C₁₋₁₀heterocyclylamino, C₁₋₁₀heterocyclyloxyalkoxy, or C₁₋₁₀heterocyclyloxyalkylamino,
wherein each of C₆₋₁₀aryl, C₄₋₁₀heteroaryl, or C₁₋₁₀heterocyclyl may be
unsubstituted or substituted with 1 to 3 members selected from C₁₋₆alkyl, C₃₋₁₀cycloalkyl, haloC₁₋₆alkyl, haloC₁₋₆alkoxy, C₁₋₆alkoxy, hydroxy, halogen, cyano, hydroxyC₁₋₆alkyl, C₁₋₆alkoxyC₁₋₆alkyl, and aminoC₁₋₆alkyl; 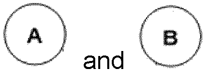
may each independently be H, C₃₋₁₀ cycloalkyl, bridged C₈₋₁₆ cycloalkyl, fused C₃₋₁₀ cycloalkyl, C₈₋₁₆ spirocycloalkyl, C₆₋₁₀ aryl, C₄₋₁₀ heteroaryl, C₁₋₁₀ heterocyclyl, bridged C₆₋₁₄ heterocyclyl, fused C₁₋₁₀heterocyclyl, or C₆₋₁₄spiroheterocyclyl, wherein C₆₋₁₀aryl, C₄₋₁₀heteroaryl, or C₁₋₁₀heterocyclyl is unsubstituted or substituted with 1 to 3 members independently selected from R^{2a} below;
L may be C₁₋₃alkylene, O, NH, NCH₃, S, SO, SO₂, or a bond;
R^{2a} may be C₁₋₆alkyl, C₃₋₁₀cycloalkyl, haloC₁₋₆alkyl, haloC₁₋₆alkylamino, haloC₁₋₆alkoxy, C₁₋₆alkoxy, hydroxy, C₁₋₆alkylsulfonyl, halogen, cyano, carboxy, C₁₋₆alkoxycarbonyl, hydroxyC₁₋₆alkyl, C₁₋₆alkoxyalkyl, aminoC₁₋₆alkyl, aminosulfonyl, C₁₋₆alkylaminosulfonyl, (C₁₋₆alkyl)₂aminosulfonyl, sulfonylamino, aminocarbonyl, C₁₋₆alkylaminocarbonyl, (C₁₋₆alkyl)₂aminocarbonyl, C₁₋₁₀heterocyclylcarbonyl, or ureido; and
R¹ may be H, C₁₋₆alkyl, haloC₁₋₆alkyl, hydroxyC₁₋₆alkyl, C₁₋₆alkoxyalkyl, aminoC₁₋₆alkyl, substituted aminoC₁₋₆alkyl, aminocarbonylC₁₋₆alkyl, or aminosulfonylC₁₋₆alkyl.
In an embodiment, in Formula 1 above
X and Z may each independently be N or CH;
Y may be CR^{3b};
R^{3b} may be H, C₂₋₄alkenyl, C₂₋₄alkynyl, C₁₋₃alkoxy, hydroxy, halogen, haloC₁₋₆alkyl, C₃₋₆cycloalkyl, amino, C₁₋₃alkylamino, haloC₁₋₆alkylamino, or C₂₋₆heterocyclyl; 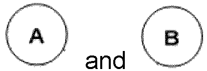
may each independently be H, C₆₋₁₀aryl, C₄₋₁₀heteroaryl, or C₁₋₁₀heterocyclyl, wherein C₆₋₁₀aryl, C₄₋₁₀heteroaryl, or fused C₁₋₁₀heterocyclyl may be unsubstituted or substituted with 1 to 3 members independently selected from R^{2b} below;
L may be C₁₋₆alkylene, O, NH, NCH₃, S, SO, SO₂, or a bond;
R^{2b} may be C₁₋₆alkyl, haloC₁₋₃alkyl, haloC₁₋₃alkoxy, C₁₋₆alkoxy, hydroxy, or halogen; and
R¹ may be H or C₁₋₆alkyl.
In an embodiment, in Formula 1 above
X and Z may each independently be N or CH;
Y may be CR^{3c};
R^{3c} may be H, C₂₋₄alkenyl, C₂₋₄alkynyl, C₁₋₃alkoxy, hydroxy, halogen, haloC₁₋₃alkyl, C₃₋₆cycloalkyl, amino, C₁₋₃alkylamino, haloC₁₋₃alkylamino, or C₂₋₆heterocyclyl; 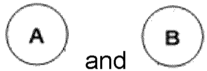
may each independently be H, phenyl, pyridinyl, imidazolyl, pyrrolidinyl, indolyl, naphthalenyl, benzodioxynyl, or benzodioxolyl, wherein 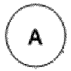 and 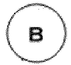 may be unsubstituted or substituted with 1 to 3 members independently selected from C₁₋₃alkyl, haloC₁₋₃alkyl, haloC₁₋₃alkoxy, C₁₋₃alkoxy, hydroxy, and halogen;
L may be C₁₋₃alkylene, O, NH, NCH₃, S, SO, SO₂, or a bond; and
R¹ may be H or C₁₋₃alkyl.
In an embodiment, in Formula 1 above 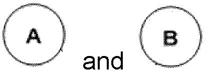
may each independently be H, phenyl, pyridinyl, imidazolyl, pyrrolidine, 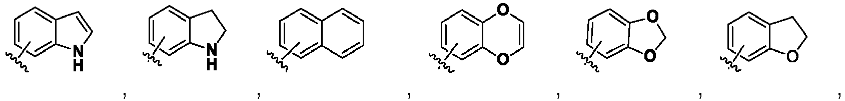 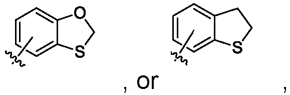
wherein 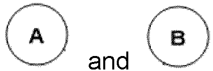 may be unsubstituted or substituted with 1 to 3 members independently selected from C₁₋₃alkyl, haloC₁₋₃alkyl, haloC₁₋₃alkoxy, C₁₋₃alkoxy, hydroxy, and halogen.

In an embodiment, in Formula 1 above 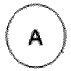
may be phenyl or pyridinyl, and
may be unsubstituted or substituted with 1 to 3 members independently selected from C₁₋₃alkyl, haloC₁₋₃alkyl, haloC₁₋₃alkoxy, C₁₋₃alkoxy, and halogen; 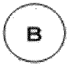
may be H, phenyl, pyridinyl, imidazolyl, pyrrolidinyl, or 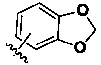 , and
may be unsubstituted or substituted with 1 to 3 members independently selected from C₁₋₃alkyl, haloC₁₋₃alkoxy, C₁₋₃alkoxy, hydroxy, and halogen;
X and Z may each be independently N or CH;
Y may be CR^{3c}, wherein R^{3c} may be H, C₂₋₄alkenyl, C₂₋₄alkynyl, C₁₋₃alkoxy, hydroxy, halogen, haloC₁₋₃alkyl, C₃₋₆cycloalkyl, amino, C₁₋₃alkylamino, haloC₁₋₃alkylamino, or C₂₋₆heterocyclyl;
L may be methylene, ethylene, O, NH, NCH₃, S, SO, or SO₂; and/or
R¹ may be H or methyl.
In an embodiment
X and Z may each independently be N or CH;
Y may be CR^{3c}, wherein R^{3c} may be H, C₂₋₄alkenyl, C₂₋₄alkynyl, C₁₋₃alkoxy, hydroxy, halogen, haloC₁₋₃alkyl, C₃₋₆cycloalkyl, amino, C₁₋₃alkylamino, haloC₁₋₃alkylamino, or C₂₋₆heterocyclyl; 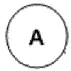
may be phenyl or pyridinyl; 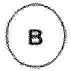
may be H, phenyl, pyridinyl, imidazolyl, pyrrolidinyl, or 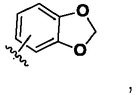
wherein 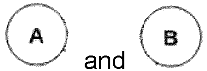 may each independently be
unsubstituted or substituted with 1 to 3 members independently selected from C₁₋₃alkyl, haloC₁₋₃alkyl, haloC₁₋₃alkoxy, C₁₋₃alkoxy, hydroxy, and halogen;
L may be methylene, ethylene, O, NH, NCH₃, S, SO, or SO₂; and
R¹ may be H or methyl.

In an embodiment, the compound may be selected from the group consisting of the following compounds and enantiomers, diastereomers, solvates and hydrates thereof, and pharmaceutically acceptable salts thereof.
1) 2-benzyl-7-chloro-1-methyl-5-phenyl-1,5-dihydro-4*H*-imidazo[4,5-*c*]quinolin-4-one;
2) 7-chloro-2-(4-methoxybenzyl)-1-methyl-5-phenyl-1,5-dihydro-4*H-*imidazo[4,5-*c*]quinolin-4-one;
3) 7-chloro-2-(4-hydroxybenzyl)-1-methyl-5-phenyl-1,5-dihydro-4*H-*imidazo[4,5-*c*]quinolin-4-one;
4) 7-chloro-2-(3-methoxybenzyl)-1-methyl-5-phenyl-1,5-dihydro-4*H-*imidazo[4,5-*c*]quinolin-4-one;
5) 7-chloro-2-(2-methoxybenzyl)-1-methyl-5-phenyl-1,5-dihydro-4*H-*imidazo[4,5-*c*]quinolin-4-one;
6) 7-chloro-2-(4-chlorobenzyl)-1-methyl-5-phenyl-1,5-dihydro-4*H-*imidazo[4,5-*c*]quinolin-4-one;
7) 7-chloro-1-methyl-5-phenyl-2-(pyridin-4-ylmethyl)-1,5-dihydro-4*H-*imidazo[4,5-*c*]quinolin-4-one;
8) 7-chloro-2-(3,4-dimethoxybenzyl)-1-methyl-5-phenyl-1,5-dihydro-4*H-*imidazo[4,5-*c*]quinolin-4-one;
9) 7-chloro-1-methyl-2-phenethyl-5-phenyl-1,5-dihydro-4*H*-imidazo[4,5-*c*]quinolin-4-one;
10) 7-chloro-2-(4-methoxyphenyl)-1-methyl-5-(*o*-tolyl)-1,5-dihydro-4*H-*imidazo[4,5-*c*]quinolin-4-one;
11) 2-benzyl-7-chloro-5-phenyl-15-dihydro-4*H*-imidazo[4,5-c]quinolin-4-one-,
12) 2-((1*H*-imidazol-1-yl)methyl)-7-chloro-1-methyl-5-phenyl-1,5-dihydro-4*H-*imidazo[4,5-*c*]quinolin-4-one;
13) 7-chloro-2-((3-methoxypyrrolidin-1-yl)methyl)-1-methyl-5-phenyl-1,5-dihydro-4*H*-imidazo[4,5-*c*]quinolin-4-one;
14) 7-chloro-2-((3-hydroxypyrrolidin-1-yl)methyl)-1-methyl-5-phenyl-1,5-dihydro-4*H*-imidazo[4,5-*c*]quinolin-4-one;
15) 2-(4-methoxybenzyl)-1-methyl-5-phenyl-7-((2,2,2-trifluoroethyl)amino)-1,5-dihydro-4*H*-imidazo[4,5-*c*][1,8]naphthyridin-4-one;
16) 7-chloro-5-(4-chloro-3-fluorophenyl)-2-(4-methoxybenzyl)-1-methyl-1,5-dihydro-4*H*-imidazo[4,5-*c*]quinolin-4-one;
17) 7-chloro-5-(3-chloro-4-fluorophenyl)-2-(4-methoxybenzyl)-1-methyl-1,5-dihydro-4*H*-imidazo[4,5-*c*]quinolin-4-one;
18) 7-chloro-2-((4-methoxyphenyl)thio)-1-methyl-5-phenyl-1,5-dihydro-4*H-*imidazo[4,5-*c*]quinolin-4-one;
19) 7-chloro-2-((4-methoxyphenyl)sulfonyl)-1-methyl-5-phenyl-1,5-dihydro-4*H*-imidazo[4,5-*c*]quinolin-4-one;
20) 7-chloro-2-((4-methoxyphenyl)sulfinyl)-1-methyl-5-phenyl-1,5-dihydro-4*H*-imidazo[4,5-*c*]quinolin-4-one;
21) 7-chloro-2-(4-methoxybenzyl)-1-methyl-5-(2-methylpyridin-3-yl)-1,5-dihydro-4*H*-imidazo[4,5-*c*]quinolin-4-one;
22) 2-(4-methoxybenzyl)-1-methyl-5-(2-methylpyridin-3-yl)-1,5-dihydro-4*H-*imidazo[4,5-*c*]quinolin-4-one;
23) 7-chloro-2-(4-methoxybenzyl)-1-methyl-5-(2-(trifluoromethyl)pyridin-3-yl)-1,5-dihydro-4*H*-imidazo[4,5-*c*]quinolin-4-one;
24) 7-chloro-5-(2-cyclopropylpyridin-3-yl)-2-(4-methoxybenzyl)-1-methyl-1,5-dihydro-4*H*-imidazo[4,5-*c*]quinolin-4-one;
25) 7-chloro-2-(4-methoxybenzyl)-1-methyl-5-(2-(trifluoromethyl)phenyl)-1,5-dihydro-4*H*-imidazo[4,5-*c*]quinolin-4-one;
26) 7-chloro-5-(2-chlorophenyl)-2-(4-methoxybenzyl)-1-methyl-1,5-dihydro-4*H*-imidazo[4,5-*c*]quinolin-4-one;
27) 2-(4-methoxybenzyl)-1-methyl-5-(2-methylpyridin-3-yl)-7-(trifluoromethyl)-1,5-dihydro-4*H*-imidazo[4,5-*c*]quinolin-4-one;
28) 7-chloro-2-(4-fluorobenzyl)-1-methyl-5-(2-methylpyridin-3-yl)-1,5-dihydro-4*H*-imidazo[4,5-*c*]quinolin-4-one;
29) 2-(benzo[*d*][1,3]dioxol-5-ylmethyl)-7-chloro-1-methyl-5-(2-methylpyridin-3-yl)-1,5-dihydro-4*H*-imidazo[4,5-*c*]quinolin-4-one;
30) 7-chloro-2-(3,4-difluorobenzyl)-1-methyl-5-(2-methylpyridin-3-yl)-1,5-dihydro-4*H*-imidazo[4,5-*c*]quinolin-4-one;
31) 7-chloro-2-(4-methoxybenzyl)-1-methyl-5-(pyridin-3-yl)-1,5-dihydro-4*H-*imidazo[4,5-*c*]quinolin-4-one;
32) 7-chloro-5-(4-fluorophenyl)-2-(4-methoxybenzyl)-1-methyl-1,5-dihydro-4*H*-imidazo[4,5-*c*]quinolin-4-one;
33) 7-chloro-2-(4-(difluoromethoxy)benzyl)-1-methyl-5-(2-methylpyridin-3-yl)-1,5-dihydro-4*H*-imidazo[4,5-*c*]quinolin-4-one;
34) 7-chloro-5-(4-methoxyphenyl)-2-((4-methoxyphenyl)thio)-1-methyl-1,5-dihydro-4*H*-imidazo[4,5-*c*]quinolin-4-one;
35) 7-chloro-2-((4-methoxyphenyl)thio)-1-methyl-5-(2-methylpyridin-3-yl)-1,5-dihydro-4*H*-imidazo[4,5-*c*]quinolin-4-one;
36) 7-chloro-2-((4-fluorophenyl)thio)-1-methyl-5-(2-methylpyridin-3-yl)-1,5-dihydro-4*H*-imidazo[4,5-*c*]quinolin-4-one;
37) 7-chloro-2-((3,4-difluorophenyl)thio)-1-methyl-5-(2-methylpyridin-3-yl)-1,5-dihydro-4*H*-imidazo[4,5-*c*]quinolin-4-one;
38) 2-(benzo[*d*][1,3]dioxol-5-ylthio)-7-chloro-1-methyl-5-(2-methylpyridin-3-yl)-1,5-dihydro-4*H*-imidazo[4,5-*c*]quinolin-4-one;
39) 7-chloro-2-((2,4-difluorophenyl)thio)-1-methyl-5-(2-methylpyridin-3-yl)-1,5-dihydro-4*H*-imidazo[4,5-*c*]quinolin-4-one;
40) 7-chloro-2-((4-methoxyphenyl)amino)-1-methyl-5-phenyl-1,5-dihydro-4*H-*imidazo[4,5-*c*]quinolin-4-one;
41) 7-chloro-2-((4-methoxyphenyl)(methyl)amino)-1-methyl-5-phenyl-1,5-dihydro-4*H*-imidazo[4,5-*c*]quinolin-4-one;
42) 7-chloro-2-(4-methoxyphenoxy)-1-methyl-5-phenyl-1,5-dihydro-4*H-*imidazo[4,5-*c*]quinolin-4-one;
43) 7-hydroxy-2-(4-methoxybenzyl)-1-methyl-5-(2-methylpyridin-3-yl)-1,5-dihydro-4*H*-imidazo[4,5-*c*]quinolin-4-one;
44) 2-(4-methoxybenzyl)-1-methyl-5-(2-methylpyridin-3-yl)-7-vinyl-1,5-dihydro-4*H*-imidazo[4,5-*c*]quinolin-4-one;
45) 7-ethyl-2-(4-methoxybenzyl)-1-methyl-5-(2-methylpyridin-3-yl)-1,5-dihydro-4*H*-imidazo[4,5-*c*]quinolin-4-one;
46) 7-cyclopropyl-2-(4-methoxybenzyl)-1-methyl-5-(2-methylpyridin-3-yl)-1,5-dihydro-4*H*-imidazo[4,5-*c*]quinolin-4-one;
47) 7-ethynyl-2-(4-methoxybenzyl)-1-methyl-5-(2-methylpyridin-3-yl)-1,5-dihydro-4*H*-imidazo[4,5-*c*]quinolin-4-one;
48) 7-isopropyl-2-(4-methoxybenzyl)-1-methyl-5-(2-methylpyridin-3-yl)-1,5-dihydro-4*H*-imidazo[4,5-*c*]quinolin-4-one;
49) 2-(4-methoxybenzyl)-1,7-dimethyl-5-(2-methylpyridin-3-yl)-1,5-dihydro-4*H*-imidazo[4,5-*c*]quinolin-4-one;
50) 2-(4-methoxybenzyl)-1-methyl-5-(2-methylpyridin-3-yl)-7-(pyrrolidin-1-yl)-1,5-dihydro-4*H*-imidazo[4,5-*c*]quinolin-4-one;
51) 7-methoxy-2-(4-methoxybenzyl)-1-methyl-5-(2-methylpyridin-3-yl)-1,5-dihydro-4*H*-imidazo[4,5-*c*]quinolin-4-one;
52) 7-amino-2-(4-methoxybenzyl)-1-methyl-5-(2-methylpyridin-3-yl)-1,5-dihydro-4*H*-imidazo[4,5-*c*]quinolin-4-one;
53) 2-(4-methoxybenzyl)-1-methyl-7-(methylamino)-5-(2-methylpyridin-3-yl)-1,5-dihydro-4*H*-imidazo[4,5-*c*]quinolin-4-one;
54) 2-(4-(difluoromethoxy)benzyl)-7-ethyl-1-methyl-5-(2-methylpyridin-3-yl)-1,5-dihydro-4*H*-imidazo[4,5-*c*]quinolin-4-one;
55) 2-(4-(difluoromethoxy)benzyl)-7-methoxy-1-methyl-5-(2-methylpyridin-3-yl)-1,5-dihydro-4*H*-imidazo[4,5-*c*]quinolin-4-one;
56) 5-(4-(difluoromethoxy)phenyl)-2-(4-methoxybenzyl)-1-methyl-7-(trifluoromethyl)-1,5-dihydro-4*H*-imidazo[4,5-*c*][1,8]naphthyridin-4-one;
57) 2-(4-methoxybenzyl)-1-methyl-5-(2-methylpyridin-3-yl)-7-(trifluoromethyl)-1,5-dihydro-4*H*-imidazo[4,5-*c*][1,8]naphthyridin-4-one;
58) 2-(4-(difluoromethoxy)benzyl)-1-methyl-5-(2-methylpyridin-3-yl)-7-(trifluoromethyl)-1,5-dihydro-4*H*-imidazo[4,5-*c*][1,8]naphthyridin-4-one;
59) 2-((4-methoxyphenyl)thio)-1-methyl-5-(2-methylpyridin-3-yl)-7-(trifluoromethyl)-1,5-dihydro-4*H*-imidazo[4,5-*c*][1,8]naphthyridin-4-one;
60) 2-((4-fluorophenyl)thio)-1-methyl-5-(2-methylpyridin-3-yl)-7-(trifluoromethyl)-1,5-dihydro-4*H*-imidazo[4,5-*c*][1,8]naphthyridin-4-one;
61) 2-((4-methoxyphenyl)thio)-1-methyl-5-(2-methylpyridin-3-yl)-7-(trifluoromethyl)-1,5-dihydro-4*H*-imidazo[4',5':4,5]pyrido[2,3-*d*]pyrimidin-4-one;
62) 7-ethoxy-2-(4-methoxybenzyl)-1-methyl-5-(2-methylpyridin-3-yl)-1,5-dihydro-4*H*-imidazo[4',5':4,5]pyrido[2,3-*d*]pyrimidin-4-one;
63) 2-(4-(difluoromethoxy)benzyl)-7-methoxy-1-methyl-5-(2-methylpyridin-3-yl)-1,5-dihydro-4*H*-imidazo[4,5-*c*][1,8]naphthyridin-4-one;
64) 7-methoxy-2-(4-methoxybenzyl)-1-methyl-5-(2-methylpyridin-3-yl)-1,5-dihydro-4*H*-imidazo[4,5-*c*][18]naphthyridin-4-one;
65) 2-(4-(difluoromethoxy)benzyl)-7-ethyl-1-methyl-5-(2-methylpyridin-3-yl)-1,5-dihydro-4*H*-imidazo[4,5-*c*][1,8]naphthyridin-4-one; and
66) 7-cyclopropyl-2-(4-(difluoromethoxy)benzyl)-1-methyl-5-(2-methylpyridin-3-yl)-1,5-dihydro-4*H*-imidazo[4,5-*c*][1,8]naphthyridin-4-one.

Another embodiment of the present disclosure provides a pharmaceutical composition for treating or preventing a methionine Adenosyltransferase 2A (MAT2A)-related disease or disorder, the pharmaceutical composition including as an active ingredient a compound selected from the compounds of Formula 1 and enantiomers, diastereomers, solvates and hydrates thereof, and pharmaceutically acceptable salts thereof.

In an embodiment, the composition may exhibit MAT2A inhibitory activity.

In an embodiment, the composition may be for treating cancer. For example, the cancer may be a solid tumor.

In an embodiment, the composition may be for treating a methylthioadenosine phosphorylase (MTAP) deficient cancer.

In an embodiment, the pharmaceutical composition may include, in a therapeutically effective amount, the compound selected from the compounds of Formula 1 and enantiomers, diastereomers, solvates and hydrates thereof, and pharmaceutically acceptable salts thereof.

As used herein, the term "therapeutically effective amount" refers to an amount of a compound of the present disclosure that treats or prevents a particular disease, condition, or disorder; attenuates, ameliorates, or eliminates one or more symptoms of a particular disease, condition, or disorder; or prevents or delays the onset of one or more symptoms of a particular disease, condition, or disorder.

A physician of ordinary skill in the relevant art may readily determine and prescribe an effective required dose of a pharmaceutical composition. For example, the pharmaceutical composition may include, but not limited to, 0.0001 mg to 10 g of the compound.

In an embodiment, the pharmaceutical composition may further include a pharmaceutically acceptable additive in addition to the active ingredient. The additive may be, for example, but not limited to, diluents, disintegrating agents, binders, lubricants, surfactants, suspending agents, or emulsifiers, etc.

The pharmaceutical compositions of the present disclosure may be formulated according to usual methods and may be prepared in various oral dosage forms, such as tablets, pills, powders, capsules, syrups, emulsions, microemulsions, or parenteral dosage forms, such as intramuscular, intravenous, or subcutaneous administration.

Another embodiment of the present disclosure provides a method of treatment by administering to a subject suffering from a MAT2A-related disease or disorder the pharmaceutical composition including as an active ingredient the compound selected from the compounds of Formula 1 and enantiomers, diastereomers, solvates and hydrates thereof, and pharmaceutically acceptable salts thereof.

As used herein, the term "treating" or "curing" refers to inhibiting a disease, for example, inhibiting a disease, condition, or disorder in a subject experiencing or exhibiting the pathology or symptoms of the disease, condition, or disorder, that is, preventing or reversing further development of the pathology and/or symptoms, or ameliorating a disease, for example, reducing disease severity.

As used herein, the term "preventing" or "prophylactic" refers to preventing a disease, for example, preventing a disease, condition, or disorder in an individual who may have a predisposition to the disease, condition, or disorder but has not yet experienced or exhibited the pathology or symptoms of the disease.

As used herein, the term "subject" or "individual" may be a vertebrate such as a mammal, fish, bird, reptile, or amphibian. For example, a subject may be a human, nonhuman primate, horse, pig, rabbit, dog, sheep, goat, cow, cat, guinea pig, or rodent.

As used herein, the terms "administering" and "administration" refer to any method of providing a disclosed composition to a subject.

The dosage, number of doses, or method of administration of a compound or pharmaceutical composition according to an embodiment may vary depending on the subject being treated, the severity of the disease or condition, the rate of administration, and the judgment of the prescribing physician. For example, a typical dosage for a person weighing 70 kg may be administered in an amount from 0.0001 mg to 10 g per day, for example 1 mg to 1 g per day. The number of doses may be administered once to several times, for example 1 to 4 times, or on an on/off schedule, and the method of administration may be by an oral or parenteral route. For example, a compound or pharmaceutical composition according to an embodiment may be administered by an oral or parenteral route in an amount ranging from 0.1 to 100 mg/kg (body weight).

The physician may start at a lower level than necessary to achieve the target therapeutic effect and gradually increase the dose of the compound or pharmaceutical composition of the present disclosure administered to the subject until the intended effect is achieved.

Another embodiment of the present disclosure provides a kit including as an active ingredient the compound selected from the compounds of Formula 1 and enantiomers, diastereomers, solvates and hydrates thereof, and pharmaceutically acceptable salts thereof.

In an embodiment, the therapeutic agent may be a drug for treating a MAT2A-related disease or disorder, such as a drug for treating cancer. For example, the therapeutic agent may be a chemotherapeutic agent for the treatment of cancer.

In an embodiment, the therapeutic agent may be a chemotherapeutic agent, an antimetabolite, an antimitotic, an alkylating agent, a DNA damaging agent, an antineoplastic antibiotic, a platinum coordination complex, a proteasome inhibitor, a hormone and hormone analog, an immunosuppressant, an anti-angiogenic compound and a vascular endothelial growth factor inhibitor, a fibroblast growth factor inhibitor, an epidermal growth factor receptor inhibitor, an antibody, a checkpoint inhibitor, a corticosteroid, a DNA repair enzyme inhibitor, a treatment for autoimmune disorders, a treatment for diabetes, a treatment for liver disease, a treatment for cardiovascular disorder, a treatment for metabolic disorder, an antiviral agent, or an ophthalmic agent.

In an embodiment, the compounds, compositions and kits of the present disclosure may be administered alone or simultaneously with at least one other therapeutic agent, separately or sequentially.

In the context of the present disclosure, the singular form of a word may include the plural and vice versa, unless the context clearly indicates otherwise.

Figures given herein shall be deemed to include the meaning of "about" even if not expressly stated. As used herein, the term "about" refers to within 5 % of a certain value or range, preferably within 1 % to 2 %.

As used herein, the numerical range indicated using the term "to" refers to a range that includes the numerical values written before and after the term "to" as the lower limit and upper limit, respectively.

As used herein, the terms "has," "may have," "includes," or "may include" refer to the presence of a particular feature (for example, a number, or a component such as an ingredient) and do not exclude the presence of additional features.

The contents of all publications incorporated by reference herein are hereby incorporated by reference in their entirety.

Hereinafter, the present disclosure will be further described in more detail through the following embodiments and experimental examples. However, these embodiments and examples are intended only to illustrate the present disclosure and are not intended to limit the scope of the present disclosure in any way.

### <Synthesis Example 1>

### Example 1: 2-benzyl-7-chloro-1-methyl-5-phenyl-1,5-dihydro-4H-imidazo[4,5-c]quinolin-4-one

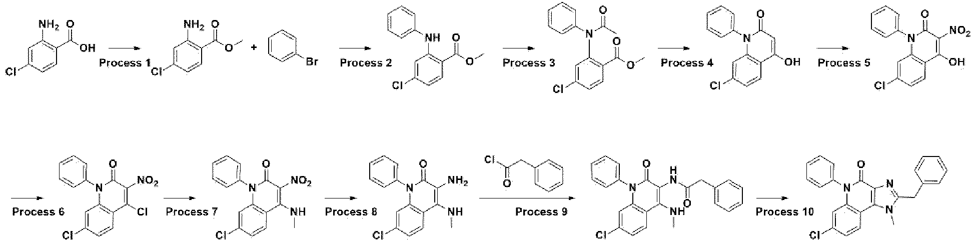

### [Process 11 Preparation of methyl 2-amino-4-chlorobenzoate

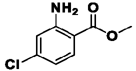

2-amino-4-chlorobenzoic acid (10 g, 57.12 mmol), potassium carbonate (11.90 g, 85.67 mmol) and iodomethane (3.63 mL, 57.12 mmol) were sequentially added to *N,N-*dimethylformamide (200 mL) and the reaction solution was stirred at room temperature for 2 hours. Water was added to the reaction product and stirred at room temperature for 2 hours. The resulting solid was collected by filtration, washed with water, and dried to obtain the title compound (8.72 g, 82.3 %).

### [Process 2] Preparation of methyl 4-chloro-2-(phenylamino)benzoate

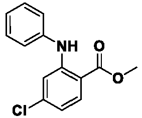

Methyl 2-amino-4-chlorobenzoate (8.72 g, 47.0 mmol) synthesized in [Process 1] above, bromobenzene (4.99 mL, 47.0 mmol), cesium carbonate (23.20 g, 70.5 mmol), palladium acetate (215 mg, 0.94 mmol), and xantphos (1.09 g, 1.88 mmol) were added to toluene (88 mL), and after nitrogen gas substitution, the mixture was refluxed at 100 °C for 12 hours. After cooling the reaction product to room temperature, the solid was filtered and washed with dichloromethane. The filtrate was concentrated and purified by silica gel column chromatography to obtain the title compound (8.56 g, 69.57 %).

### [Process 3] Preparation of methyl 4-chloro-2-(N-phenylacetamido)benzoate

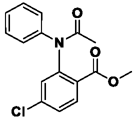

Methyl 4-chloro-2-(phenylamino)benzoate (7.56 g, 28.89 mmol) synthesized in [Process 2] above was dissolved in acetic acid (60 mL), followed by the addition of acetic anhydride (54.62 mL, 577.76 mmol) and was refluxed at 120 °C for 23 hours. The reaction product was cooled to room temperature, then poured into ice water and stirred for 30 minutes. The resulting solid was collected by filtration, washed with water and dried to obtain the title compound (8.32 g, 94.82 %), which was used without additional purification.

### [Process 4] Preparation of 7-chloro-4-hydroxy-1-phenylquinolin-2(1H)-one

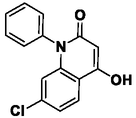

Tetrahydrofuran (125 mL) was added to methyl 4-chloro-2-(*N-*phenylacetamido)benzoate (8.32 g, 27.39 mmol) synthesized in [Process 3] above, and the reaction solution was cooled to -78 °C. A solution of potassium bis(trimethylsilyl)amide 1 M tetrahydrofuran was slowly added dropwise to the reaction solution for 1 hour. The reaction product was concentrated, water was added, and acidified with 6N hydrochloric acid. The resulting solid was collected by filtration and washed with excess water. The solid was stirred in diethyl ether for 30 minutes, collected by filtration, and dried to obtain the title compound (6.02 g, 80.89 %), which was used without additional purification.

### [Process 5] Preparation of 7-chloro-4-hydroxy-3-nitro-1-phenylquinolin-2(1H)-one

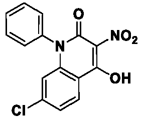

Acetic acid (100 mL), nitric acid (6.2 mL, 88.63 mmol), and sodium nitrite (312 mg, 4.43 mmol) were added to 7-chloro-4-hydroxy-1-phenylquinolin-2(1*H*)-one (6.02 g, 22.16 mmol) synthesized in [Process 4] above and stirred at room temperature for 2 hours. The reaction product was poured into ice water and stirred for 1 hour. The resulting solid was collected by filtration, washed with excess water and dried to obtain the title compound (6.5 g, 92.63 %).

### [Process 6] Preparation of 4,7-dichloro-3-nitro-1-phenylquinolin-2(1H)-one

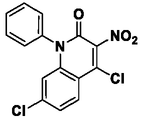

Phosphorus oxychloride (65 mL, 683.41 mmol) was added to 7-chloro-4-hydroxy-3-nitro-1-phenylquinolin-2(1*H*)-one (6.5 g, 20.52 mmol) synthesized in [Process 5] above. After cooling the reaction solution to 0 °C, triethylamine (6.5 mL, 46.17 mmol) was slowly added dropwise.

The mixture was then refluxed at 110 °C for 1 hour. The reaction product was cooled to room temperature, then poured into ice water and stirred for 1 hour. The resulting solid was collected by filtration, washed with excess water and dried to obtain the title compound (5.76 g, 83.67 %).

### [Process 7] Preparation of 7-chloro-4-(methylamino)-3-nitro-1-phenylquinolin-2(1H)-one

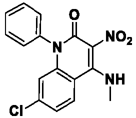

4,7-dichloro-3-nitro-1-phenylquinolin-2(1*H*)-one (5.76 g, 17.17 mmol) synthesized in [Process 6] above was dissolved in dichloromethane, followed by adding methylamine hydrochloride (1.42 g, 20.61 mmol) and triethylamine (7.26 mL, 51.52 mmol). The reaction solution was stirred at room temperature for 1 hour, concentrated, and then water was added and stirred for 1 hour. The resulting solid was collected by filtration, washed with excess water and dried to obtain the title compound (5.05 g, 89.19 %).

### [Process 8] Preparation of 3-amino-7-chloro-4-(methylamino)-1-phenylquinolin-2(1H)-one

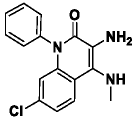

Raney-nickel (5.39 g, 45.95 mmol), dichloromethane (60 mL)/methanol (30 mL) were added to 7-chloro-4-(methylamino)-3-nitro-1-phenylquinolin-2(1*H*)-one (5.05 g, 15.32 mmol) synthesized in [Process 7] above and was fitted with a hydrogen balloon. The reaction solution was stirred at room temperature for 16 hours, filtered through celite, and purified by silica gel column chromatography to obtain the title compound (2.16 g, 47.05 %).

### [Process 9] Preparation of N-(7-chloro-4-(methylamino)-2-oxo-1-phenyl-1,2-dihydroquinolin-3-yl)-2-phenylacetamide

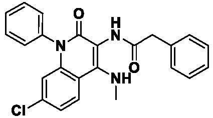

3-amino-7-chloro-4-(methylamino)-1-phenylquinolin-2(1*H*)-one (40 mg, 0.13 mmol) synthesized in [Process 8] above was dissolved in dichloromethane (1 mL), cooled to 0 °C, and triethylamine (21 µL, 0.15 mmol) and 2-phenylacetyl chloride (18 µL, 0.13 mmol) were slowly added dropwise. The reaction solution was stirred at room temperature for 1 hour, f and was subjected to extraction with dichloromethane by adding water. The separated organic layer was dried with anhydrous sodium sulfate and concentrated by vacuum distillation. Purification was performed using silica gel column chromatography to obtain the title compound (15.3 mg, 27.44 %).

### [Process 10] Preparation of 2-benzyl-7-chloro-1-methyl-5-phenyl-1,5-dihydro-4H-imidazo[4,5-c]quinolin-4-one

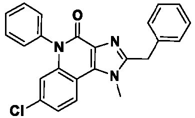

*N*-(7-chloro-4-(methylamino)-2-oxo-1-phenyl-1,2-dihydroquinolin-3-yl)-2-phenylacetamide (15 mg, 0.04 mmol) synthesized in [Process 9] above was dissolved in 1,4-dioxane (0.5 mL) and 2 N sodium hydroxide (0.5 mL) was added. The reaction solution was stirred at 120 °C for 2 hours, after completion of the reaction, the reaction solution was cooled to 0 °C and neutralized with 1 N hydrochloric acid. The resulting solid was collected by filtration, washed with excess water and dried to obtain the title compound (13.2 mg, 78.5 %).

### Example 2: 7-chloro-2-(4-methoxybenzyl)-1-methyl-5-phenyl-1,5-dihydro-4H-imidazo[4,5-c]quinolin-4-one

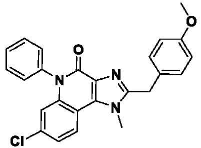

The same reaction was carried out using 2-(4-methoxyphenyl)acetyl chloride instead of 2-phenylacetyl chloride, in [Process 9] of Example 1, to obtain the title compound (8.7 mg, 15.17 %).

### Example 3: 7-chloro-2-(4-hydroxybenzyl)-1-methyl-5-phenyl-1,5-dihydro-4H-imidazo[4,5-c]quinolin-4-one

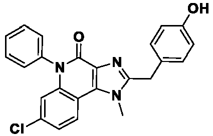

7-chloro-2-(4-methoxybenzyl)-1-methyl-5-phenyl-1,5-dihydro-4*H*-imidazo[4,5-*c*]quinolin-4-one [title compound of Example 2] (20 mg, 0.05 mmol) was dissolved in dichloromethane (0.5 mL), cooled to 0 °C, and boric acid tribromide (9 µL, 0.1 mmol) was slowly added dropwise. The reaction solution was stirred at room temperature for 1 hour, poured into ice water, followed by adding a saturated aqueous solution of sodium hydrogen carbonate and was subjected to extraction with dichloromethane. The separated organic layer was dried with anhydrous magnesium sulfate and concentrated by vacuum distillation. Then purified by silica gel column chromatography to obtain the title compound (3.4 mg, 17.18 %).

### Example 4: 7-chloro-2-(3-methoxybenzyl)-1-methyl-5-phenyl-1,5-dihydro-4H-imidazo[4,5-c]quinolin-4-one

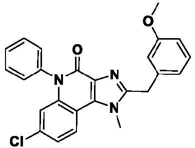

The same reaction was carried out up to [Process 10] using 2-(3-methoxyphenyl)acetyl chloride instead of 2-phenylacetyl chloride, in [Process 9] of Example 1, to obtain the title compound (22 mg, 30.68 %).

### Example 5: 7-chloro-2-(2-methoxybenzyl)-1-methyl-5-phenyl-1,5-dihydro-4H-imidazo[4,5-c]quinolin-4-one

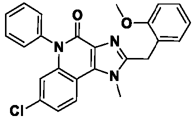

The same reaction was carried out up to [Process 10] using 2-(2-methoxyphenyl)acetyl chloride instead of 2-phenylacetyl chloride, in [Process 9] of Example 1, to obtain the title compound (15 mg, 20.92 %).

### Example 6: 7-chloro-2-(4-chlorobenzyl)-1-methyl-5-phenyl-1,5-dihydro-4H-imidazo[4,5-c]quinolin-4-one

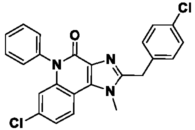

The same reaction was carried out up to [Process 10] using 2-(4-chlorophenyl)acetyl chloride instead of 2-phenylacetyl chloride, in [Process 9] of Example 1, to obtain the title compound (12.9 mg, 64.46 %).

### Example 7: 7-chloro-1-methyl-5-phenyl-2-(pyridin-4-ylmethyl)-1,5-dihydro-4H-imidazo[4,5-c]quinolin-4-one

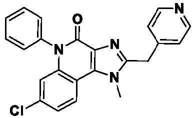

3-amino-7-chloro-4-(methylamino)-1-phenylquinolin-2(1*H*)*-*one (20 mg, 0.06 mmol) synthesized in [Process 8] of Example 1 was dissolved in *N,N*-dimethylformamide (1 mL), followed by adding 4-pyridyl acetic acid hydrochloride (12.4 mg, 0.07 mmol), 1-[bis(dimethylamino)methylene]-1*H*-1,2,3-triazolo[4,5-*b*]pyridinium 3-oxide hexafluorophosphate (HATU) (50.7 mg, 0.13 mmol), and diisopropylethylamine (43 mg, 0.33 mmol). The reaction solution was stirred at room temperature for 16 hours, and was subjected to extraction with ethyl acetate. The separated organic layer was dried with anhydrous sodium sulfate and concentrated by vacuum distillation. The same reaction was carried out up to [Process 10] without purification to obtain the title compound (8 mg, 21.50 %).

### Example 8: 7-chloro-2-(3,4-dimethoxybenzyl)-1-methyl-5-phenyl-1,5-dihydro-4H-imidazo[4,5-c]quinolin-4-one

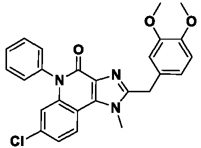

The same reaction was carried out up to [Process 10] using 2-(3,4-dimethoxyphenyl)acetyl chloride instead of 2-phenylacetyl chloride, in [Process 9] of Example 1, to obtain the title compound (37 mg, 46.59 %).

### Example 9: 7-chloro-1-methyl-2-phenethyl-5-phenyl-1,5-dihydro-4H-imidazo[4,5-c]quinolin-4-one

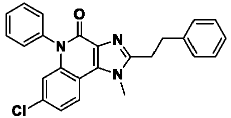

The same reaction was carried out up to [Process 10] using 3-phenylpropanoyl chloride instead of 2-phenylacetyl chloride, in [Process 9] of Example 1, to obtain the title compound (16.4 mg, 60.05 %).

### Example 10: 7-chloro-2-(4-methoxyphenyl)-1-methyl-5-(o-tolyl)-1,5-dihydro-4H-imidazo[4,5-c]quinolin-4-one

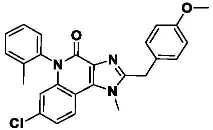

The same reaction was carried out up to [Process 10] using 1-bromo-2-methylbenzene instead of bromobenzene, in [Process 2] of Example 1, and 2-(4-methoxyphenyl)acetyl chloride instead of 2-phenylacetyl chloride, in [Process 9] of Example 1, to obtain the title compound (45.6 mg, 78.56 %).

### Example 11: 2-benzyl-7-chloro-5-phenyl-1,5-dihydro-4H-imidazo[4,5-c]quinolin-4-one

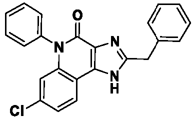

The same reaction was carried out, except that the compound was dissolved in tetrahydrofuran instead of dissolving in dichloromethane, and ammonia water was added instead of adding methylamine hydrochloride and triethylamine, to synthesize 4-amino-7-chloro-3-nitro-1-phenylquinolin-2(1*H*)-one, in [Process 7] of Example 1, to obtain the title compound (2 mg, 41.87 %).

According to an embodiment of the present disclosure, the compound of Formula 1 may be a compound selected from the group consisting of the compounds listed in [Table 1] below.

**[Table 1]**

| Compound No. | Structure | Nomenclature | MS[M+H]⁺ |
|---|---|---|---|
| | ¹H-NMR spectrum (300MHz) | | |
| 1 | 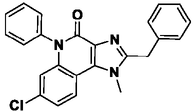 | 2-benzyl-7-chloro-1-methyl-5-phenyl-1,5-dihydro-4*H*-imidazo[4,5-*c*]quinolin-4-one | 400.11 |
| | ¹H-NMR spectrum (300 MHz, DMSO-d₆) *δ* 8.27(d, 1H), 7.68-7.57(m, 3H), 7.38-7.25(m, 8H), 6.49(d, 1H), 4.36(s, 2H), 4.05(s, 3H). | | |
| 2 | 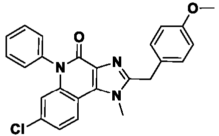 | 7-chloro-2-(4-methoxybenzyl)-1-methyl-5-phenyl-1,5-dihydro-4*H-*imidazo[4,5-*c*]quinolin-4-one | 430.12 |
| | ¹H-NMR spectrum (300 MHz, CDCl₃) *δ* 7.92(d, 1H), 7.64-7.54(m, 3H), 7.30-7.16(m, 5H), 6.85(d, 2H), 6.73(d, 1H), 4.35(s, 2H), 3.94(s, 3H), 3.78(s, 3H). | | |
| 3 | 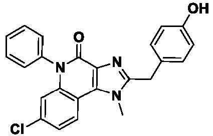 | 7-chloro-2-(4-hydroxybenzyl)-1-methyl-5-phenyl-1,5-dihydro-4*H-*imidazo[4,5-*c*]quinolin-4-one | 416.11 |
| | ¹H-NMR spectrum (300 MHz, DMSO-d₆) *δ* 9.30 (brs, 1H), 8.27(d, 1H), 7.69-7.59(m, 3H), 7.35-7.32(m, 3H), 7.07(d, 2H), 6.72(d, 2H), 6.50(d, 1H), 4.24(s, 2H), 4.04(s, 3H). | | |
| 4 | 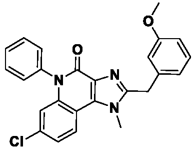 | 7-chloro-2-(3-methoxybenzyl)-1-methyl-5-phenyl-1,5-dihydro-4*H-*imidazo[4,5-*c*]quinolin-4-one | 430.12 |
| | ¹H-NMR spectrum (300 MHz, CDCl₃) *δ* 7.92(d, 1H), 7.64-7.55(m, 3H), 7.31-7.17(m, 4H), 6.86-6.79(m, 3H), 6.73(d, 1H), 4.39(s, 2H), 3.94(s, 3H), 3.77(s, 3H). | | |
| 5 | 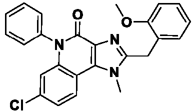 | 7-chloro-2-(2-methoxybenzyl)-1-methyl-5-phenyl-1,5-dihydro-4*H-*imidazo[4,5-*c*]quinolin-4-one | 430.12 |
| | ¹H-NMR spectrum (300 MHz, CDCl₃) *δ* 7.94(d, 1H), 7.64-7.54(m, 3H), 7.30-7.17(m, 4H), 7.08(d, 1H), 6.94-6.84(m, 2H), 6.72(s, 1H), 4.39(s, 2H), 3.95(s, 3H), 3.93(s, 3H). | | |
| 6 | 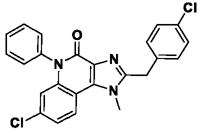 | 7-chloro-2-(4-chlorobenzyl)-1-methyl-5-phenyl-1,5-dihydro-4*H*-imidazo[4,5-*c*]quinolin-4-one | 434.07 |
| | ¹H-NMR spectrum (300 MHz, DMSO-d₆) *δ* 8.26(d, 1H), 7.64-7.57(m, 3H), 7.40-7.28(m, 7H), 6.48(d, 1H), 4.35(s, 2H), 4.04(s, 3H). | | |
| 7 | 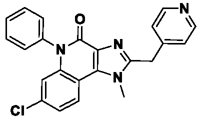 | 7-chloro-1-methyl-5-phenyl-2-(pyridin-4-ylmethyl)-1,5-dihydro-4*H-*imidazo[4,5-*c*]quinolin-4-one | 401.11 |
| | ¹H-NMR spectrum (300 MHz, DMSO-d₆) *δ* 8.51 (d, 2H), 8.28(d, 1H), 7.69-7.55(m, 3H), 7.36-7.26(m, 5H), 6.49(d, 1H), 4.40(s, 2H), 4.06(s, 3H). | | |
| 8 | 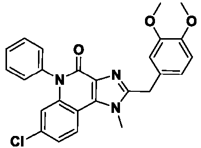 | 7-chloro-2-(3,4-dimethoxybenzyl)-1-methyl-5-phenyl-1,5-dihydro-4*H-*imidazo[4,5-*c*]quinolin-4-one | 460.13 |
| | ¹H-NMR spectrum (300 MHz, CDCl₃) *δ* 7.93(d, 1H), 7.65-7.55(m, 3H), 7.31-7.18(m, 3H), 6.81-6.74(m, 4H), 4.35(s, 2H), 3.96(s, 3H), 3.86(s, 3H), 3.82(s, 3H). | | |
| 9 | 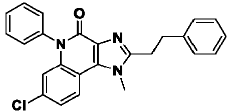 | 7-chloro-1-methyl-2-phenethyl-5-phenyl-1,5-dihydro-4*H*-imidazo[4,5-*c*]quinolin-4-one | 414.13 |
| | ¹H-NMR spectrum (300 MHz, CDCl₃) *δ* 7.92(d, 1H), 7.63-7.53(m, 3H), 7.31-7.17(m, 8H), 6.72(d, 1H), 3.80(s, 3H), 3.21(s, 4H). | | |
| 10 | 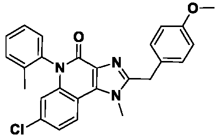 | 7-chloro-2-(4-methoxyphenyl)-1-methyl-5-(*o*-tolyl)-1,5-dihydro-4*H-*imidazo[4,5-*c*]quinolin-4-one | 444.14 |
| | ¹H-NMR spectrum (300 MHz, CDCl₃) *δ* 7.93(d, 1H), 7.42-7.35(m, 3H), 7.19-7.11(m, 4H), 6.83(d, 2H), 6.61(s, 1H), 4.31(s, 2H), 3.92(s, 3H), 3.76(s, 3H), 2.00(s, 3H). | | |
| 11 | 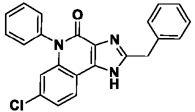 | 2-benzyl-7-chloro-5-phenyl-1,5-dihydro-4*H*-imidazo[4,5-*c*]quinolin-4-one | 386.10 |
| | ¹H-NMR spectrum (300 MHz, CDCl₃) *δ* 12.43(brs, 1H), 8.31 (d, 1H), 7.64-7.54(m, 3H), 7.33-7.29(m, 3H), 7.26-7.15(m, 5H), 6.75(d, 1H), 4.12(s, 2H). | | |

### <Synthesis Example 2>

### Example 12: 2-((1H-imidazol-1-yl)methyl)-7-chloro-1-methyl-5-phenyl-1,5-dihydro-4H-imidazo[4,5-c]quinolin-4-one

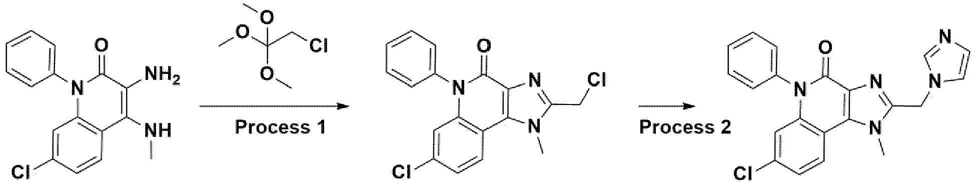

### [Process 11 Preparation of 7-chloro-2-(chloromethyl)-1-methyl-5-phenyl-1,5-dihydro-4H-imidazo[4,5-c]quinolin-4-one

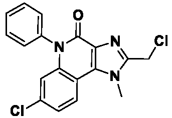

2-chloro-1,1,1-trimethoxyethane (0.25 mL, 1.84 mmol), *p*-toluenesulfonic acid monohydrate (8.25 mg, 0.04 mmol) were added to 3-amino-7-chloro-4-(methylamino)-1-phenylquinolin-2(1*H*)-one synthesized in [Process 8] of Example 1 and stirred at 100 °C for 1 hour. The reaction solution was concentrated by cooling to room temperature and stirred in diethyl ether for 1 hour. The resulting solid was collected by filtration, washed with diethyl ether to obtain the title compound (152.6 mg, 98.23 %), which was used without additional purification.

### [Process 2] Preparation of 2-((1H-imidazol-1-yl)methyl)-7-chloro-1-methyl-5-phenyl-1,5-dihydro-4H-imidazo[4,5-c]quinolin-4-one

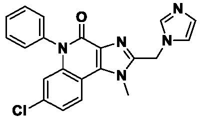

Imidazole (7.7 mg, 0.11 mmol) was dissolved in *N,N*-dimethylformamide (0.2 mL), cooled to 0 °C, and sodium hydride (6.7 mg, 0.17 mmol) was added fractionally under nitrogen. After stirring for 30 minutes, 7-chloro-2-(chloromethyl)-1-methyl-5-phenyl-1,5-dihydro-4*H*-imidazo[4,5-*c*]quinolin-4-one (20 mg, 0.06 mmol) synthesized in [Process 1] was slowly added dropwise to the mixture above dissolved in *N,N*-dimethylformamide (0.3 mL). The reaction solution was stirred at room temperature for 30 minutes, followed by adding ice water and was subjected to extraction with ethyl acetate. The obtained organic layer was dried with anhydrous magnesium sulfate and vacuum distilled. The concentrated reactant was purified by silica gel column chromatography to obtain the title compound (4.4 mg, 20.22 %).

### Example 13: 7-chloro-2-((3-methoxypyrrolidin-1-yl)methyl)-1-methyl-5-phenyl-1,5-dihydro-4H-imidazo[4,5-c]quinolin-4-one

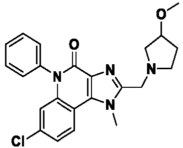

The same reaction was carried out, except for the use of 3-methoxypyrrolidine instead of imidazole, in [Process 2] of Example 12, to obtain the title compound (30.5 mg, 51.67 %).

### Example 14: 7-chloro-2-((3-hydroxypyrrolidin-1-yl)methyl)-1-methyl-5-phenyl-1,5-dihydro-4H-imidazo[4,5-c]quinolin-4-one

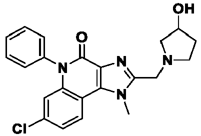

The same reaction was carried out, except for the use of 7-chloro-2-((3-methoxypyrrolidin-1-yl)methyl)-1-methyl-5-phenyl-1,5-dihydro-4H-imidazo[4,5-c]quinolin-4-one [the title compound of Example 13] instead of 7-chloro-2-(4-methoxybenzyl)-1-methyl-5-phenyl-1,5-dihydro-4*H*-imidazo[4,5-*c*]quinolin-4-one, in Example 3, to obtain the title compound (12.4 mg, 64.13 %).

### <Synthesis Example 3>

### Example 15: 2-(4-methoxybenzyl)-1-methyl-5-phenyl-7-((2,2,2-trifluoroethyl)amino)-1,5-dihydro-4H-imidazo[4,5-c][1,8]naphthyridin-4-one

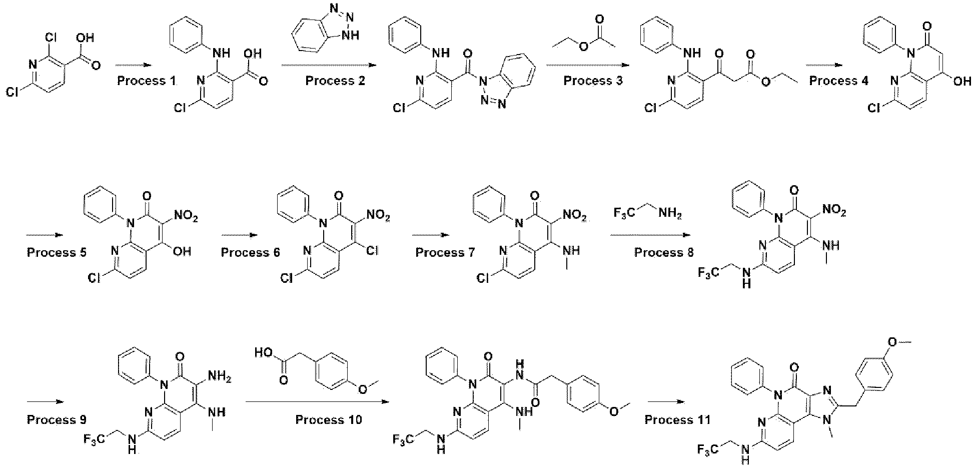

### [Process 11 Preparation of 6-chloro-2-(phenylamino)nicotinic acid

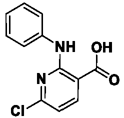

Aniline (6.15 g, 65.3 mmol) was diluted with tetrahydrofuran (60 mL) and the reaction solution was cooled to -75 °C, then a 1 M lithium bis(trimethylsilyl)amide tetrahydrofuran solution (16.7 g, 100 mmol) was added dropwise and stirred for 1 hour. To this mixture 2,6-dichloronicotinic acid (6.00 g, 31.3 mmol) was added and the reaction solution was stirred at room temperature for 16 hours. After completion of the reaction, the mixture was diluted with ethyl acetate and washed with 1 N aqueous hydrochloric acid solution and saturated brine. The separated organic layer was dried with anhydrous sodium sulfate, followed by vacuum filtration and vacuum distillation. The obtained residue was separated by silica gel column chromatography to obtain the title compound (3.7 g, 48 %).

### [Process 2] Preparation of (1H-benzo[d][1,2,3]triazol-1-yl)(6-chloro-2-(phenylamino)pyridin-3-yl)methanone

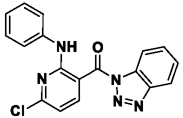

6-chloro-2-(phenylamino)nicotinic acid (3.70 g, 14.9 mmol), 1,2,3-benzotriazole (1.81 g, 14.9 mmol) and 1-ethyl-3-(3-dimethylaminopropyl)carbodiimide (2.85 g, 14.9 mmol) synthesized in [Process 1] above were sequentially added to dichloromethane (13 mL), and the reaction solution was stirred at room temperature for 16 hours. After completion of the reaction, the mixture was diluted with dichloromethane and washed with distilled water. The separated organic layer was dried with anhydrous sodium sulfate, followed by vacuum filtration and vacuum distillation. The obtained residue was separated by silica gel column chromatography to obtain the title compound (3.83 g, 74 %).

### [Process 3] Preparation of ethyl 3-(6-chloro-2-(phenylamino)pyridin-3-yl)-3-oxopropanoate

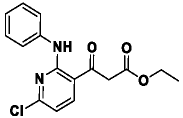

Ethyl acetate (1.45 g, 16.5 mmol) was diluted with tetrahydrofuran (30 mL), cooled to -78 °C and a 2 N lithium diisopropylamide tetrahydrofuran/heptane/benzene solution (12.1 mL, 24.2 mmol) was slowly added dropwise. The reaction solution was stirred at - 78 °C for 1 hour, then a dilute solution of (1*H*-benzo[*d*][1,2,3]triazol-1-yl)(6-chloro-2-(phenylamino)pyridin-3-yl)methanone (3.83 g, 11.0 mmol) synthesized in [Process 2] above in tetrahydrofuran (10 mL) was added dropwise and stirred at the same temperature for 2 hours, followed by stirring at room temperature for 16 hours. After completion of the reaction, the mixture was diluted with ethyl acetate and washed with 1 N aqueous hydrochloric acid solution. The separated organic layer was dried with anhydrous sodium sulfate, followed by vacuum filtration and vacuum distillation. The obtained residue was separated by silica gel column chromatography to obtain the title compound (1.3 g, 29 %).

### [Process 4] Preparation of 7-chloro-4-hydroxy-1-phenyl-1,8-naphthyridin-2(1H)-one

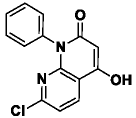

*N,N*-diisopropylethylamine (4.62 mL, 26.5 mmol) and 1,8-diazabicyclo[5,4,0]undec-7-ene (0.61 mL, 4.08 mmol) were added to ethyl 3-(6-chloro-2-(phenylamino)pyridin-3-yl)-3-oxopropanoate (1.30 g, 4.08 mmol) synthesized in [Process 3] above and stirred at 120 °C for 2 hours. After completion of the reaction, the resulting reaction mixture was cooled to room temperature, diluted with ethyl acetate and washed with an 1 N aqueous hydrochloric acid solution. The separated organic layer was dried with anhydrous sodium sulfate, followed by vacuum filtration and vacuum distillation. The obtained residue was separated by silica gel column chromatography to obtain the title compound (1.1 g, 98 %).

### [Process 5] Preparation of 7-chloro-4-hydroxy-3-nitro-1-phenyl-1,8-naphthyridin-2(1H)-one

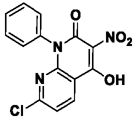

The same method was carried out, except for the use of 7-chloro-4-hydroxy-1-phenyl-1,8-naphthyridin-2(1*H*)-one synthesized in [Process 4] above instead of 7-chloro-4-hydroxy-1-phenylquinolin-2(1*H*)-one in [Process 5] of Example 1, to obtain the title compound (450 mg, 39%).

### [Process 6] Preparation of 4,7-dichloro-3-nitro-1-phenyl-1,8-naphthyridin-2(1H)-one

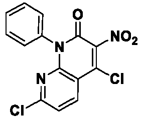

The same method was carried out, except for the use of 7-chloro-4-hydroxy-3-nitro-1-phenyl-1,8-naphthyridin-2(1*H*)-one synthesized in [Process 5] above instead of 7-chloro-4-hydroxy-3-nitro-1-phenylquinolin-2(1*H*)-one in [Process 6] of Example 1, to obtain the title compound (460 mg, 97%).

### [Process 7] Preparation of 7-chloro-4-(methylamino)-3-nitro-1-phenyl-1,8-naphthyridin-2(1H)-one

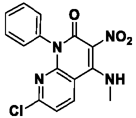

The same reaction was carried out, except for the use of 4,7-dichloro-3-nitro-1-phenyl-1,8-naphthyridin-2(1*H*)-one synthesized in [Process 6] above instead of 4,7-dichloro-3-nitro-1-phenylquinolin-2(1*H*)-one in [Process 7] of Example 1, to obtain the title compound (347 mg, 80.15%).

### [Process 8] Preparation of 4-(methylamino)-3-nitro-1-phenyl-7-((2,2,2-trifluoroethyl)amino)-1,8-naphthyridin-2(1H)-one

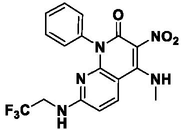

7-chloro-4-(methylamino)-3-nitro-1-phenyl-1,8-naphthyridin-2(1*H*)-one (160 mg, 0.48 mmol) synthesized in [Process 7] above was dissolved in *N*-methylpyrrolidone (2 mL), followed by adding 2,2,2-trifluoroethylamine (0.78 mL, 9.62 mmol), *N,N-*diisopropylethylamine (0.42 mL, 2.40 mmol), and the mixture was stirred for 3 hours at 160 °C in a microwave reactor. After cooling to room temperature, water was added to the reaction product and was subjected to extraction with ethyl acetate. The obtained organic layer was dried with anhydrous sodium sulfate and vacuum distilled.

### [Process 9] Preparation of 3-amino-4-(methylamino)-1-phenyl-7-((2,2,2-trifluoroethyl)amino)-1,8-naphthyridin-2(1H)-one

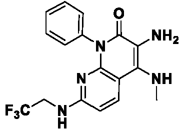

The same reaction was carried out except for the use of 4-(methylamino)-3-nitro-1-phenyl-7-((2,2,2-trifluoroethyl)amino)-1,8-naphthyridin-2(1*H*)-one synthesized in [Process 8] above instead of 7-chloro-4-(methylamino)-3-nitro-1-phenylquinolin-2(1*H*)-one, in [Process 8] of Example 1, to obtain the title compound (76 mg, 58.35 %).

### [Process 10] Preparation of 2-(4-methoxyphenyl)-N-(4-(methylamino)-2-oxo-1-phenyl-7-((2,2,2-trifluoroethyl)amino)-1,2-dihydro-1,8-naphthyridin-3-yl)acetamide

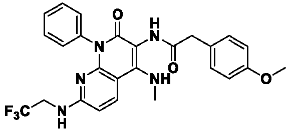

3-amino-4-(methylamino)-1-phenyl-7-((2,2,2-trifluoroethyl)amino)-1,8-naphthyridin-2(1*H*)-one (76 mg, 0.21 mmol) synthesized in [Process 9] above was dissolved in *N,N*-dimethylformamide (1 mL), followed by adding 2-(4-methoxyphenyl)acetic acid (38.24 mg, 0.23 mmol), 1-[bis(dimethylamino)methylene]-1*H-*1,2,3-triazolo[4,5-*b*]pyridinium 3-oxide hexafluorophosphate (HATU) (159.07 mg, 0.42 mmol), diisopropylethylamine (135.18 mg, 1.05 mmol). The reaction solution was stirred at room temperature for 5 hours, and was subjected to extraction with ethyl acetate by adding water. The separated organic layer was dried with anhydrous sodium sulfate and concentrated by vacuum distillation to obtain the title compound (104 mg, 97.19 %), which was proceeded to the next reaction [Process 11] without additional purification.

### [Process 11] Preparation of 2-(4-methoxybenzyl)-1-methyl-5-phenyl-7-((2,2,2-trifluoroethyl)amino)-1,5-dihydro-4H-imidazo[4,5-c][1,8]naphthyridin-4-one

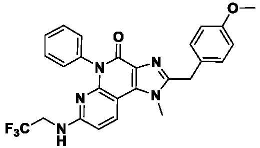

The same reaction was carried out, except for the use of 2-(4-methoxyphenyl)-*N-*(4-(methylamino)-2-oxo-1-phenyl-7-((2,2,2-trifluoroethyl)amino)-1,2-dihydro-1,8-naphthyridin-3-yl)acetamide synthesized in [Process 10] above instead of *N*-(7-chloro-4-(methylamino)-2-oxo-1-phenyl-1,2-dihydroquinolin-3-yl)-2-phenylacetamide, in [Process 10] of Example 1, to obtain the title compound (6.2 mg, 5.49 %).

### <Synthesis Example 4>

### Example 16: 7-chloro-5-(4-chloro-3-fluorophenyl)-2-(4-methoxybenzyl)-1-methyl-1,5-dihydro-4H-imidazo[4,5-c]quinolin-4-one

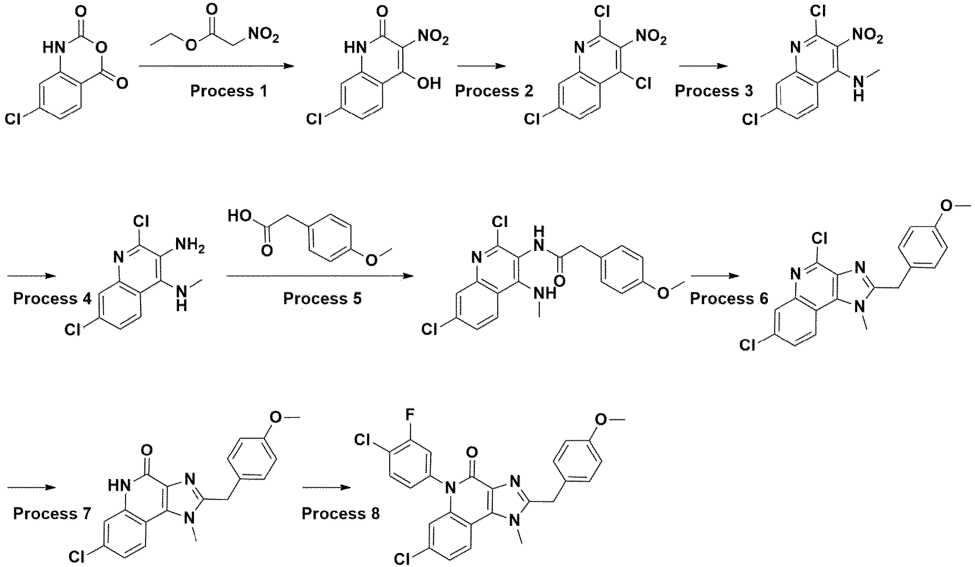

### [Process 11 Preparation of 7-chloro-4-hydroxy-3-nitroquinolin-2(1H)-one

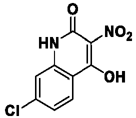

7-chloro-2*H*-benzo[*d*][1,3]oxazine-2,4(1*H*)-dione (12.0 g, 60.7 mmol), ethyl nitroacetate (12.5 g, 91.1 mmol) and triethylamine (12.7 mL, 91.1 mmol) were sequentially added to tetrahydrofuran (120 mL), and the reaction solution was stirred at 55 °C for 16 hours. After completion of the reaction, the mixture was cooled to room temperature and the solvent was distilled under decompression. The obtained residue was diluted with water and washed with diethyl ether to remove the organic layer, and the remaining aqueous layer was adjusted to pH 2 to 3 with an 1 N aqueous hydrochloric acid solution, and the resulting solid was collected by filtration, washed with methanol and excess water, and dried to obtain the title compound (5.6 g, 38 %).

### [Process 2] Preparation of 2,4,7-trichloro-3-nitroquinoline

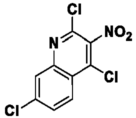

Phosphoryl chloride (10.8 mL, 116 mmol) was added to 7-chloro-4-hydroxy-3-nitroquinolin-2(1*H*)-one (995 mg, 4.14 mmol) synthesized in [Process 1] above, the reaction solution was cooled to 0 °C, and triethylamine (0.98 mL, 7.04 mmol) was slowly added dropwise. The reaction mixture was refluxed at 100 °C for 4 hours and the reaction solution was cooled to room temperature, poured into ice water and was subjected to 1st extraction with ethyl acetate. After adjusting the pH to 7 to 8 with saturated sodium bicarbonate solution, the mixture was subjected to 2nd extraction with dichloromethane. The obtained organic layer was dried with anhydrous sodium sulfate, followed by vacuum filtration and vacuum distillation to obtain the title compound (1.15 g, 100 %).

### [Process 3] Preparation of 2,7-dichloro-N-methyl-3-nitroquinolin-4-amine

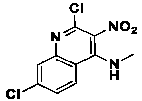

The same method was carried out, except for the use of 2,4,7-trichloro-3-nitroquinoline synthesized in [Process 2] above instead of 4,7-dichloro-3-nitro-1-phenylquinolin-2(1*H*)-one in [Process 7] of Example 1, to obtain the title compound (740 mg, 64 %).

### [Process 4] Preparation of 2,7-dichloro-N4-methylquinolin-3,4-diamine

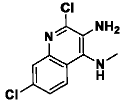

The same method was carried out ,except for the use of 2,7-dichloro-*N*-methyl-3-nitroquinolin-4-amine synthesized in [Process 3] above instead of 7-chloro-4-(methylamino)-3-nitro-1-phenylquinolin-2(1*H*)-one in [Process 8] of Example 1, to obtain the title compound (470 mg, 71 %).

### [Process 5] Preparation of N-(2,7-dichloro-4-(methylamino)quinolin-3-yl)-2-(4-methoxyphenylacetamide

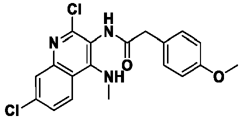

2,7-dichloro-*N*4-methylquinolin-3,4-diamine (530 mg, 2.19 mmol) synthesized in [Process 4] above, 2-(4-methoxyphenyl)acetic acid (400 mg, 2.41 mmol) and 1-[bis(dimethylamino)methylene]-1*H*-1,2,3-triazolo[4,5-b]pyridinium 3-oxide hexafluorophosphate (1.25 g, 3.28 mmol) were diluted with *N,N*-dimethylformamide (5 mL), then *N,N*-diisopropylethylamine (849 mg, 6.57 mmol) was added and the mixture was stirred at room temperature for 16 hours. After completion of the reaction, the reaction mixture was diluted with ethyl acetate and washed with water. The separated organic layer was vacuum distilled and the obtained residue was dissolved with hexane:ethyl acetate (4:1 (volume ratio), 20 mL) mixed solvent and the resulting solid was filtered to obtain the title compound (570 mg, 67 %).

### [Process 6] Preparation of 4,7-dichloro-2-(4-methoxybenzyl)-1-methyl-1H-imidazo[4,5-c]quinoline

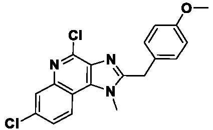

*N*-(2,7-dichloro-4-(methylamino)quinolin-3-yl)-2-(4-methoxyphenyl)acetamide (520 mg, 1.40 mmol) synthesized in [Process 5] above was diluted with 1,4-dioxane (9.7 mL) and water (9.7 mL), then sodium hydroxide (520 mg, 1.40 mmol) was added and the above mixture was stirred at 100 °C for 1 hour. After completion of the reaction, the reaction solution was cooled to room temperature and adjusted to pH 7 to 8 with an 1 N aqueous hydrochloric acid solution, and the solvent was removed by vacuum distillation. The resulting solid from the addition of saturated sodium bicarbonate solution was collected by filtration, washed with water, and dried to obtain the title compound (520 mg, 96 %).

### [Process 7] Preparation of 7-chloro-2-(4-methoxybenzyl)-1-methyl-1,5-dihydro-4H-imidazo[4,5-c]quinolin-4-one

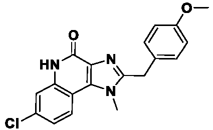

5 N aqueous hydrochloric acid solution (7 mL) and ethanol (17 mL) were added to 4,7-dichloro-2-(4-methoxybenzyl)-1-methyl-1*H*-imidazo[4,5-c]quinoline (520 mg, 1.40 mmol) synthesized in [Process 6] above, and the reaction solution was stirred at 100 °C for 4 hours. After completion of the reaction, the reaction solution was cooled to room temperature and adjusted to pH 7 to 8 with a saturated aqueous solution of sodium bicarbonate and water, and stirred for 10 minutes. The resulting solid was collected by filtration, washed with water, and dried to obtain the title compound (440 mg, 89 %).

### [Process 8] Preparation of 7-chloro-5-(4-chloro-3-fluorophenyl)-2-(4-methoxybenzyl)-1-methyl-1,5-dihydro-4H-imidazo[4,5-c]quinolin-4-one

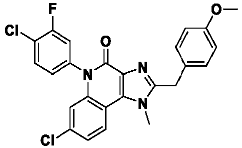

7-chloro-2-(4-methoxybenzyl)-1-methyl-1,5-dihydro-4*H*-imidazo[4,5-*c*]quinolin-4-one (40 mg, 0.11 mmol) synthesized in [Process 7] above was dissolved in dimethyl sulfoxide (1 mL), followed by adding 4-chloro-3-fluorophenylboronic acid (40 mg, 0.23 mmol), pyridine (0.04 mL, 0.45 mmol), molecular sieve (4 Å), and cobalt(II) acetate (42 mg, 0.23 mmol), and the mixture was stirred in an open vial at 80 °C for 16 hours. After cooling the reaction product to room temperature, water was added to the reaction product and was subjected to extraction with ethyl acetate. The obtained organic layer was dried with anhydrous sodium sulfate and vacuum distilled. Purification was performed using silica gel column chromatography to obtain the title compound (2.7 mg, 4.95 %).

### Example 17: 7-chloro-5-(3-chloro-4-fluorophenyl)-2-(4-methoxybenzyl)-1-methyl-1,5-dihydro-4H-imidazo[4,5-c]quinolin-4-one

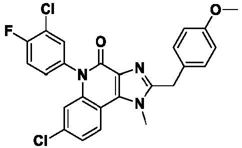

The same reaction was carried out using 3-chloro-4-fluorophenylboronic acid instead of 4-chloro-3-fluorophenylboronic acid, in [Process 8] of Example 16, to obtain the title compound (2.6 mg, 4.77 %).

### <Synthesis Example 5>

### Example 18: 7-chloro-2-((4-methoxyphenyl)thio)-1-methyl-5-phenyl-1,5-dihydro-4H-imidazo[4,5-c]quinolin-4-one

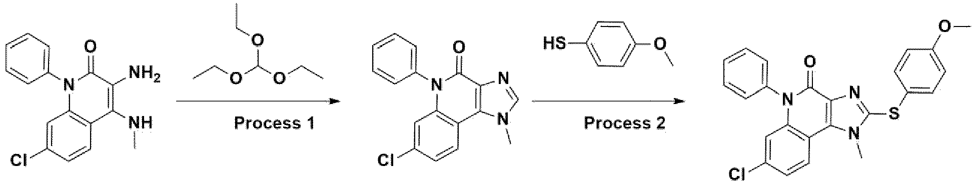

### [Process 11 Preparation of 7-chloro-1-methyl-5-phenyl-1,5-dihydro-4H-imidazo[4,5-c]quinolin-4-one

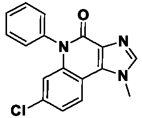

3-amino-7-chloro-4-(methylamino)-1-phenylquinolin-2(1*H*)-one (300 mg, 1.00 mmol) synthesized in [Process 8] of Example 1 was dissolved in triethyl orthoformate (3.4 mL, 20.02 mmol) and stirred at 120 °C for 2 hours. After completion of the reaction, the reaction solution was cooled to room temperature and the resulting solid was collected by filtration, washed with ethyl acetate and dried to obtain the title compound (265 mg, 85.5 %).

### [Process 2] Preparation of 7-chloro-2-((4-methoxyphenyl)thio)-1-methyl-5-phenyl-1,5-dihydro-4H-imidazo[4,5-c]quinolin-4-one

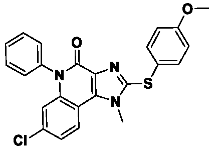

7-chloro-1-methyl-5-phenyl-1,5-dihydro-4*H*-imidazo[4,5-c]quinolin-4-one (100 mg, 0.32 mmol) synthesized in [Process 1] above was dissolved in dimethylformamide (3 mL), followed by adding 4-methoxybenzenthiol (67.9 mg, 0.48 mmol), copper(I) chloride (32.3 mg, 0.32 mmol), 1,10-phenanthroline (59.4 mg, 0.32 mmol) and sodium carbonate (86.4 mg, 0.81 mmol). The reaction solution was stirred at 140 °C for 3 days, and after completion of the reaction, the reaction solution was cooled to room temperature, followed by adding water and was subjected to extraction with ethyl acetate. The separated organic layer was dried with magnesium sulfate and concentrated by vacuum distillation. Then purified by silica gel column chromatography and crystallized from methanol to obtain the title compound (29 mg, 20.0 %).

### Example 19: 7-chloro-2-((4-methoxyphenyl)sulfonyl)-1-methyl-5-phenyl-1,5-dihydro-4H-imidazo[4,5-c]quinolin-4-one

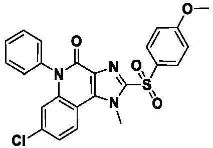

### Example 20: 7-chloro-2-((4-methoxyphenyl)sulfinyl)-1-methyl-5-phenyl-1,5-dihydro-4H-imidazo[4,5-c]quinolin-4-one

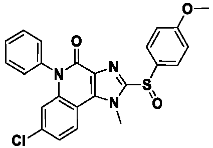

7-chloro-2-((4-methoxyphenyl)thio)-1-methyl-5-phenyl-1,5-dihydro-4*H-*imidazo[4,5-*c*]quinolin-4-one (25 mg, 0.06 mmol) synthesized in Example 18 above was dissolved in dichloromethane (0.56 mL), cooled to 0 °C, followed by slowly adding 3-chloroperoxycarboxylic acid (19 mg, 0.08 mmol). The reaction solution was stirred at 0 °C for 30 minutes, and after completion of the reaction, a saturated aqueous solution of sodium thiosulfate was added and was subjected to extraction with dichloromethane. The separated organic layer was dried with anhydrous magnesium sulfate and concentrated by vacuum distillation. Then purified by silica gel column chromatography to each obtain [the title compound of Example 19] 7-chloro-2-((4-methoxyphenyl)sulfonyl)-1-methyl-5-phenyl-1,5-dihydro-4*H*-imidazo[4,5-c]quinolin-4-one (15 mg, 56.0 %) and [the title compound of Example 20] 7-chloro-2-((4-methoxyphenyl)sulfinyl)-1-methyl-5-phenyl-1,5-dihydro-4*H*-imidazo[4,5-*c*]quinolin-4-one (8 mg, 25.9 %).

According to an embodiment of the present disclosure, the compound of Formula 1 may be a compound selected from the group consisting of the compounds listed in [Table 2] below.

**[Table 2]**

| Compound No. | Structure | Nomenclature | MS[M+H]⁺ |
|---|---|---|---|
| | ¹H-NMR spectrum (300MHz) | | |
| 12 | 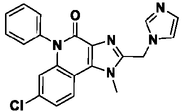 | 2-((1*H*-imidazol-1-yl)methyl)-7-chloro-1-methyl-5-phenyl-1,5-dihydro-4*H*-imidazo[4,5-c]quinolin-4-one | 390.11 |
| | ¹H-NMR spectrum (300 MHz, CDCl₃) *δ* 7.94(d, 1H), 7.66-7.56(m, 4H), 7.30-7.22(m, 3H), 7.12(s, 1H), 7.03(s, 1H), 6.76(d, 1H), 5.52(s, 2H), 4.00(s, 3H). | | |
| 13 | 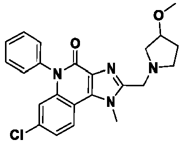 | 7-chloro-2-((3-methoxypyrrolidin-1-yl)methyl)-1-methyl-5-phenyl-1,5-dihydro-4*H*-imidazo[4,5-*c*]quinolin-4-one | 423.15 |
| | ¹H-NMR spectrum (300 MHz, CDCl₃) *δ* 8.03(d, 1H), 7.64-7.53(m, 3H), 7.29-7.21 (m, 3H), 6.72(d, 1H), 4.26(s, 3H), 3.98(s, 2H), 3.98-3.90(m, 1H), 3.26(s, 3H), 2.87-2.85(m, 1H), 2.73(d, 2H), 7.55-7.47(m, 1H), 2.16-2.06(m, 1H), 1.87-1.76(m, 1H) | | |
| 14 | 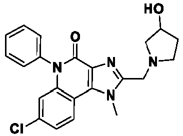 | 7-chloro-2-((3-hydroxypyrrolidin-1-yl)methyl)-1-methyl-5-phenyl-1,5-dihydro-4*H*-imidazo[4,5-*c*]quinolin-4-one | 409.14 |
| | ¹H-NMR spectrum (300 MHz, DMSO-d₆) *δ* 8.03(d, 1H), 7.64-7.51 (m, 3H), 7.29-7.21(m, 3H), 6.73(d, 1H), 4.41-4.38(m, 1H), 4.25(s, 3H), 4.02(s, 2H), 3.00-2.94(m, 1H), 2.79-2.76(m, 1H), 2.72-2.67(m, 1H), 2.53-2.45(m, 1H), 2.24-2.17(m, 1H), 1.81-1.77(m, 1H) | | |
| 15 | 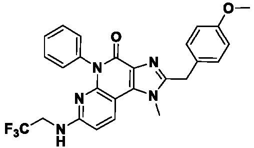 | 2-(4-methoxybenzyl)-1-methyl-5-phenyl-7-((2,2,2-trifluoroethyl)amino)-1,5-dihydro-4*H-*imidazo[4,5-*c*][1,8]naphthyridin-4-one | 494.17 |
| | ¹H-NMR spectrum (300 MHz, DMSO-d₆) *δ* 8.27(d, 1H), 7.54-7.40(m, 4H), 7.20-7.16(m, 4H), 6.87(d, 2H), 6.52(d, 1H), 4.22(s, 2H), 3.91(s, 3H), 3.71(s, 3H), 3.65-3.60(m, 2H). | | |
| 16 | 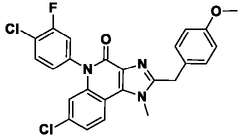 | 7-chloro-5-(4-chloro-3-fluorophenyl)-2-(4-methoxybenzyl)-1-methyl-1,5-dihydro-4*H*-imidazo[4,5-*c*]quinolin-4-one | 482.08 |
| | ¹H-NMR spectrum (300 MHz, CDCl₃) *δ* 7.93(d, 1H), 7.66(t, 1H), 7.26-7.13(m, 4H), 7.09-7.05(q, 1H), 6.84(d, 2H), 6.75(d, 1H), 4.34(s, 2H), 3.94(s, 3H), 3.78(s, 3H). | | |
| 17 | 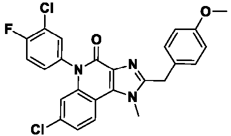 | 7-chloro-5-(3-chloro-4-fluorophenyl)-2-(4-methoxybenzyl)-1-methyl-1,5-dihydro-4*H*-imidazo[4,5-c]quinolin-4-one | 482.08 |
| | ¹H-NMR spectrum (300 MHz, CDCl₃) *δ* 7.93(d, 1H), 7.42-7.37(m, 2H), 7.25-7.16(m, 4H), 6.84(d, 2H), 6.73(d, 1H), 4.34(s, 2H), 3.94(s, 3H), 3.78(s, 3H). | | |
| 18 | 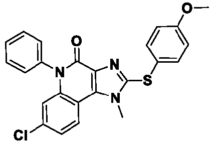 | 7-chloro-2-((4-methoxyphenyl)thio)-1-methyl-5-phenyl-1,5-dihydro-4*H-*imidazo[4,5-*c*]quinolin-4-one | 448.08 |
| | ¹H-NMR spectrum (300 MHz, DMSO) *δ* 8.31 (d, 1H), 7.69-7.59(m, 3H), 7.46-7.42(m, 2H), 7.40-7.33(m, 3H), 1.0₁₋₆.98(m, 2H), 6.50(d, 1H), 4.21(s, 3H), 3.76(s, 3H). | | |
| 19 | 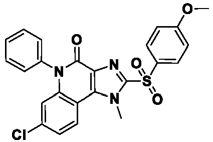 | 7-chloro-2-((4-methoxyphenyl)sulfonyl)-1-methyl-5-phenyl-1,5-dihydro-4*H*-imidazo[4,5-*c*]quinolin-4-one | 480.07 |
| | ¹H-NMR spectrum (300 MHz, CDCl₃) *δ* 8.09-8.04(m, 3H), 7.63-7.54(m, 3H), 7.29-7.19(m, 3H), 7.08-7.04(m, 2H), 6.74(d, 1H), 4.57(s, 3H), 3.89(s, 3H). | | |
| 20 | 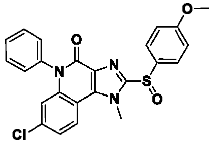 | 7-chloro-2-((4-methoxyphenyl)sulfinyl)-1-methyl-5-phenyl-1,5-dihydro-4*H*-imidazo[4,5-*c*]quinolin-4-one | 464.08 |
| | ¹H-NMR spectrum (300 MHz, CDCl₃) *δ* 7.94(d, 1H), 7.71-7.54(m, 5H), 7.29-7.20(m, 3H), 7.07-7.02(m, 2H), 6.74(d, 1H), 4.30(s, 3H), 3.85(s, 3H). | | |

### <Synthesis Example 6>

### [Intermediate 6-1] 2-(4-methoxybenzyl)-4,4,5,5-tetramethyl-1,3,2-dioxaborolane

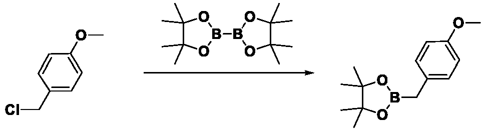

Bis(pinacolato)diboron (1.99 g, 7.66 mmol), potassium carbonate (2.66 g, 19.2 mmol), tetrakis(triphenylphosphine)palladium(0) (369 mg, 0.32 mmol) were sequentially added to anhydrous 1,4-dioxane, and after argon gas substitution, 1-(chloromethyl)-4-methoxybenzene (1.02 g, 6.38 mmol) was added, and the mixture was refluxed at 120 °C for 18 hours. After the reaction product was cooled to room temperature, the solid was filtered and washed with dichloromethane. The filtrate was concentrated and purified by silica gel column chromatography to obtain the title compound (725 mg, 45.76 %).

### [Intermediate 6-2] ethyl 5-bromo-2-(4-methoxybenzyl)-1-methyl-1H-imidazole-4-carboxylate

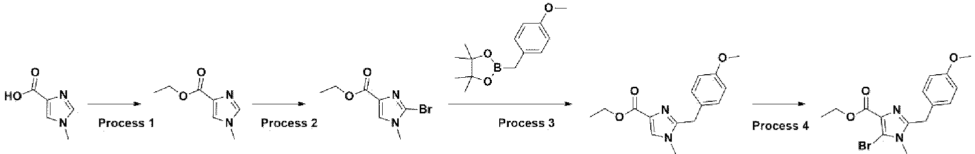

### [Process 11 Preparation of ethyl 1-methyl-1H-imidazole-4-carboxylate

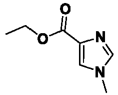

1-,ethylimidazole-4-carboxylic acid (10 g, 77.71 mmol) was dissolved in ethanol (100 mL), cool to 0 °C and thionyl chloride (20 mL, 77.71 mmol) was slowly added dropwise over 15 minutes. The mixture was refluxed at 80 °C for 14 hours. The reaction product was cooled to room temperature, neutralized with a saturated aqueous solution of sodium hydrogen carbonate (200 mL), and was subjected to extraction with dichloromethane. The separated organic layer was dried with anhydrous magnesium sulfate, followed by concentration under decompression. The obtained residue (10.7 g, 89.3 %) was used in the following reaction without a purification procedure.

### [Process 2] Preparation of ethyl 2-bromo-1-methyl-1H-imidazole-4-carboxylate

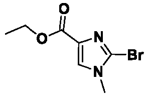

Ethyl 1-methyl-1*H*-imidazole-4-carboxylate (6.3 g, 40.9 mmol) obtained in [Process 1] was dissolved in tetrahydrofuran (63 mL), and cooled to 0 °C, followed by adding *N*-bromosuccinimide (11.1 g, 61.3 mmol) and potassium triphosphate (13.0 g, 61.3 mmol). The reaction solution was stirred at room temperature for 12 hours, followed by adding a saturated aqueous solution of sodium thiosulfate and was subjected to extraction using ethyl acetate. The separated organic layer was dried with anhydrous magnesium sulfate and vacuum distilled. The obtained residue was purified by silica gel column chromatography to obtain the title compound (6.3 g, 66.1 %).

### [Process 3] Preparation of ethyl 2-(4-methoxybenzyl)-1-methyl-1H-imidazole-4-carboxylate

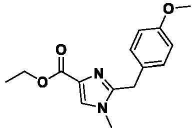

Ethyl 2-bromo-1-methyl-1*H*-imidazole-4-carboxylate (11.0 g, 47.2 mmol) obtained in [Process 2] was dissolved in anhydrous 1,4-dioxane, followed by sequentially adding [Intermediate 6-1] 2-(4-methoxybenzyl)-4,4,5,5-tetramethyl-1,3,2-dioxaborolane (23.4 g, 94.4 mmol) synthesized above, potassium triphosphate (30.1 g, 142 mmol) and [1,1'-bis(diphenylphosphino)ferrocene]dichloropalladium(II) (1.09 g, 1.42 mmol). The mixed solution was substituted with nitrogen gas for 30 minutes, then refluxed at 120 °C for 48 hours. The reaction product was cooled to room temperature, followed by adding water and was subjected to extraction with ethyl acetate. The separated organic layer was dried with anhydrous magnesium sulfate and vacuum distilled. The obtained residue was purified by silica gel column chromatography to obtain the title compound (5.24 g, 40.47 %).

### [Process 4] Preparation of ethyl 5-bromo-2-(4-methoxybenzyl)-1-methyl-1Himidazole-4-carboxylate

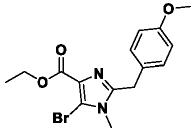

Ethyl 2-(4-methoxybenzyl)-1-methyl-1*H*-imidazole-4-carboxylate (1.27 g, 4.63 mmol) obtained in [Process 3] was dissolved in dichloromethane (12.7 mL), and cooled to 0 °C, followed by adding *N*-bromosuccinimide (832 mg, 4.63 mmol). The reaction solution was stirred at room temperature for 12 hours, followed by adding a saturated aqueous solution of sodium thiosulfate and was subjected to extraction using dichloromethane. The separated organic layer was dried with anhydrous magnesium sulfate and vacuum distilled. The obtained residue was purified by silica gel column chromatography to obtain the title compound (1.27 g, 77.7 %).

### [Intermediate 6-3] ethyl 5-bromo-1-methyl-1H-imidazole-4-carboxylate

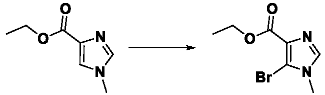

Ethyl 1-methyl-1*H*-imidazole-4-carboxylate (7.43 g, 48.2 mmol) obtained in [Process 1] was dissolved in dichloromethane (2 mL), and cooled to 0 °C, followed by adding *N*-bromosuccinimide (13 g, 72.3 mmol). The reaction solution was stirred at room temperature for 12 hours, followed by adding a saturated aqueous solution of sodium thiosulfate and was subjected to extraction using dichloromethane. The separated organic layer was dried with anhydrous sodium sulfate and vacuum distilled. The obtained residue was purified by silica gel column chromatography to obtain the title compound (3.73 g, 33.2 %).

### Example 21: 7-chloro-2-(4-methoxybenzyl)-1-methyl-5-(2-methylpyridin-3-yl)-1,5-dihydro-4H-imidazo[4,5-c]quinolin-4-one

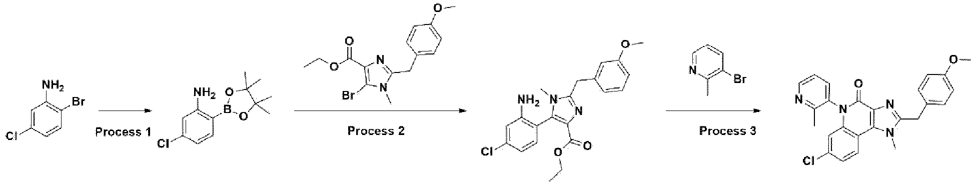

### [Process 1] Preparation of 5-chloro-2-(4,4,5,5-tetramethyl-1,3,2-dioxaborolan-2-yl)aniline

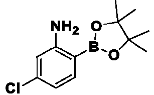

2-bromo-5-chloroaniline (8 g, 37.98 mmol), bis(pinacolato)diboron (17.71 g, 68.3 mmol), potassium acetate (7.53 g, 75.9 mmol), and 1,1'-bis(diphenylphosphino)ferrocene-palladium(II) dichloride dichloromethane complex (1.57 g, 1.90 mmol) was added to dimethyl sulfoxide (100 mL) and stirred at 85 °C for 3 hours. The reaction product was cooled to room temperature, followed by adding water and then was subjected to extraction with ethyl acetate. The separated organic layer was dried with anhydrous sodium sulfate and vacuum distilled. The obtained residue was purified by silica gel column chromatography to obtain the title compound (9.1 g, 94.5 %).

### [Process 2] Preparation of ethyl 5-(2-amino-4-chlorophenyl)-2-(3-methoxybenzyl)-1-methyl-1H-imidazole-4-carboxylate

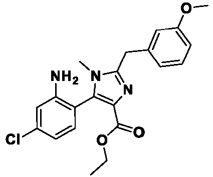

5-Chloro-2-(4,4,5,5-tetramethyl-1,3,2-dioxaborolan-2-yl)aniline (511 mg, 2.02 mmol) obtained in [Process 1] and ethyl 5-bromo-2-(4-methoxybenzyl)-1-methyl-1*H-*imidazole-4-carboxylate (475 mg, 1.34 mmol) obtained in [Intermediate 6-1] were added to 1,4-dioxane (4.75 mL), water (0.95 mL), to substitute nitrogen gas, followed by adding potassium triphosphate (571 mg, 2.69 mmol), [1,1'-bis(diphenylphosphino)ferrocene]dichloropalladium(II) (31 mg, 0.04 mmol). The mixed solution was refluxed at 100 °C for 20 hours and cooled to room temperature after completion of the reaction. Water was added to the reaction product and was subjected to extraction with ethyl acetate, and the separated organic layer was dried with anhydrous magnesium sulfate and vacuum distilled. The obtained residue was purified by silica gel column chromatography to obtain the title compound (115 mg, 21.4 %).

### [Process 3] Preparation of 7-chloro-2-(4-methoxybenzyl)-1-methyl-5-(2-methylpyridin-3-yl)-1,5-dihydro-4H-imidazo[4,5-c]quinolin-4-one

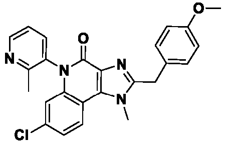

Ethyl 5-(2-amino-4-chlorophenyl)-2-(3-methoxybenzyl)-1-methyl-1*H*-imidazole-4-carboxylate (115 mg, 0.29 mmol) obtained in [Process 2] was added to toluene (2.9 mL), followed by sequentially adding 3-bromo-2-methylpyridine (0.05 mL, 0.43 mmol), cesium carbonate (189 mg, 0.58 mmol), tris(dibenzylideneacetone)dipalladium(0) (26 mg, 0.03 mmol), and xantphos (17 mg, 0.03 mmol), nitrogen gas was substituted, and refluxed at 100 °C 18 hours. The reaction mixture was cooled at room temperature, filtered through celite and vacuum distilled, the filtrate was concentrated, stirred again in ethyl acetate and vacuum filtered, and the obtained solid was purified by silica gel column chromatography to obtain the title compound (43 mg, 33.6 %).

### Example 22: 2-(4-methoxybenzyl)-1-methyl-5-(2-methylpyridin-3-yl)-1,5-dihydro-4H-imidazo[4,5-c]quinolin-4-one

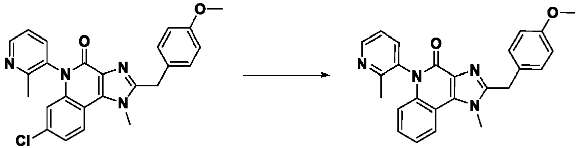

7-chloro-2-(4-methoxybenzyl)-1-methyl-5-(2-methylpyridin-3-yl)-1,5-dihydro-4*H-*imidazo[4,5-*c*]quinolin-4-one (20 mg, 0.05 mmol) synthesized in [Process 3] of Synthesis Example 6 and 10 % palladium/carbon (14.4 mg) were dissolved in methanol (0.43 mL) and was fitted with a hydrogen balloon. The reaction solution was stirred at room temperature for 14 hours and additionally stirred at 40 °C for 2 hours, and after completion of the reaction was cooled to room temperature. The reaction mixture was filtered through celite and vacuum distilled, and the obtained residue was purified by silica gel column chromatography to obtain the title compound (12 mg, 65.03 %).

### Example 23: 7-chloro-2-(4-methoxybenzyl)-1-methyl-5-(2-(trifluoromethyl)pyridin-3-yl)-1,5-dihydro-4H-imidazo[4,5-c]quinolin-4-one

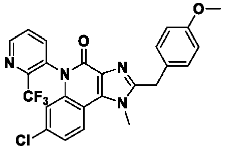

The same reaction was carried out using 3-bromo-2-(trifluoromethyl)pyridine instead of 3-bromo-2-methylpyridine, in [Process 3] of Example 21, to obtain the title compound (15 mg, 21.03 %).

### Example 24: 7-chloro-5-(2-cyclopropylpyridin-3-yl)-2-(4-methoxybenzyl)-1-methyl-1,5-dihydro-4H-imidazo[4,5-c]quinolin-4-one

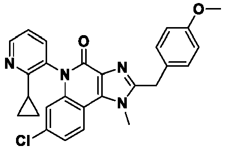

The same reaction was carried out using 3-bromo-2-cyclopropyl-pyridine instead of 3-bromo-2-methylpyridine, in [Process 3] of Example 21, to obtain the title compound (6 mg, 8.91 %).

### Example 25: 7-chloro-2-(4-methoxybenzyl)-1-methyl-5-(2-(trifluoromethyl)phenyl)-1,5-dihydro-4H-imidazo[4,5-c]quinolin-4-one

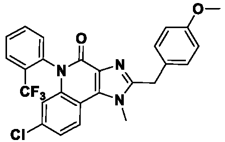

The same reaction was carried out using 1-bromo-2-(trifluoromethyl)benzene instead of 3-bromo-2-methylpyridine, in [Process 3] of Example 21, to obtain the title compound (25.7 mg, 24.82 %).

### Example 26: 7-chloro-5-(2-chlorophenyl)-2-(4-methoxybenzyl)-1-methyl-1,5-dihydro-4H-imidazo[4,5-c]quinolin-4-one

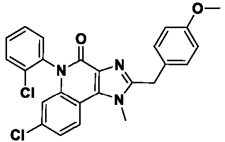

The same reaction was carried out using 1-bromo-2-chlorobenzene instead of 3-bromo-2-methylpyridine, in [Process 3] of Example 21, to obtain the title compound (16.7 mg, 17.38 %).

### Example 27: 2-(4-methoxybenzyl)-1-methyl-5-(2-methylpyridin-3-yl)-7-(trifluoromethyl)-1,5-dihydro-4H-imidazo[4,5-c]quinolin-4-one

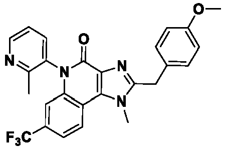

The same reaction was carried out using ethyl 5-(2-amino-4-(trifluoromethyl)phenyl)-2-(4-methoxybenzyl)-1-methyl-1*H*-imidazole-4-carboxylate instead of ethyl 5-(2-amino-4-chlorophenyl)-2-(3-methoxybenzyl)-1-methyl-1*H*-imidazole-4-carboxylate, in [Process 3] of Example 21, to obtain the title compound (36.4 mg, 39.65 %).

### Example 28: 7-chloro-2-(4-fluorobenzyl)-1-methyl-5-(2-methylpyridin-3-yl)-1,5-dihydro-4H-imidazo[4,5-c]quinolin-4-one

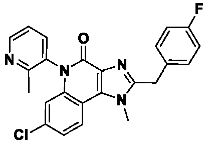

The same reaction was carried out using ethyl 5-(2-amino-4-chlorophenyl)-2-(4-fluorobenzyl)-1-methyl-1*H*-imidazole-4-carboxylate instead of ethyl 5-(2-amino-4-chlorophenyl)-2-(3-methoxybenzyl)-1-methyl-1*H*-imidazole-4-carboxylate, in [Process 3] of Example 21, to obtain the title compound (18.2 mg, 24.82 %).

### Example 29: 2-(benzo[d][1,3]dioxol-5-ylmethyl)-7-chloro-1-methyl-5-(2-methylpyridin-3-yl)-1,5-dihydro-4H-imidazo[4,5-c]quinolin-4-one

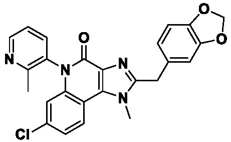

The same reaction was carried out using ethyl 5-(2-amino-4-chlorophenyl)-2-(benzo[*d*][1,3]dioxol-5-ylmethyl)-1-methyl-1*H*-imidazole-4-carboxylate instead of ethyl 5-(2-amino-4-chlorophenyl)-2-(3-methoxybenzyl)-1-methyl-1*H*-imidazole-4-carboxylate, in [Process 3] of Example 21, to obtain the title compound (33.5 mg, 29.18 %).

### Example 30: 7-chloro-2-(3,4-difluorobenzyl)-1-methyl-5-(2-methylpyridin-3-yl)-1,5-dihydro-4H-imidazo[4,5-c]quinolin-4-one

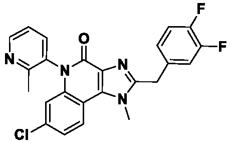

The same reaction was carried out using ethyl 5-(2-amino-4-chlorophenyl)-2-(3,4-difluorobenzyl)-1-methyl-1*H*-imidazole-4-carboxylate instead of ethyl 5-(2-amino-4-chlorophenyl)-2-(3-methoxybenzyl)-1-methyl-1*H*-imidazole-4-carboxylate, in [Process 3] of Example 21, to obtain the title compound (16 mg, 21.82 %).

### Example 31: 7-chloro-2-(4-methoxybenzyl)-1-methyl-5-(pyridin-3-yl)-1,5-dihydro-4H-imidazo[4,5-c]quinolin-4-one

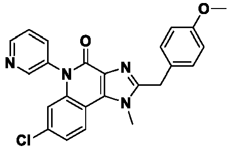

The same reaction was carried out using 3-bromopyridine instead of 3-bromo-2-methylpyridine, in [Process 3] of Example 21, to obtain the title compound (11 mg, 12.76 %).

### Example 32: 7-chloro-5-(4-fluorophenyl)-2-(4-methoxybenzyl)-1-methyl-1,5-dihydro-4H-imidazo[4,5-c]quinolin-4-one

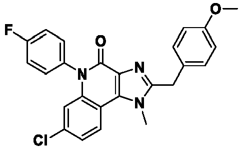

5-chloro-*N*-(4-fluorophenyl)-2-(4,4,5,5-tetramethyl-1,3,2-dioxaborolan-2-yl)aniline (70 mg, 0.20 mmol) and ethyl 5-bromo-2-(4-methoxybenzyl)-1-methyl-1*H*-imidazole-4-carboxylate (106 mg, 0.30 mmol) obtained in [Intermediate 6-2] were added to 1,4-dioxane (1 mL), water (0.1 mL), to substitute nitrogen gas, followed by adding fluorocesium (95 mg, 0.61 mmol), [1,1'-bis(diphenylphosphino)ferrocene]dichloropalladium(II) (16 mg, 0.02 mmol). The mixed solution was refluxed at 100 °C for 72 hours and cooled to room temperature after completion of the reaction. Water was added to the reaction product and was subjected to extraction with dichloromethane and methanol, and the separated organic layer was dried with anhydrous magnesium sulfate and vacuum distilled. The obtained residue was purified by silica gel column chromatography to obtain the title compound (3.5 mg, 3.88 %).

### Example 33: 7-chloro-2-(4-(difluoromethoxy)benzyl)-1-methyl-5-(2-methylpyridin-3-yl)-1,5-dihydro-4H-imidazo[4,5-c]quinolin-4-one

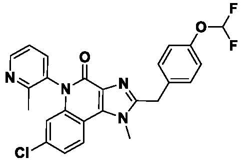

The same reaction was carried out using ethyl 5-(2-amino-4-chlorophenyl)-2-(4-difluoromethoxy)benzyl)-1-methyl-1*H*-imidazole-4-carboxylate instead of ethyl 5-(2-amino-4-chlorophenyl)-2-(3-methoxybenzyl)-1-methyl-1*H*-imidazole-4-carboxylate, in [Process 3] of Example 21, to obtain the title compound (12 mg, 10.88 %).

### Example 34: 7-chloro-5-(4-methoxyphenyl)-2-((4-methoxyphenyl)thio)-1-methyl-1,5-dihydro-4H-imidazo[4,5-c]quinolin-4-one

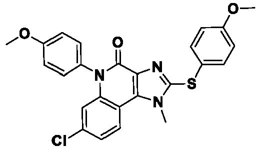

The same reaction was carried out using 7-chloro-5-(4-methoxyphenyl)-1-methyl-1,5-dihydro-4*H*-imidazo[4,5-c]quinolin-4-one instead of 7-chloro-1-methyl-5-phenyl-1,5-dihydro-4*H*-imidazo[4,5-c]quinolin-4-one, in [Process 2] of Example 18, to obtain the title compound (8 mg, 18.95 %).

### Example 35: 7-chloro-2-((4-methoxyphenyl)thio)-1-methyl-5-(2-methylpyridin-3-yl)-1,5-dihydro-4H-imidazo[4,5-c]quinolin-4-one

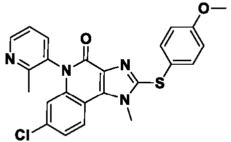

The same reaction was carried out using 7-chloro-1-methyl-5-(2-methylpyridin-3-yl)-1,5-dihydro-4*H*-imidazo[4,5-c]quinolin-4-one instead of 7-chloro-1-methyl-5-phenyl-1,5-dihydro-4*H*-imidazo[4,5-c]quinolin-4-one, in [Process 2] of Example 18, to obtain the title compound (65 mg, 18.24 %).

### Example 36: 7-chloro-2-((4-fluorophenyl)thio)-1-methyl-5-(2-methylpyridin-3-yl)-1,5-dihydro-4H-imidazo[4,5-c]quinolin-4-one

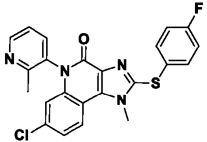

The same reaction was carried out using 7-chloro-1-methyl-5-(2-methylpyridin-3-yl)-1,5-dihydro-4H-imidazo[4,5-c]quinolin-4-one and 4-fluorobenzenthiol instead of 7-chloro-1-methyl-5-phenyl-1,5-dihydro-4*H*-imidazo[4,5-c]quinolin-4-one and 4-methoxybenzenthiol, in [Process 2] of Example 18, to obtain the title compound (18 mg, 8.50 %).

### Example 37: 7-chloro-2-((3,4-difluorophenyl)thio)-1-methyl-5-(2-methylpyridin-3-yl)-1,5-dihydro-4H-imidazo[4,5-c]quinolin-4-one

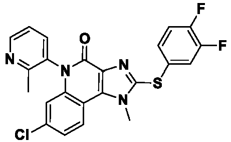

The same reaction was carried out using 7-chloro-1-methyl-5-(2-methylpyridin-3-yl)-1,5-dihydro-4*H*-imidazo[4,5-c]quinolin-4-one and 3,4-difluorobenzenthiol instead of 7-chloro-1-methyl-5-phenyl-1,5-dihydro-4*H*-imidazo[4,5-*c*]quinolin-4-one and 4-methoxybenzenthiol, in [Process 2] of Example 18, to obtain the title compound (37 mg, 24.50 %).

### Example 38: 2-(benzo[d][1,3]dioxol-5-ylthio)-7-chloro-1-methyl-5-(2-methylpyridin-3-yl)-1,5-dihydro-4H-imidazo[4,5-c]quinolin-4-one

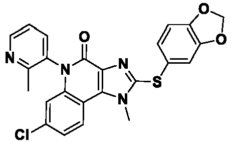

The same reaction was carried out using 7-chloro-1-methyl-5-(2-methylpyridin-3-yl)-1,5-dihydro-4*H*-imidazo[4,5-c]quinolin-4-one and benzo[*d*][1,3]dioxol-5-thiol instead of 7-chloro-1-methyl-5-phenyl-1,5-dihydro-4*H*-imidazo[4,5-*c*]quinolin-4-one and 4-methoxybenzenthiol, in [Process 2] of Example 18, to obtain the title compound (37 mg, 25.20 %).

### Example 39: 7-chloro-2-((2,4-difluorophenyl)thio)-1-methyl-5-(2-methylpyridin-3-yl)-1,5-dihydro-4H-imidazo[4,5-c]quinolin-4-one

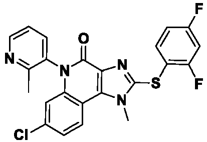

The same reaction was carried out using 7-chloro-1-methyl-5-(2-methylpyridin-3-yl)-1,5-dihydro-4*H*-imidazo[4,5-*c*]quinolin-4-one and 2,4-difluorobenzenthiol instead of 7-chloro-1-methyl-5-phenyl-1,5-dihydro-4*H*-imidazo[4,5-*c*]quinolin-4-one and 4-methoxybenzenthiol, in [Process 2] of Example 18, to obtain the title compound (9.5 mg, 6.58 %).

### Example 40: 7-chloro-2-((4-methoxyphenyl)amino)-1-methyl-5-phenyl-1,5-dihydro-4H-imidazo[4,5-c]quinolin-4-one

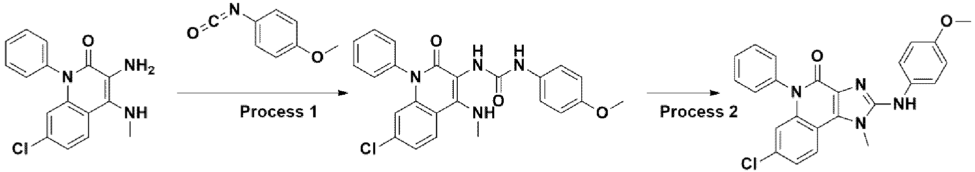

### [Process 11 Preparation of 1-(7-chloro-4-(methylamino)-2-oxo-1-phenyl-1,2-dihydroquinolin-3-yl)-3-(4-methoxyphenyl)urea

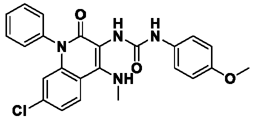

3-amino-7-chloro-4-(methylamino)-1-phenylquinolin-2(1H)-one (200 mg, 0.67 mmol) and 4-methoxyphenyl isocyanate (0.1 mL, 0.80 mmol) synthesized in [Process 8] of Example 1 were added to dichloromethane (3 mL), and the reaction solution was stirred at room temperature for 16 hours and at 70 °C for 4 hours. The reaction solution was concentrated by vacuum distillation and then purification was performed using silica gel column chromatography to obtain the title compound (130 mg, 43.40 %).

### [Process 2] Preparation of 7-chloro-2-((4-methoxyphenyl)amino)-1-methyl-5-phenyl-1,5-dihydro-4H-imidazo[4,5-c]quinolin-4-one

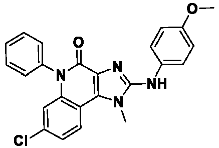

1-(7-chloro-4-(methylamino)-2-oxo-1-phenyl-1,2-dihydroquinolin-3-yl)-3-(4-methoxyphenyl)urea (48 mg, 0.11 mmol) synthesized in [Process 1] above was added to phosphoryl chloride (1 mL), and the reaction solution was stirred at 80 °C for 2 hours. The reaction solution was adjusted to pH 7 to 8 with saturated sodium bicarbonate solution at room temperature and then was subjected to extraction with ethyl acetate. The separated organic layer was dried with anhydrous sodium sulfate and concentrated by vacuum distillation. Purification was performed using silica gel column chromatography to obtain the title compound (12.8 mg, 24.64 %).

### Example 41: 7-chloro-2-((4-methoxyphenyl)(methyl)amino)-1-methyl-5-phenyl-1,5-dihydro-4H-imidazo[4,5-c]quinolin-4-one

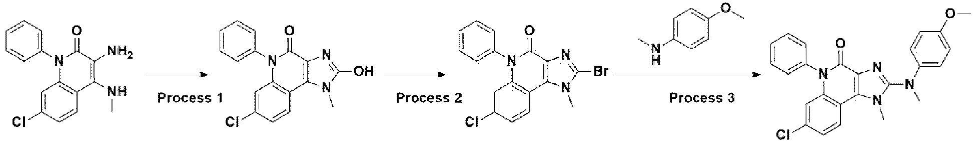

### [Process 1] Preparation of 7-chloro-2-hydroxy-1-methyl-5-phenyl-1,5-dihydro-4H-imidazo[4,5-c]quinolin-4-one

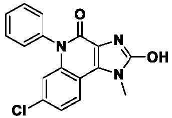

3-amino-7-chloro-4-(methylamino)-1-phenylquinolin-2(1*H*)-one (150 mg, 0.50 mmol), triphosgene (189 mg, 0.60 mmol) and triethylamine (0.14 mL, 1.00 mmol) synthesized in [Process 8] of Example 1 were added to tetrahydrofuran (5 mL), and the reaction solution was stirred at room temperature for 20 hours. The reaction solution was adjusted to pH 7 to 8 with saturated sodium bicarbonate solution at room temperature and then was subjected to extraction with dichloromethane:methanol = 20:1 (volume ratio). The separated organic layer was dried with anhydrous sodium sulfate and concentrated by vacuum distillation. Then purified by silica gel column chromatography to obtain the title compound (86.9 mg, 53.31 %).

### [Process 2] Preparation of 2-bromo-7-chloro-1-methyl-5-phenyl-1,5-dihydro-4H-imidazo[4,5-c]quinolin-4-one

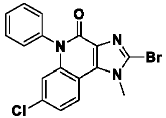

7-chloro-2-hydroxy-1-methyl-5-phenyl-1,5-dihydro-4*H*-imidazo[4,5-c]quinolin-4-one (60 mg, 0.18 mmol) and phosphoryl bromide (5.6 g, 18.4 mmol) synthesized in [Process 1] above were added to toluene (1 mL), and the reaction solution was stirred at 110 °C for 2 days. The reaction solution was adjusted to pH 7 to 8 with saturated sodium bicarbonate solution at room temperature and then was subjected to extraction with ethyl acetate. The separated organic layer was dried with anhydrous sodium sulfate, concentrated by vacuum distillation, and purified by silica gel column chromatography to obtain the title compound (13.7 mg, 19.14 %).

### [Process 3] Preparation of 7-chloro-2-((4-methoxyphenyl)(methyl)amino)-1-methyl-5-phenyl-1,5-dihydro-4H-imidazo[4,5-c]quinolin-4-one

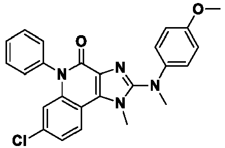

2-bromo-7-chloro-1-methyl-5-phenyl-1,5-dihydro-4*H*-imidazo[4,5-*c*]quinolin-4-one (17 mg, 0.04 mmol) synthesized in [Process 2] above and 4-methoxy-*N*-methylaniline (12 mg, 0.09 mmol), cesium carbonate (29 mg, 0.09 mmol), tris(dibenzylideneacetone)dipalladium(0) (4 mg, 0.004 mmol), rac-BINAP (8.4 mg, 0.013 mmol) were added to toluene (0.4 mL), and after nitrogen gas substitution, the mixture was refluxed at 100 °C for 16 hours. After the reaction product was cooled to room temperature, the solid was filtered and washed with dichloromethane: Methanol= 10:1 (volume ratio). The filtrate was concentrated and purified by silica gel column chromatography to obtain the title compound (6.8 mg, 34.94 %).

### Example 42: 7-chloro-2-(4-methoxyphenoxy)-1-methyl-5-phenyl-1,5-dihydro-4H-imidazo[4,5-c]quinolin-4-one

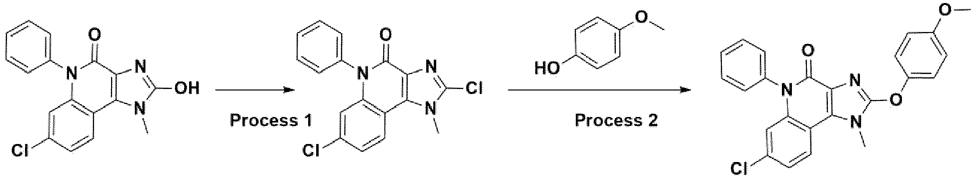

### [Process 11 Preparation of 2,7-dichloro-1-methyl-5-phenyl-1,5-dihydro-4H-imidazo[4,5-c]quinolin-4-one

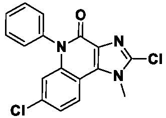

Phosphorus oxychloride (0.35 mL, 3.68 mmol) was added to 7-chloro-2-hydroxy-1-methyl-5-phenyl-1,5-dihydro-4*H*-imidazo[4,5-*c*]quinolin-4-one (20 mg, 0.06 mmol) synthesized in [Process 1] of Example 41 and refluxed at 120 °C for 3 days. The reaction product was cooled to room temperature and then vacuum distilled. Then a saturated aqueous solution of sodium hydrogen carbonate was added and the mixture was subjected to extraction with dichloromethane and methanol. The separated organic layer was dried with anhydrous sodium sulfate, concentrated by vacuum distillation, and purified by silica gel column chromatography to obtain the title compound (15 mg, 70.98 %).

### [Process 2] Preparation of 7-chloro-2-(4-methoxyphenoxy)-1-methyl-5-phenyl-1,5-dihydro-4H-imidazo[4,5-c]quinolin-4-one

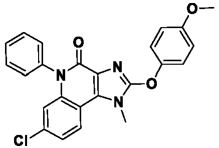

4-methoxyphenol (218.58 mg, 1.74 mmol) and cesium carbonate (28.68 mg, 0.09 mmol) were added to 2,7-dichloro-1-methyl-5-phenyl-1,5-dihydro-4*H*-imidazo[4,5-*c*]quinolin-4-one (15 mg, 0.04 mmol) synthesized in [Process 1] above and refluxed at 140 °C for 24 hours. The reaction product was cooled to room temperature and purification was performed using silica gel column chromatography to obtain the title compound (6 mg, 31.88 %).

According to an embodiment of the present disclosure, the compound of Formula 1 may be a compound selected from the group consisting of the compounds listed in [Table 3] below.

**[Table 3]**

| Compound No. | Structure | Nomenclature | MS[M+H]⁺ |
|---|---|---|---|
| | ¹H-NMR spectrum (300MHz) | | |
| 21 | 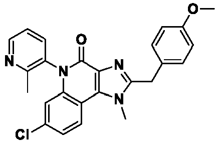 | 7-chloro-2-(4-methoxybenzyl)-1-methyl-5-(2-methylpyridin-3-yl)-1,5-dihydro-4*H*-imidazo[4,5-*c*]quinolin-4-one | 445.14 |
| | ¹H-NMR spectrum (300 MHz, CDCl₃) *δ* 8.72(dd, 1H), 7.96(d, 1H), 7.55(dd, 1H), 7.41(dd, 1H), 7.23-7.18(m, 3H), 6.86(d, 2H), 6.58(d, 1H), 4.36(s, 2H), 3.96(s, 3H), 3.79(s, 3H), 2.29(s, 3H) | | |
| 22 | 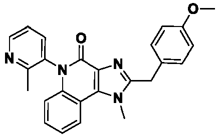 | 2-(4-methoxybenzyl)-1-methyl-5-(2-methylpyridin-3-yl)-1,5-dihydro-4*H-*imidazo[4,5-*c*]quinolin-4-one | 411.17 |
| | ¹H-NMR spectrum (300 MHz, CDCl₃) *δ* 8.67(d, 1H), 8.06(m, 1H), 7.55(dd, 1H), 7.38(dd, 1H), 7.28(m, 2H), 7.20(d, 2H), 6.86(d, 2H), 6.60(m, 1H), 4.36(s, 2H), 3.98(s, 3H), 3.79(s, 3H), 2.28(s, 3H) | | |
| 23 | 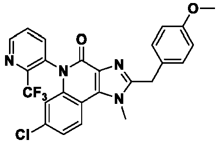 | 7-chloro-2-(4-methoxybenzyl)-1-methyl-5-(2-(trifluoromethyl)pyridin-3-yl)-1,5-dihydro-4*H*-imidazo[4,5-*c*]quinolin-4-one | 499.11 |
| | ¹H-NMR spectrum (300 MHz, CDCl₃) *δ* 8.94(qd, 1H), 7.96(d, 1H), 7.83-7.77(m, 2H), 7.24(dd, 1H), 7.17(d, 2H), 6.86(td, 2H), 6.46(d, 1H), 4.34(d, 2H), 3.95(s, 3H), 3.79(s, 3H) | | |
| 24 | 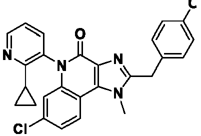 | 7-chloro-5-(2-cyclopropylpyridin-3-yl)-2-(4-methoxybenzyl)-1-methyl-1,5-dihydro-4*H*-imidazo[4,5-*c*]quinolin-4-one | 471.15 |
| | ¹H-NMR spectrum (300 MHz, CDCl₃) *δ* 8.64(dd, 1H), 7.96(d, 1H), 7.51(dd, 1H), 7.32-7.21(m, 4H), 6.86(qd, 2H), 6.67(d, 1H), 4.35(s, 2H), 3.95(s, 3H), 3.79(s, 3H), 1.57-1.52(m, 1H), 1.24-1.17(m, 1H), 1.07-1.01(m, 1H), 0.87-0.79(m, 1H), 0.72-0.67(m, 1H) | | |
| 25 | 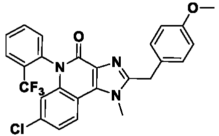 | 7-chloro-2-(4-methoxybenzyl)-1-methyl-5-(2-(trifluoromethyl)phenyl)-1,5-dihydro-4*H*-imidazo[4,5-*c*]quinolin-4-one | 498.11 |
| | ¹H-NMR spectrum (300 MHz, CDCl₃) *δ* 7.95-7.90(m, 2H), 7.82(m, 1H), 7.71(m, 1H), 7.35(d, 1H), 7.20-7.17(m, 3H), 6.85(td, 2H), 6.49(d, 1H), 4.33(d, 2H), 3.93(s, 3H), 3.78(s, 3H) | | |
| 26 | 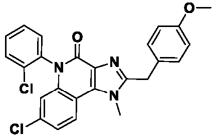 | 7-chloro-5-(2-chlorophenyl)-2-(4-methoxybenzyl)-1-methyl-1,5-dihydro-4*H*-imidazo[4,5-*c*]quinolin-4-one | 464.09 |
| | ¹H-NMR spectrum (300 MHz, CDCl₃) *δ* 7.93(d, 1H), 7.65(m, 1H), 7.52-49(m, 2H), 7.36-32(m, 1H), 7.21-7.16(m, 3H), 6.85(d, 2H), 6.59(d, 1H), 4.33(d, 2H), 3.93(s, 3H), 3.77(s, 3H) | | |
| 27 | 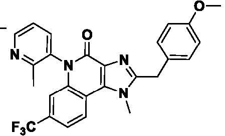 | 2-(4-methoxybenzyl)-1-methyl-5-(2-methylpyridin-3-yl)-7-(trifluoromethyl)-1,5-dihydro-4*H*-imidazo[4,5-*c*]quinolin-4-one | 479.16 |
| | ¹H-NMR spectrum (300 MHz, CDCl₃) *δ* 8.71(dd, 1H), 8.14(d, 1H), 7.55-46(m, 2H), 7.42-37(m, 1H), 7.17(d, 2H), 6.86-82(m, 3H), 4.36(s, 2H), 3.99(s, 3H), 3.77(s, 3H), 2.26(s, 3H) | | |
| 28 | 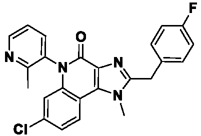 | 7-chloro-2-(4-fluorobenzyl)-1-methyl-5-(2-methylpyridin-3-yl)-1,5-dihydro-4*H*-imidazo[4,5-*c*]quinolin-4-one | 433.12 |
| | ¹H-NMR spectrum (300 MHz, CDCl₃) *δ* 8.74-8.72 (dd, 1H), 7.98(d, 1H), 7.56(dd, 1H), 7.42(m, 1H), 7.25(m, 3H), 7.03(t, 2H), 6.61 (d, 1H), 4.40 (s, 2H), 3.98(s, 3H), 2.30(s, 3H) | | |
| 29 | 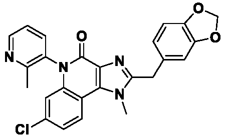 | 2-(benzo[*d*][1,3]dioxol-5-ylmethyl)-7-chloro-1-methyl-5-(2-methylpyridin-3-yl)-1,5-dihydro-4*H*-imidazo[4,5-*c*]quinolin-4-one | 459.11 |
| | ¹H-NMR spectrum (300 MHz, CDCl₃) *δ* 8.72(dd, 1H), 7.97(d, 1H), 7.55(dd, 1H), 7.41(dd, 1H), 7.26-7.23(m, 1H), 6.78-6.72(m, 3H), 6.59(d, 1H), 5.94(s, 2H), 4.33(s, 2H), 3.98(s, 3H), 2.29(s, 3H) | | |
| 30 | 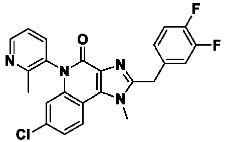 | 7-chloro-2-(3,4-difluorobenzyl)-1-methyl-5-(2-methylpyridin-3-yl)-1,5-dihydro-4*H*-imidazo[4,5-*c*]quinolin-4-one | 451.11 |
| | ¹H-NMR spectrum (300 MHz, CDCl₃) *δ* 8.71 (dd, 1H), 7.98(d, 1H), 7.55(dd, 1H), 7.41(dd, 1H), 7.28-7.24(m, 1H), 7.15-7.09(m, 2H), 7.08-7.03(m, 1H), 6.60(d, 1H), 4.37(s, 2H), 3.98(s, 3H), 2.30(s, 3H) | | |
| 31 | 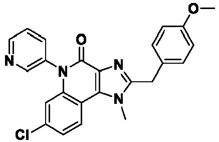 | 7-chloro-2-(4-methoxybenzyl)-1-methyl-5-(pyridin-3-yl)-1,5-dihydro-4*H-*imidazo[4,5-*c*]quinolin-4-one | 431.12 |
| | ¹H-NMR spectrum (300 MHz, CDCl₃) *δ* 8.81 (d, 1H), 8.58(d, 1H), 7.95(d, 1H), 7.71(td, 1H), 7.59(dd, 1H), 7.26-7.17(m, 3H), 6.84(td, 2H), 6.69(d, 1H), 4.35(s, 2H), 3.95(s, 3H), 3.79(s, 3H) | | |
| 32 | 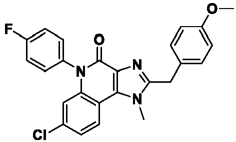 | 7-chloro-5-(4-fluorophenyl)-2-(4-methoxybenzyl)-1-methyl-1,5-dihydro-4*H*-imidazo[4,5-*c*]quinolin-4-one | 448.11 |
| | ¹H-NMR spectrum (300 MHz, MeOD) *δ* 8.24(d, 1H), 7.44-7.32(m, 5H), 7.18(d, 2H), 6.88(d, 2H), 6.72(d, 1H), 4.33(s, 2H), 4.04(s, 3H), 3.75(s, 3H) | | |
| 33 | 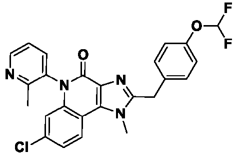 | 7-chloro-2-(4-(difluoromethoxy)benzyl)-1-methyl-5-(2-methylpyridin-3-yl)-1,5-dihydro-4*H-*imidazo[4,5-*c*]quinolin-4-one | 481.12 |
| | ¹H-NMR spectrum (300 MHz, CDCl₃) *δ* 8.72(dd, 1H), 7.97(d, 1H), 7.55(dd, 1H), 7.41(dd, 1H), 7.30-7.23(m, 3H), 7.10(d, 2H), 6.74-6.25(t, 1H), 6.59(d, 1H), 4.40(s, 2H), 3.98(s, 3H), 2.29(s, 3H) | | |
| 34 | 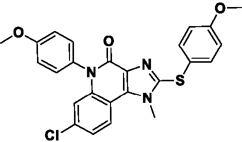 | 7-chloro-5-(4-methoxyphenyl)-2-((4-methoxyphenyl)thio)-1-methyl-1,5-dihydro-4*H*-imidazo[4,5-*c*]quinolin-4-one | 478.09 |
| | ¹H-NMR spectrum (300 MHz, CDCl₃) *δ* 7.96(d, 1H), 7.45(td, 2H), 7.23-7.08(m, 5H), 6.85(td, 2H), 6.80(d, 1H), 4.17(s, 3H), 3.90(s, 3H), 3.78(s, 3H) | | |
| 35 | 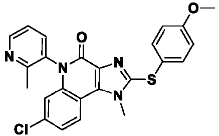 | 7-chloro-2-((4-methoxyphenyl)thio)-1-methyl-5-(2-methylpyridin-3-yl)-1,5-dihydro-4*H*-imidazo[4,5-*c*]quinolin-4-one | 463.09 |
| | ¹H-NMR spectrum (300 MHz, CDCl₃) *δ* 8.71 (dd, 1H), 8.02(d, 1H), 7.53-7.47(m, 3H), 7.42-7.39(m, 1H), 7.28-7.24(m, 1H), 6.88(td, 2H), 6.58(d, 1H), 4.20(s, 3H), 3.79(s, 3H), 2.27(s, 3H) | | |
| 36 | 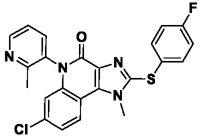 | 7-chloro-2-((4-fluorophenyl)thio)-1-methyl-5-(2-methylpyridin-3-yl)-1,5-dihydro-4*H*-imidazo[4,5-*c*]quinolin-4-one | 451.07 |
| | ¹H-NMR spectrum (300 MHz, CDCl₃) *δ* 8.72(m, 1H), 8.03(d, 1H), 7.54-7.49(m, 3H), 7.43-7.41(m, 1H), 7.30-7.26(m, 1H), 7.08-7.02(m, 2H), 6.60(d, 1H), 4.22(s, 3H), 2.28(s, 3H) | | |
| 37 | 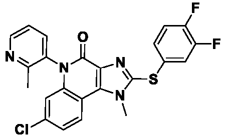 | 7-chloro-2-((3,4-difluorophenyl)thio)-1-methyl-5-(2-methylpyridin-3-yl)-1,5-dihydro-4*H*-imidazo[4,5-*c*]quinolin-4-one | 469.06 |
| | ¹H-NMR spectrum (300 MHz, CDCl₃) *δ* 8.71(m, 1H), 8.05(d, 1H), 7.53(dd, 1H), 7.41(dd, 1H), 7.35-7.13(m, 3H), 6.61(d, 2H), 4.23(s, 3H), 2.29(s, 3H) | | |
| 38 | 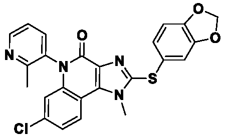 | 2-(benzo[*d*][1,3]dioxol-5-ylthio)-7-chloro-1-methyl-5-(2-methylpyridin-3-yl)-1,5-dihydro-4*H*-imidazo[4,5-*c*]quinolin-4-one | 477.07 |
| | ¹H-NMR spectrum (300 MHz, CDCl₃) *δ* 8.71 (dd, 1H), 8.03(d, 1H), 7.52(dd, 1H), 7.42-7.40(m, 1H), 7.29-7.26(m, 1H), 7.05(dd, 1H), 6.99(d, 1H), 6.78(d, 1H), 6.59(d, 1H), 5.97(s, 2H), 4.20(s, 3H), 2.28(s, 3H) | | |
| 39 | 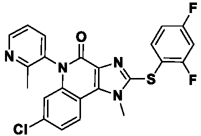 | 7-chloro-2-((2,4-difluorophenyl)thio)-1-methyl-5-(2-methylpyridin-3-yl)-1,5-dihydro-4*H*-imidazo[4,5-*c*]quinolin-4-one | 469.06 |
| | ¹H-NMR spectrum (300 MHz, CDCl₃) *δ* 8.71(m, 1H), 8.06(d, 1H), 7.65-7.61(m, 1H), 7.52-7.49(m, 1H), 7.42-7.36(m, 1H), 7.31-7.26(m, 1H), 6.93-6.85(m, 2H), 6.59(d, 1H), 4.30(s, 3H), 2.27(s, 3H) | | |
| 40 | 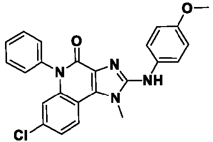 | 7-chloro-2-((4-methoxyphenyl)amino)-1-methyl-5-phenyl-1,5-dihydro-4*H-*imidazo[4,5-*c*]quinolin-4-one | 431.12 |
| | ¹H-NMR spectrum (300 MHz, DMSO-d₆) *δ* 8.85(s, 1H), 8.26(d, 1H), 7.68-7.58(m, 5H), 7.34(d, 2H), 6.92(d, 2H), 6.48(s, 1H), 4.06(s, 3H), 3.73(s, 3H) | | |
| 41 | 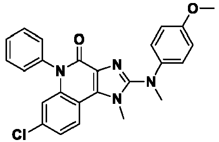 | 7-chloro-2-((4-methoxyphenyl)(methyl)amino)-1-methyl-5-phenyl-1,5-dihydro-4*H-*imidazo[4,5-*c*]quinolin-4-one | 445.14 |
| | ¹H-NMR spectrum (300 MHz, CDCl₃) *δ* 7.85 (d, 1H), 7.64-7.54(m, 3H), 7.30-7.26(m, 2H), 7.17(dd, 1H), 6.97(d, 2H), 6.87(d, 2H), 6.72(d, 1H), 3.80(s, 3H), 3.61(s, 3H), 3.52(s, 3H) | | |
| 42 | 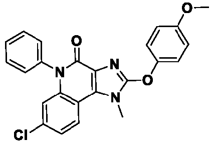 | 7-chloro-2-(4-methoxyphenoxy)-1-methyl-5-phenyl-1,5-dihydro-4*H-*imidazo[4,5-*c*]quinolin-4-one | 432.10 |
| | ¹H-NMR spectrum (300 MHz, CDCl₃) *δ* 7.98(d, 1H), 7.63-7.53(m, 3H), 7.35(td, 2H), 7.27-7.22(m, 3H), 6.91(td, 2H), 6.73(d, 1H), 4.12(s, 3H), 3.81(s, 3H) | | |

### <Synthesis Example 7>

### [Intermediate 7-1] Ethyl 5-iodo-2-(4-methoxybenzyl)-1-methyl-1H-imidazole-4-carboxylate

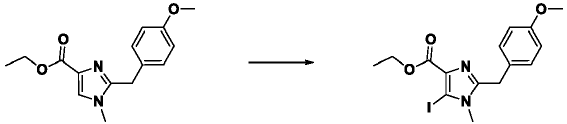

Ethyl 2-(4-methoxybenzyl)-1-methyl-1*H*-imidazole-4-carboxylate (1 g, 3.65 mmol) obtained in [Process 3] of [Intermediate 6-2] was dissolved in *N,N*-dimethylformamide (20 mL), followed by adding *N*-iodosuccinimide (2.54 g, 10.94 mmol). The reaction solution was refluxed at 100 °C for 17 hours, then cooled to room temperature, followed by adding a saturated aqueous solution of sodium thiosulfate and was subjected to extraction using ethyl acetate. The separated organic layer was dried with anhydrous sodium sulfate and vacuum distilled. The obtained residue was purified by silica gel column chromatography to obtain the title compound (1.4 g, 95.96 %).

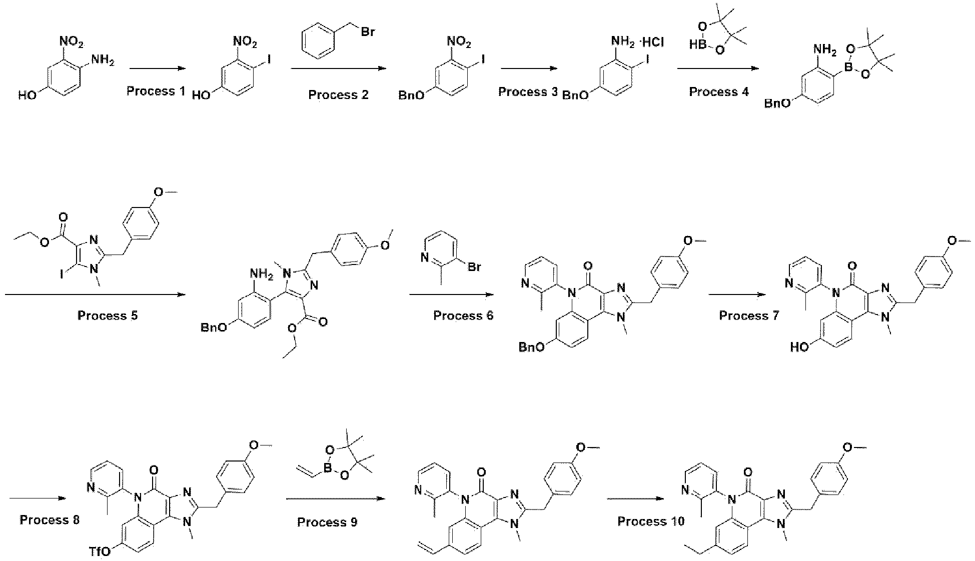

### [Process 1] Preparation of 4-Iodo-3-nitrophenol

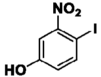

4-amino-3-nitrophenol (10.3 g, 64.89 mmol) was dissolved in 38 % aqueous hydrochloric acid solution (5 mL), cooled to 0 °C and an aqueous solution (20 mL) of sodium nitrite (9.14 g, 129.77 mmol) was slowly added dropwise. After stirring at 0 °C for 1 h, an aqueous solution (40 mL) of potassium iodide (21.54 g, 129.77 mmol) was slowly added dropwise. After the reaction solution was stirred at room temperature for 15 hours, the resulting solid was collected by filtration, washed with excess water and dried to obtain the title compound (16.5 g, 95.96 %).

### [Process 2] Preparation of 4-(benzyloxy)-1-iodo-2-nitrobenzene

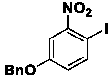

4-Iodo-3-nitrophenol (16.5 g, 62.26 mmol) obtained in [Process 1] above was dissolved in acetone (165 mL), followed by adding potassium carbonate (17.30 g, 124.52 mmol) and benzyl bromide (8.31 mL, 68.49 mmol). The reaction solution was stirred at room temperature for 18 hours and the resulting solid was removed by filtration, and the filtrate was vacuum distilled. Water was added and the mixture was subjected to extraction using ethyl acetate. The separated organic layer was dried with anhydrous sodium sulfate, followed by vacuum filtration and vacuum distillation to obtain the title compound (21 g, 94.98 %).

### [Process 3] Preparation of 5-(benzyloxy)-2-iodoaniline

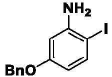

4-(Benzyloxy)-1-iodo-2-nitrobenzene (5 g, 14.08 mmol) obtained in [Process 2] above was dissolved in methanol (37.5 mL), followed by adding activated carbon (845 mg, 70.40 mmol) and iron(III) chloride (235 mg, 1.41 mmol), and after nitrogen gas substitution, the mixture was stirred for 10 minutes at 70 °C. After cooling the reaction solution to room temperature, hydrazine monohydrate (3.48 mL, 70.40 mmol) was slowly added dropwise. The reaction solution was refluxed at 70 °C for 21 hours, then cooled to room temperature and filtered through celite. The filtrate was vacuum distilled, followed by adding water and was subjected to extraction with ethyl acetate. The separated organic layer was dried with anhydrous sodium sulfate, followed by vacuum filtration and vacuum distillation. The obtained residue was dissolved in ethyl acetate (40 mL), followed by adding 4 N hydrochloric acid 1,4-dioxane solution (20 mL) and stirred at room temperature for 30 minutes. The resulting solid was collected by filtration, washed with excess ethyl acetate and dried to obtain the title compound (3.22 g, 63.25 %).

### [Process 4] Preparation of 5-(benzyloxy)-2-(4,4,5,5-tetramethyl-1,3,2-dioxaborolan-2-yl)aniline

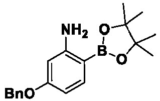

5-(benzyloxy)-2-iodoaniline hydrochloride (3.22 g, 8.90 mmol) obtained in [Process 3] above was added to anhydrous 1,4-dioxane (48.3 mL) to substitute nitrogen gas, followed by adding triethylamine (6.27 mL, 44.52 mmol), 1,1'-bis(diphenylphosphino)ferrocene-palladium(II) dichloride dichloromethane complex (368 mg, 0.44 mmol), 4,4,5,5-tetramethyl-1,3,2-dioxaborolane (3.52 g, 26.71 mmol). The mixed solution was refluxed at 100 °C for 14 hours and cooled to room temperature after completion of the reaction. A saturated aqueous solution of ammonium chloride was added to the reaction product and was subjected to extraction with ethyl acetate, and the separated organic layer was dried with anhydrous sodium sulfate and vacuum distilled. The obtained residue was purified by silica gel column chromatography to obtain the title compound (1 g, 34.53 %).

### [Process 5] Preparation of ethyl 5-(2-amino-4-(benzyloxy)phenyl)-2-(4-methoxybenzyl)-1-methyl-1H-imidazole-4-carboxylate

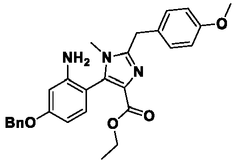

5-(benzyloxy)-2-(4,4,5,5-tetramethyl-1,3,2-dioxaborolan-2-yl)aniline (2.68 g, 8.25 mmol) synthesized in [Process 4] above and [Intermediate 7-1] ethyl 5-iodo-2-(4-methoxybenzyl)-1-methyl-1*H*-imidazole-4-carboxylate (3 g, 7.50 mmol) were added to anhydrous 1,4-dioxane (30 mL), water (15 mL), to substitute nitrogen gas, followed by adding sodium hydrogen carbonate (1.9 g, 22.49 mmol), tetrakis(triphenylphosphine)palladium(0) (446 mg, 0.37 mmol). The mixed solution was refluxed at 90 °C for 14 hours and cooled to room temperature after completion of the reaction. A saturated aqueous solution of ammonium chloride was added to the reaction product and was subjected to extraction with ethyl acetate, and the separated organic layer was dried with anhydrous sodium sulfate and vacuum distilled. The obtained residue was purified by silica gel column chromatography to obtain the title compound (1.4 g, 39.61 %).

### [Process 6] Preparation of 7-(benzyloxy)-2-(4-methoxybenzyl)-1-methyl-5-(2-methylpyridin-3-yl)-1,5-dihydro-4H-imidazo[4,5-c]quinolin-4-one

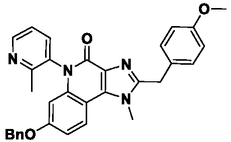

The same reaction was carried out using ethyl 5-(2-amino-4-(benzyloxy)phenyl)-2-(4-methoxybenzyl)-1-methyl-1*H*-imidazole-4-carboxylate instead of ethyl 5-(2-amino-4-chlorophenyl)-2-(3-methoxybenzyl)-1-methyl-1*H*-imidazole-4-carboxylate, in [Process 3] of Example 21, to obtain the title compound (1.4 g, 91.28 %).

### [Process 7] Preparation of 7-hydroxy-2-(4-methoxybenzyl)-1-methyl-5-(2-methylpyridin-3-yl)-1,5-dihydro-4H-imidazo[4,5-c]quinolin-4-one [Example 43]

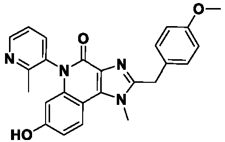

Acetic acid (1 mL) and a 38 % aqueous hydrochloric acid solution (1 mL) were added to 7-(benzyloxy)-2-(4-methoxybenzyl)-1-methyl-5-(2-methylpyridin-3-yl)-1,5-dihydro-4*H*-imidazo[4,5-c]quinolin-4-one (1.4 g, 2.71 mmol) synthesized in [Process 6] above. The mixed solution was refluxed at 140 °C for 7 hours and cooled to room temperature after completion of the reaction. The reaction product was vacuum distilled, followed by adding a saturated aqueous solution of sodium hydrogen carbonate, stirred for 30 minutes, and was subjected to extraction with dichloromethane and methanol. The separated organic layer was dried with anhydrous sodium sulfate and vacuum distilled. The obtained residue was purified by silica gel column chromatography to obtain the title compound (630 mg, 54.51 %).

### [Process 8] Preparation of 2-(4-methoxybenzyl)-1-methyl-5-(2-methylpyridin-3-yl)-4-oxo-4,5-dihydro-1H-imidazo[4,5-c]quinolin-7-yl trifluoromethanesulfonate

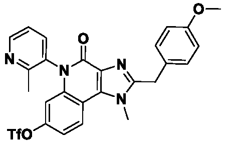

7-hydroxy-2-(4-methoxybenzyl)-1-methyl-5-(2-methylpyridin-3-yl)-1,5-dihydro-4H-imidazo[4,5-c]quinolin-4-one (150 mg, 0.35 mmol) synthesized in [Process 7] above was dissolved in dichloromethane (1.5 mL), followed by adding triethylamine (0.10 mL, 0.70 mmol) and 1,1,1-trifluoro-*N*-phenyl-*N*-((trifluoromethyl)sulfonyl)methanesulfonamide (192 mg, 0.53 mmol). The reaction solution was stirred at room temperature for 4 hours and then vacuum distilled. The obtained residue was purified by silica gel column chromatography to obtain the title compound (196 mg, 100 %).

### [Process 9] Preparation of 2-(4-methoxybenzyl)-1-methyl-5-(2-methylpyridin-3-yl)-7-vinyl-1,5-dihydro-4H-imidazo[4,5-c]quinolin-4-one [Example 44]

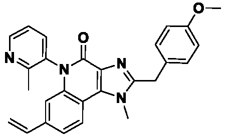

(4-methoxybenzyl)-1-methyl-5-(2-methylpyridin-3-yl)-4-oxo-4,5-dihydro-1*H-*imidazo[4,5-*c*]quinolin-7-yl trifluoromethanesulfonate (196 mg, 0.35 mmol) synthesized in [Process 8] above was dissolved in anhydrous 1,4-dioxane (2 mL), substituted with argon gas, followed by adding potassium carbonate (146 mg, 1.05 mmol), tetrakis(triphenylphosphine) palladium(0) (41.8 mg, 0.04 mmol) and 4,4,5,5-tetramethyl-2-vinyl-1,3,2-dioxaborolane (0.09 mL, 0.53 mmol). The mixed solution was refluxed at 100 °C for 2 hours and cooled to room temperature after completion of the reaction. Water was added to the reaction product and was subjected to extraction with ethyl acetate, and the separated organic layer was dried with anhydrous sodium sulfate and vacuum distilled. The obtained residue was purified by silica gel column chromatography to obtain the title compound (145 mg, 94.66 %).

### [Process 10] Preparation of 7-ethyl-2-(4-methoxybenzyl)-1-methyl-5-(2-methylpyridin-3-yl)-1,5-dihydro-4H-imidazo[4,5-c]quinolin-4-one [Example 45]

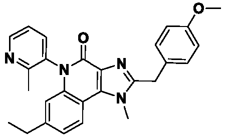

2-(4-methoxybenzyl)-1-methyl-5-(2-methylpyridin-3-yl)-7-vinyl-1,5-dihydro-4*H-*imidazo[4,5-*c*]quinolin-4-one (145 mg, 0.33 mmol) synthesized in [Process 9] above and 10 % palladium/carbon (177 mg) were dissolved in methanol (3.32 mL) and was fitted with a hydrogen balloon. The reaction solution was stirred at room temperature for 3 hours. The reaction mixture was filtered through celite and vacuum distilled, and the obtained residue was purified by silica gel column chromatography to obtain the title compound (63 mg, 43.25 %).

### Example 46: 7-cyclopropyl-2-(4-methoxybenzyl)-1-methyl-5-(2-methylpyridin-3-yl)-1,5-dihydro-4H-imidazo[4,5-c]quinolin-4-one

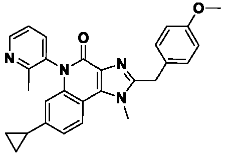

The same reaction was carried out using cyclopropyl boronic acid instead of 4,4,5,5-tetramethyl-2-vinyl-1,3,2-dioxaborolane, in [Process 9] of <Synthesis Example 7>, to obtain the title compound (5 mg, 65.79 %).

### Example 47: 7-ethynyl-2-(4-methoxybenzyl)-1-methyl-5-(2-methylpyridin-3-yl)-1,5-dihydro-4H-imidazo[4,5-c]quinolin-4-one

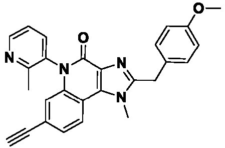

2-(4-methoxybenzyl)-1-methyl-5-(2-methylpyridin-3-yl)-4-oxo-4,5-dihydro-1*H-*imidazo[4,5-c]quinolin-7-yl trifluoromethanesulfonate (20 mg, 0.04 mmol) synthesized in [Process 8] of <Synthesis Example 7>, ethynyltrimethylsilane (0.02 mL, 0.18 mmol), tetrakis(triphenylphosphine)palladium(0) (4.3 mg, 0.004 mmol), cuprous iodide (0.68 mg, 0.004 mmol), trimethylamine (0.01 mL, 0.07 mmol) were dissolved in *N,N-*dimethylformamide (1 mL) and the mixed solution was stirred at 40 °C for 18 hours. After completion of the reaction, the reaction product was cooled to room temperature, followed by adding water and was subjected to extraction with ethyl acetate, and the separated organic layer was dried with anhydrous sodium sulfate and vacuum distilled. The obtained residue was purified by silica gel column chromatography to obtain 2-(4-methoxybenzyl)-1-methyl-5-(2-methylpyridin-3-yl)-7-((trimethylsilyl)ethynyl)-1,5-dihydro-4*H*-imidazo[4,5-c]quinolin-4-one (14 mg, 77.16 %).

2-(4-methoxybenzyl)-1-methyl-5-(2-methylpyridin-3-yl)-7-((trimethylsilyl)ethynyl)-1,5-dihydro-4*H*-imidazo[4,5-*c*]quinolin-4-one (14 mg, 0.03 mmol) and potassium carbonate (38 mg, 0.28 mmol) were dissolved in methanol (0.28 mL) and stirred at room temperature for 16 hours. After completion of the reaction, water was added to the reaction product and was subjected to extraction with dichloromethane, and the separated organic layer was dried with anhydrous sodium sulfate and vacuum distilled. The obtained residue was purified by silica gel column chromatography to obtain the title compound (2.4 mg, 19.19 %).

### Example 48: 7-isopropyl-2-(4-methoxybenzyl)-1-methyl-5-(2-methylpyridin-3-yl)-1,5-dihydro-4H-imidazo[4,5-c]quinolin-4-one

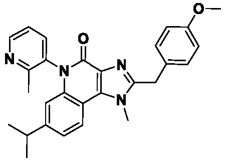

The same reaction was carried out using 2-(4-methoxybenzyl)-1-methyl-5-(2-methylpyridin-3-yl)-7-(prop-1-en-2-yl)-1,5-dihydro-4*H*-imidazo[4,5-*c*]quinolin-4-one instead of 2-(4-methoxybenzyl)-1-methyl-5-(2-methylpyridin-3-yl)-7-vinyl-1,5-dihydro-4*H-*imidazo[4,5-*c*]quinolin-4-one, in [Process 10] of <Synthesis Example 7>, to obtain the title compound (11.5 mg, 77.55 %).

### Example 49: 2-(4-methoxybenzyl)-1,7-dimethyl-5-(2-methylpyridin-3-yl)-1,5-dihydro-4H-imidazo[4,5-c]quinolin-4-one

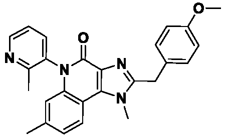

The same reaction was carried out using 2,4,6-trimethyl-1,3,5,2,4,6-trioxatriborinane instead of 4,4,5,5-tetramethyl-2-vinyl-1 ,3,2-dioxaborolane, in [Process 9] of <Synthesis Example 7>, to obtain the title compound (3 mg, 37.19 %).

### Example 50: 2-(4-methoxybenzyl)-1-methyl-5-(2-methylpyridin-3-yl)-7-(pyrrolidin-1-yl)-1,5-dihydro-4H-imidazo[4,5-c]quinolin-4-one

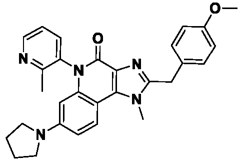

2-(4-methoxybenzyl)-1-methyl-5-(2-methylpyridin-3-yl)-4-oxo-4,5-dihydro-1*H-*imidazo[4,5-*c*]quinolin-7-yl trifluoromethanesulfonate (10 mg, 0.02 mmol) synthesized in [Process 8] of <Synthesis Example 7> and cesium carbonate (11.78 mg, 0.04 mmol), palladium acetate (0.41 mg, 0.002 mmol), 2,2'-bis(diphenylphosphino)-1,1'-binaphthalene (1.7 mg, 0.002 mmol), pyrrolidine (0.005 mL, 0.05 mmol) were dissolved in anhydrous 1,4-dioxane (0.2 mL), and substituted with argon gas. The mixed solution was refluxed at 100 °C for 1 hour and cooled to room temperature after completion of the reaction. Water was added to the reaction product and was subjected to extraction with ethyl acetate, and the separated organic layer was dried with anhydrous sodium sulfate and vacuum distilled. The obtained residue was purified by silica gel column chromatography to obtain the title compound (2 mg, 23.29 %).

### Example 51: 7-methoxy-2-(4-methoxybenzyl)-1-methyl-5-(2-methylpyridin-3-yl)-1,5-dihydro-4H-imidazo[4,5-c]quinolin-4-one

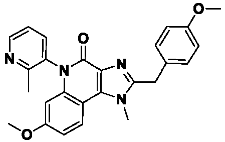

7-hydroxy-2-(4-methoxybenzyl)-1-methyl-5-(2-methylpyridin-3-yl)-1,5-dihydro-4*H*-imidazo[4,5-c]quinolin-4-one (10 mg, 0.03 mmol) synthesized in [Process 7] of <Synthesis Example 7> was dissolved in *N,N*-dimethylformamide (0.23 mL), followed by adding potassium carbonate (6.5 mg, 0.05 mmol) and methyl iodide (0.003 mL, 0.05 mmol). The mixed solution was stirred at room temperature for 1 hour, then water and ethyl acetate were added and was subjected to extraction with ethyl acetate. The separated organic layer was dried with anhydrous sodium sulfate and vacuum distilled. The obtained residue was purified by silica gel column chromatography to obtain the title compound (4.3 mg, 41.63 %).

### Example 52: 7-amino-2-(4-methoxybenzyl)-1-methyl-5-(2-methylpyridin-3-yl)-1,5-dihydro-4H-imidazo[4,5-c]quinolin-4-one

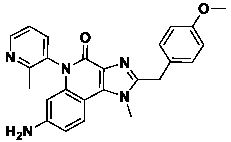

2-(4-methoxybenzyl)-1-methyl-5-(2-methylpyridin-3-yl)-4-oxo-4,5-dihydro-1*H-*imidazo[4,5-c]quinolin-7-yl trifluoromethanesulfonate (100 mg, 0.18 mmol) synthesized in [Process 8] of <Synthesis Example 7> and cesium carbonate (117.85 mg, 0.36 mmol), palladium acetate (4.1 mg, 0.02 mmol), 2,2'-bis(diphenylphosphino)-1,1'-binaphthalene (17.24 mg, 0.03 mmol), benzophenoneimine (0.15 mL, 0.90 mmol) were dissolved in anhydrous 1,4-dioxane (1 mL), and substituted with argon gas. The mixed solution was refluxed at 100 °C for 2 hours and cooled to room temperature after completion of the reaction. The reaction product was vacuum distilled and purified by silica gel column chromatography to obtain 7-((diphenylmethylene)amino)-2-(4-methoxybenzyl)-1-methyl-5-(2-methylpyridin-3-yl)-1,5-dihydro-4*H*-imidazo[4,5-*c*]quinolin-4-one (63.8 mg).

The obtained intermediate was dissolved in tetrahydrofuran (1 mL) and 1 N aqueous hydrochloric acid solution (0.1 mL) and stirred at room temperature for 30 minutes. After completion of the reaction, sodium hydrogen carbonate saturated aqueous solution was added and the mixture was subjected to extraction with ethyl acetate, and the separated organic layer was dried with anhydrous sodium sulfate and vacuum distilled. The obtained residue was purified by silica gel column chromatography to obtain the title compound (39 mg, 51.19 %).

### Example 53: 2-(4-methoxybenzyl)-1-methyl-7-(methylamino)-5-(2-methylpyridin-3-yl)-1,5-dihydro-4H-imidazo[4,5-c]quinolin-4-one

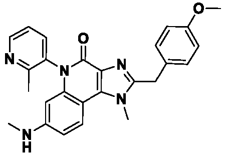

7-amino-2-(4-methoxybenzyl)-1-methyl-5-(2-methylpyridin-3-yl)-1,5-dihydro-4*H-*imidazo[4,5-*c*]quinolin-4-one (10 mg, 0.02 mmol) synthesized in Example 52, a 25 wt% sodium methoxide methanol solution (0.05 mL) and 10 % palladium/carbon (0.5 mg, 0.0005 mmol) were dissolved in methanol (0.5 mL) and refluxed at 130 °C for 3 days and cooled to room temperature after completion of the reaction. The reaction mixture was filtered through celite and vacuum distilled, and the obtained residue was purified by silica gel column chromatography to obtain the title compound (4 mg, 38.72 %).

### Example 54: 2-(4-(difluoromethoxy)benzyl)-7-ethyl-1-methyl-5-(2-methylpyridin-3-yl)-1,5-dihydro-4H-imidazo[4,5-c]quinolin-4-one

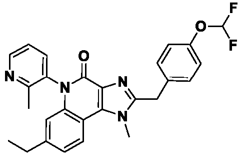

The same reaction was carried out using ethyl 5-(2-amino-4-ethylphenyl)-2-(4-(difluoromethoxy)benzyl)-1-methyl-1*H*-imidazole-4-carboxylate instead of ethyl 5-(2-amino-4-chlorophenyl)-2-(3-methoxybenzyl)-1-methyl-1*H*-imidazole-4-carboxylate, in [Process 3] of Example 21, to obtain the title compound (290 mg, 52.50 %).

### Example 55: 2-(4-(difluoromethoxy)benzyl)-7-methoxy-1-methyl-5-(2-methylpyridin-3-yl)-1,5-dihydro-4H-imidazo[4,5-c]quinolin-4-one

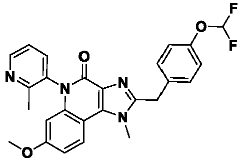

The same reaction was carried out using ethyl 5-(2-amino-4-methoxyphenyl)-2-(4-(difluoromethoxy)benzyl)-1-methyl-1*H*-imidazole-4-carboxylate instead of ethyl 5-(2-amino-4-chlorophenyl)-2-(3-methoxybenzyl)-1-methyl-1*H*-imidazole-4-carboxylate, in [Process 3] of Example 21, to obtain the title compound (124 mg, 61.69 %).

According to an embodiment of the present disclosure, the compound of Formula 1 may be a compound selected from the group consisting of the compounds listed in [Table 4] below.

**[Table 4]**

| Compound No. | Structure | Nomenclature | MS[M+H]⁺ |
|---|---|---|---|
| | 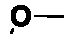 ¹H-NMR spectrum (300MHz) | | |
| 43 | 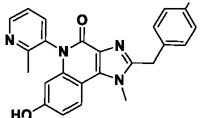 | 7-Hydroxy-2-(4-methoxybenzyl)-1-methyl-5-(2-methylpyridin-3-yl)-1,5-dihydro-4*H*-imidazo[4,5-*c*]quinolin-4-one | 427.17 |
| | ¹H-NMR spectrum (300 MHz, CDCl₃) *δ* 8.61 (dd, 1H), 7.94(d, 1H), 7.63(dd, 1H), 7.45(dd, 1H), 7.18(d, 2H), 6.87(d, 2H), 6.84(m, 1H), 6.04(d, 1H), 4.31(s, 2H), 3.95(s, 3H), 3.79(s, 3H), 2.28(s, 3H) | | |
| 44 | 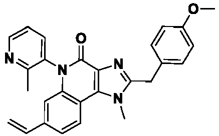 | 2-(4-methoxybenzyl)-1-methyl-5-(2-methylpyridin-3-yl)-7-vinyl-1,5-dihydro-4*H*-imidazo[4,5-*c*]quinolin-4-one | 437.19 |
| | ¹H-NMR spectrum (300 MHz, CDCl₃) *δ* 8.70(dd, 1H), 8.00(d, 1H), 7.56(dd, 1H), 7.38(m, 2H), 7.20(d, 2H), 6.86(d, 2H), 6.55(m, 1H), 5.63(d, 1H), 5.26(d, 1H), 4.36(s, 2H), 3.97(s, 3H), 3.79(s, 3H), 2.29(s, 3H) | | |
| 45 | 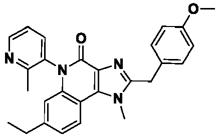 | 7-Ethyl-2-(4-methoxybenzyl)-1-methyl-5-(2-methylpyridin-3-yl)-1,5-dihydro-4*H*-imidazo[4,5-*c*]quinolin-4-one | 439.21 |
| | ¹H-NMR spectrum (300 MHz, CDCl₃) *δ* 8.69(dd, 1H), 7.96(d, 1H), 7.56(dd, 1H), 7.39(dd, 1H), 7.19(d, 2H), 7.12(dd, 1H), 6.85(d, 2H), 6.38(s, 1H), 4.35(s, 2H), 3.95(s, 3H), 3.79(s, 3H), 2.56(dd, 2H), 2.28(s, 3H), 1.12(t, 3H) | | |
| 46 | 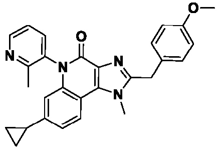 | 7-Cyclopropyl-2-(4-methoxybenzyl)-1-methyl-5-(2-methylpyridin-3-yl)-1,5-dihydro-4*H*-imidazo[4,5-*c*]quinolin-4-one | 451.21 |
| | ¹H-NMR spectrum (300 MHz, CDCl₃) *δ* 8.68(m, 1H), 7.91(d, 1H), 7.55(m, 1H), 7.39(dd, 1H), 7.19(d, 2H), 6.86(m, 3H), 6.32(d, 1H), 4.34(s, 2H), 3.94(s, 3H), 3.79(s, 3H), 2.29(s, 3H), 1.77(m, 1H), 0.94(m, 2H), 0.57(m, 2H) | | |
| 47 | 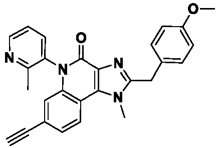 | 7-ethynyl-2-(4-methoxybenzyl)-1-methyl-5-(2-methylpyridin-3-yl)-1,5-dihydro-4*H*-imidazo[4,5-*c*]quinolin-4-one | 435.17 |
| | ¹H-NMR spectrum (300 MHz, CDCl₃) *δ* 8.71 (dd, 1H), 7.99(d, 1H), 7.55(dd, 1H), 7.42(m, 1H), 7.36(dd, 1H), 7.20(d, 2H), 6.86(d, 2H), 6.71(d, 1H), 4.36(s, 2H), 3.97(s, 3H), 3.79(s, 3H), 3.10(s, 1H), 2.29(s, 3H) | | |
| 48 | 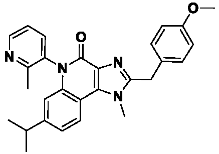 | 7-isopropyl-2-(4-methoxybenzyl)-1-methyl-5-(2-methylpyridin-3-yl)-1,5-dihydro-4*H*-imidazo[4,5-*c*]quinolin-4-one | 453.22 |
| | ¹H-NMR spectrum (300 MHz, CDCl₃) *δ* 8.69(dd, 1H), 7.97(d, 1H), 7.57(dd, 1H), 7.39(dd, 1H), 7.16(m, 3H), 6.85(d, 2H), 6.39(d, 1H), 4.35(s, 2H), 3.95(s, 3H), 3.79(s, 3H), 2.78(m, 1H), 2.28(s, 3H), 1.13(d, 6H) | | |
| 49 | 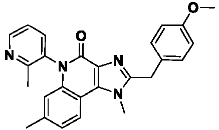 | 2-(4-methoxybenzyl)-1,7-dimethyl-5-(2-methylpyridin-3-yl)-1,5-dihydro-4*H-*imidazo[4,5-*c*]quinolin-4-one | 425.19 |
| | ¹H-NMR spectrum (300 MHz, CDCl₃) *δ* 8.69(dd, 1H), 7.93(d, 1H), 7.55(dd, 1H), 7.39(m, 1H), 7.20(d, 2H), 7.08(d, 1H), 6.85 (d, 2H), 6.37(s, 1H), 4.35(s, 2H), 3.95(s, 3H), 3.79(s, 3H), 2.28(s, 6H) | | |
| 50 | 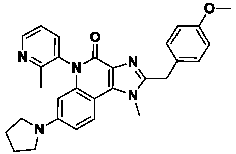 | 2-(4-methoxybenzyl)-1-methyl-5-(2-methylpyridin-3-yl)-7-(pyrrolidin-1-yl)-1,5-dihydro-4*H*-imidazo[4,5-*c*]quinolin-4-one | 480.23 |
| | ¹H-NMR spectrum (300 MHz, CDCl₃) *δ* 8.66(dd, 1H), 7.84(d, 1H), 7.57(dd, 1H), 7.36(dd, 1H), 7.20(d, 2H), 6.86(d, 2H), 6.51(dd, 1H), 5.50(d, 1H), 4.31(s, 2H), 3.89(s, 3H), 3.79(s, 3H), 3.08(m, 4H), 2.32(s, 3H), 1.94(m, 4H) | | |
| 51 | 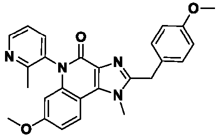 | 7-methoxy-2-(4-methoxybenzyl)-1-methyl-5-(2-methylpyridin-3-yl)-1,5-dihydro-4*H*-imidazo[4,5-*c*]quinolin-4-one | 441.18 |
| | ¹H-NMR spectrum (300 MHz, CDCl₃) *δ* 8.68(dd, 1H), 7.95(d, 1H), 7.55(dd, 1H), 7.38(dd, 1H), 7.20(d, 2H), 6.85(m, 3H), 6.05(d, 1H), 4.34(s, 2H), 3.93(s, 3H), 3.79(s, 3H), 3.67(s, 3H), 2.30(s, 3H) | | |
| 52 | 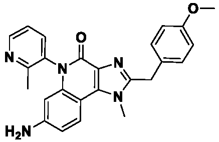 | 7-amino-2-(4-methoxybenzyl)-1-methyl-5-(2-methylpyridin-3-yl)-1,5-dihydro-4*H*-imidazo[4,5-*c*]quinolin-4-one | 426.19 |
| | ¹H-NMR spectrum (300 MHz, CDCl₃) *δ* 8.66(m, 1H), 7.81 (d, 1H), 7.55(d, 1H), 7.38(dd, 1H), 7.19(d, 2H), 6.85(d, 2H), 6.59(dd, 1H), 5.77(d, 1H), 4.32(s, 2H), 3.39(s, 3H), 3.79(s, 3H), 2.31(s, 3H) | | |
| 53 | 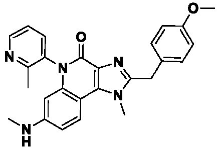 | 2-(4-methoxybenzyl)-1-methyl-7-(methylamino)-5-(2-methylpyridin-3-yl)-1,5-dihydro-4*H*-imidazo[4,5-*c*]quinolin-4-one | 440.20 |
| | ¹H-NMR spectrum (300 MHz, CDCl₃) *δ* 8.66(dd, 1H), 7.83(d, 1H), 7.55(dd, 1H), 7.37(dd, 1H), 7.19(d, 2H), 6.85(d, 2H), 6.54(dd, 1H), 5.62(d, 1H), 4.31(s, 2H), 3.89(s, 3H), 3.79(s, 3H), 2.68(s, 3H), 2.31(s, 3H) | | |
| 54 | 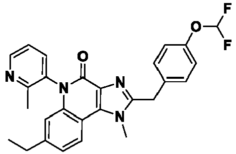 | 2-(4-(difluoromethoxy)benzyl)-7-ethyl-1-methyl-5-(2-methylpyridin-3-yl)-1,5-dihydro-4*H*-imidazo[4,5-*c*]quinolin-4-one | 475.19 |
| | ¹H-NMR spectrum (300 MHz, MeOD) *δ* 8.67(dd, 1H), 8.25(d, 1H), 7.75(dd, 1H), 7.57(dd, 1H), 7.31(m, 3H), 7.12(d, 2H), 6.78(t, 1H), 6.44(s, 1H), 4.41(s, 2H), 4.08(s, 3H), 2.59(dd, 2H), 2.20(s, 3H), 1.11 (t, 3H) | | |
| 55 | 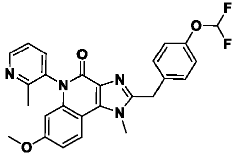 | 2-(4-(Difluoromethoxy)benzyl)-7-methoxy-1-methyl-5-(2-methylpyridin-3-yl)-1,5-dihydro-4*H*-imidazo[4,5-*c*]quinolin-4-one | 477.17 |
| | ¹H-NMR spectrum (300 MHz, CDCl₃) *δ* 8.68(dd, 1H), 7.96(d, 1H), 7.56(dd, 1H), 7.38(dd, 1H), 7.29(d, 2H), 7.09(d, 2H), 6.86(dd, 1H), 6.49(t, 1H), 6.06(d, 1H), 4.38(s, 2H), 3.94(s, 3H), 3.67(s, 3H), 2.30(s, 3H) | | |

### <Synthesis Example 8>

### Example 56: 5-(4-(difluoromethoxy)phenyl)-2-(4-methoxybenzyl)-1-methyl-7-(trifluoromethyl)-1,5-dihydro-4H-imidazo[4,5-c][1,8]naphthyridin-4-one

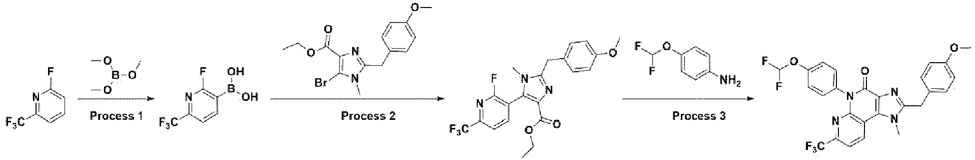

### [Process 1] Preparation of (2-fluoro-6-(trifluoromethyl)pyridin-3-yl)boronic acid

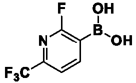

2-fluoro-6-(trifluoromethyl)pyridine (15 g, 80.04 mmol) was dissolved in anhydrous tetrahydrofuran (150 mL), and substituted with nitrogen gas. The mixed solution was cooled to -78 °C and then 2.0 M lithium diisopropylamide solution (53.43 mL, 106.85 mmol) was slowly added dropwise and stirred at the same temperature for 1 hour. Trimethylboronic acid (12 mL, 106.85 mmol) was slowly added dropwise and stirred at room temperature for 22 hours. To the reaction solution 5 wt% aqueous sodium hydroxide solution (200 mL) was added, then neutralized with 5 N aqueous hydrochloric acid solution and was subjected to extraction with ethyl acetate. The separated organic layer was dried with anhydrous sodium sulfate, and vacuum distilled to obtain the title compound (16 g, 86.01 %).

### [Process 2] Preparation of ethyl 5-(2-fluoro-6-(trifluoromethyl)pyridin-3-yl)-2-(4-methoxybenzyl)-1-methyl-1H-imidazole-4-carboxylate

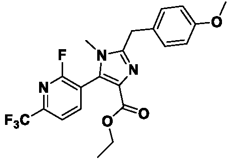

(2-fluoro-6-(trifluoromethyl)pyridin-3-yl)boronic acid (1.18 g, 5. 66 mmol) synthesized in [Process 1] and ethyl 5-bromo-2-(4-methoxybenzyl)-1-methyl-1*H-*imidazole-4-carboxylate (1.00 g, 2.83 mmol) obtained from [Intermediate 6-2] were added to anhydrous 1,4-dioxane (10 mL), water (1 mL), to substitute nitrogen gas, followed by adding cesium carbonate (1.11 g, 7.08 mmol), 2-dicyclohexylphosphino-2', 6'-diisopropoxybiphenyl (67.4 mg, 0.14 mmol), (2-dicyclohexylphosphino-2', 6'-diisopropoxy-1,1'-biphenyl)[2-(2'-amino-1,1'-biphenyl)]palladium(II) methanesulfonate (123 mg, 0.14 mmol). The mixed solution was refluxed at 120 °C for 14 hours and cooled to room temperature after completion of the reaction. Water was added to the reaction product and was subjected to extraction with dichloromethane and methanol, and the separated organic layer was dried with anhydrous magnesium sulfate and vacuum distilled. The obtained residue was purified by silica gel column chromatography to obtain the title compound (539 mg, 43.53 %).

### [Process 3] Preparation of 5-(4-(difluoromethoxy)phenyl)-2-(4-methoxybenzyl)-1-methyl-7-(trifluoromethyl)-1,5-dihydro-4H-imidazo[4,5-c][1,8]naphthyridin-4-one

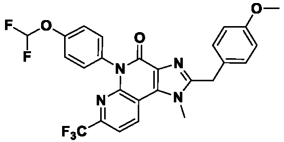

Ethyl 5-(2-fluoro-6-(trifluoromethyl)pyridin-3-yl)-2-(4-methoxybenzyl)-1-methyl-1H-imidazole-4-carboxylate (100 mg, 0.23 mmol) and 4-(difluoromethoxy)aniline (45 mg, 0.27 mmol) synthesized in [Process 2] above were dissolved in anhydrous tetrahydrofuran (2.28 mL), and substituted with nitrogen gas. The mixed solution was cooled to 0 °C and a 1.0 M lithium bis(trimethylsilyl)amide tetrahydrofuran solution (0.69 mL, 0.69 mmol) was slowly added dropwise. The mixed solution was stirred at room temperature for 1 hour, after completion of the reaction, water was added and was subjected to extraction with dichloromethane and methanol. The separated organic layer was dried with anhydrous magnesium sulfate and vacuum distilled. The obtained residue was purified by silica gel column chromatography to obtain the title compound (17.8 mg, 14.68 %).

### Example 57: 2-(4-methoxybenzyl)-1-methyl-5-(2-methylpyridin-3-yl)-7-(trifluoromethyl)-1,5-dihydro-4H-imidazo[4,5-c][1,8]naphthyridin-4-one

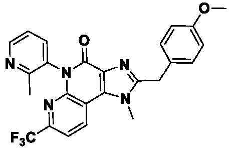

The same reaction was carried out using 2-methylpyridin-3-amine instead of 4-(difluoromethoxy)aniline, in [Process 3] of <Synthesis Example 8>, to obtain the title compound (27.3 mg, 19.16 %).

### Example 58: 2-(4-(difluoromethoxy)benzyl)-1-methyl-5-(2-methylpyridin-3-yl)-7-(trifluoromethyl)-1,5-dihydro-4H-imidazo[4,5-c][1,8]naphthyridin-4-one

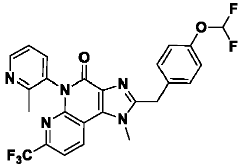

The same reaction was carried out using ethyl 2-(4-(difluoromethoxy)benzyl)-5-(2-fluoro-6-(trifluoromethyl)pyridin-3-yl)-1-methyl-1*H*-imidazole-4-carboxylate and 2-methylpyridin-3-amine instead of ethyl 5-(2-fluoro-6-(trifluoromethyl)pyridin-3-yl)-2-(4-methoxybenzyl)-1-methyl-1*H*-imidazole-4-carboxylate and 4-(difluoromethoxy)aniline, in [Process 3] of <Synthesis Example 8>, to obtain the title compound (150 mg, 68.88 %).

### Example 59: 2-((4-methoxyphenyl)thio)-1-methyl-5-(2-methylpyridin-3-yl)-7-(trifluoromethyl)-1,5-dihydro-4H-imidazo[4,5-c][1,8]naphthyridin-4-one

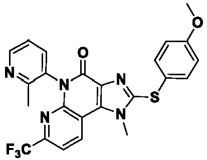

The same reaction was carried out using 1-methyl-5-(2-methylpyridin-3-yl)-7-(trifluoromethyl)-1,5-dihydro-4*H*-imidazo[4,5-*c*][1,8]naphthyridin-4-one instead of 7-chloro-1-methyl-5-phenyl-1,5-dihydro-4*H*-imidazo[4,5-*c*]quinolin-4-one, in [Process 2] of Example 18, to obtain the title compound (81 mg, 71.42 %).

### Example 60: 2-((4-fluorophenyl)thio)-1-methyl-5-(2-methylpyridin-3-yl)-7-(trifluoromethyl)-1,5-dihydro-4H-imidazo[4,5-c][1,8]naphthyridin-4-one

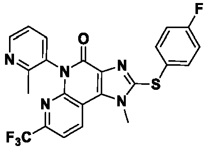

The same reaction was carried out using 1-methyl-5-(2-methylpyridin-3-yl)-7-(trifluoromethyl)-1,5-dihydro-4*H*-imidazo[4,5-*c*][1,8]naphthyridin-4-one and 4-fluorobenzenthiol instead of 7-chloro-1-methyl-5-phenyl-1,5-dihydro-4*H*-imidazo[4,5-*c*]quinolin-4-one and 4-methoxybenzenthiol, in [Process 2] of Example 18, to obtain the title compound (70 mg, 64.76 %).

### Example 61: 2-((4-methoxyphenyl)thio)-1-methyl-5-(2-methylpyridin-3-yl)-7-(trifluoromethyl)-1,5-dihydro-4H-imidazo[4',5':4,5]pyrido[2,3-d]pyrimidin-4-one

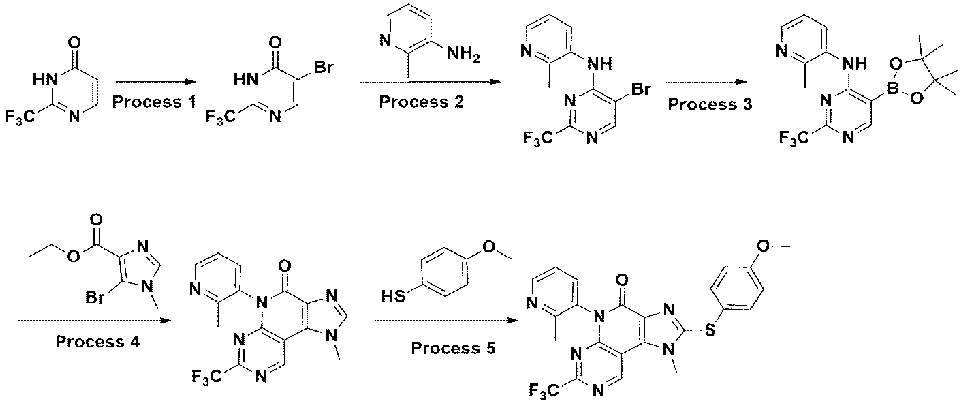

### [Process 1] Preparation of 5-bromo-2-(trifluoromethyl)pyrimidin-4(3H)-one

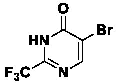

2-(Trifluoromethyl)pyrimidin-4(3*H*)-one (6.18 g, 35.8 mmol) was dissolved in *N,N-*dimethylformamide (50 mL), followed by adding *N*-bromosuccinimide (7.80 g, 42.9 mmol). The mixed solution was stirred at 40 °C for 22 hours and cooled to room temperature after completion of the reaction. Water was added to the reaction solution and was subjected to extraction with ethyl acetate, and the separated organic layer was dried with anhydrous sodium sulfate, and vacuum distilled to obtain the title compound (8.69 g, 100 %).

### [Process 2] Preparation of 5-bromo-N-(2-methylpyridin-3-yl)-2-(trifluoromethyl)pyrimidin-4-amine

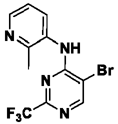

5-bromo-2-(trifluoromethyl)pyrimidin-4(3H)-one (8.69 g, 35.76 mmol) synthesized in [Process 1] above was dissolved in tetrahydrofuran (86.9 mL), followed by adding 2,6-lutidine (6.3 mL, 53.6 mmol) and cooled to -78 °C. After 30 minutes, trifluoromethanesulfonic anhydride (8.0 mL, 46.5 mmol) was slowly added dropwise at the same temperature and stirred at 0 °C for 3 hours. 2-methylpyridin-3-amine (11.8 g, 107 mmol) was added to the reaction solution and refluxed at 70 °C for 4 hours. After completion of the reaction, the reaction solution was cooled to room temperature, water was added to the reaction solution and was subjected to extraction with ethyl acetate, and the separated organic layer was dried with anhydrous sodium sulfate and vacuum distilled. The obtained residue was purified by silica gel column chromatography to obtain the title compound (5.47 g, 45.92 %).

### [Process 3] Preparation of N-(2-methylpyridin-3-yl)-5-(4,4,5,5-tetramethyl-1,3,2-dioxaborolan-2-yl)-2-(trifluoromethyl)pyrimidin-4-amine

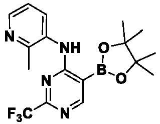

5-bromo-*N*-(2-methylpyridin-3-yl)-2-(trifluoromethyl)pyrimidin-4-amine (717 mg, 1.50 mmol) synthesized in [Process 2] above was added to anhydrous 1,4-dioxane (5 mL) to substitute nitrogen gas followed by adding bis(pinacolato)diboron (837 mg, 3.23 mmol), [1,1'-bis(diphenylphosphino)ferrocene]dichloropalladium(II) (166 mg, 0.22 mmol) and potassium acetate (640 mg, 6.46 mmol). The mixed solution was refluxed at 100 °C for 19 hours and cooled to room temperature after completion of the reaction. Water was added to the reaction product and was subjected to extraction with ethyl acetate, and the separated organic layer was dried with anhydrous magnesium sulfate and vacuum distilled. The obtained residue was purified by silica gel column chromatography to obtain the title compound (240 mg, 29.33 %).

### [Process 4] Preparation of 1-methyl-5-(2-methylpyridin-3-yl)-7-(trifluoromethyl)-1,5-dihydro-4H-imidazo[4',5':4,5]pyrido[2,3-d]pyrimidin-4-one

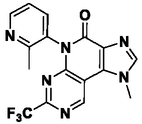

*N*-(2-methylpyridin-3-yl)-5-(4,4,5,5-tetramethyl-1,3,2-dioxaborolan-2-yl)-2-(trifluoromethyl)pyrimidin-4-amine (798 mg, 1.05 mmol) synthesized in [Process 3] above was added to 1,4-dioxane (1 mL) and water (0.1 mL), to substitute nitrogen gas, followed by adding ethyl 5-bromo-1-methyl-1*H*-imidazole-4-carboxylate (245 mg, 1.05 mmol) obtained from [Intermediate 6-3], fluorocesium (493 mg, 3.15 mmol), [1,1'-bis(di-tert-butylphosphino)ferrocene]dichloropalladium(II) (70.5 mg, 0.11 mmol). The mixed solution was refluxed at 100 °C for 18 hours and cooled to room temperature after completion of the reaction. Water was added to the reaction product and was subjected to extraction with ethyl acetate, and the separated organic layer was dried with anhydrous magnesium sulfate and vacuum distilled. The obtained residue was purified by silica gel column chromatography to obtain the title compound (17 mg, 4.49 %).

### [process 5] Preparation of 2-((4-methoxyphenyl)thio)-1-methyl-5-(2-methylpyridin-3-yl)-7-(trifluoromethyl)-1,5-dihydro-4H-imidazo[4',5':4,5]pyrido[2,3-d]pyrimidin-4-one

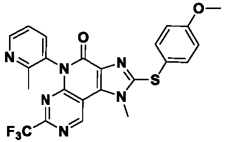

The same reaction was carried out using 1-methyl-5-(2-methylpyridin-3-yl)-7-(trifluoromethyl)-1,5-dihydro-4*H*-imidazo[4',5':4,5]pyrido[2,3-*d*]pyrimidin-4-one instead of 7-chloro-1-methyl-5-phenyl-1,5-dihydro-4*H*-imidazo[4,5-c]quinolin-4-one, in [Process 2] of Example 18, to obtain the title compound (7.8 mg, 33.16 %).

### Example 62: 7-ethoxy-2-(4-methoxybenzyl)-1-methyl-5-(2-methylpyridin-3-yl)-1,5-dihydro-4H-imidazo[4',5':4,5]pyrido[2,3-d]pyrimidin-4-one

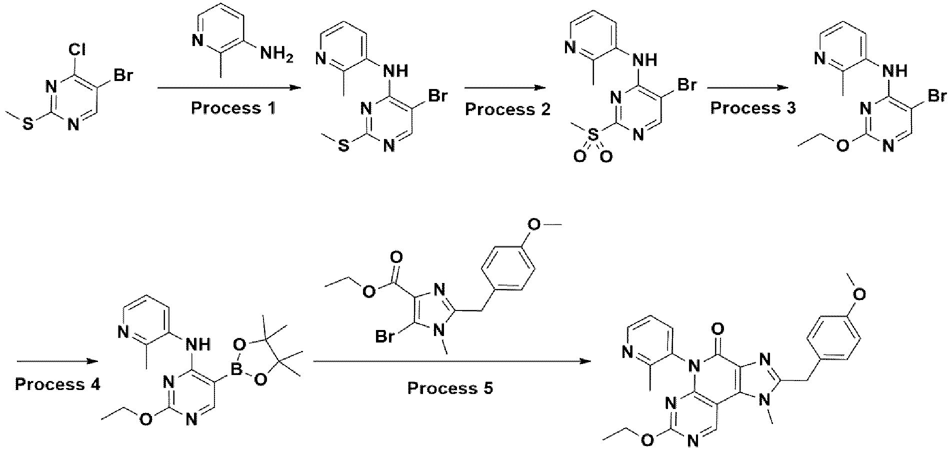

### [Process 1] Preparation of 5-bromo-N-(2-methylpyridin-3-yl)-2-(methylthio)pyrimidin-4-amine

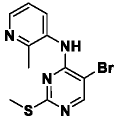

2-methylpyridin-3-amine (994 mg, 9.00 mmol) was dissolved in tetrahydrofuran (14 mL) and cooled to 0 °C. At the same temperature, sodium hydride (720 mg, 18.00 mmol) was added and stirred at room temperature for 30 minutes. The reaction solution was cooled to 0 °C and a tetrahydrofuran solution of 5-bromo-4-chloro-2-(methylthio)pyrimidine (2 g, 8.18 mmol) (6 mL) was added dropwise and refluxed at 55 °C for 15 hours. After completion of the reaction, the mixture was cooled to room temperature, followed by adding water and the resulting solid was collected by filtration, washed with excess water. The obtained residue was purified by silica gel column chromatography to obtain the title compound (1.0 g, 38 %).

### [Process 2] Preparation of 5-bromo-N-(2-methylpyridin-3-yl)-2-(methylsulfonyl)pyrimidin-4-amine

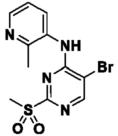

5-bromo-*N*-(2-methylpyridin-3-yl)-2-(methylthio)pyrimidin-4-amine (500 mg, 1.61 mmol) synthesized in [Process 1] above was added to dichloromethane (5 mL) and cooled to 0 °C. 3-chloroperoxybenzoic acid (1.19 g, 4.82 mmol) was added slowly and stirred at room temperature for 1 hour. After completion of the reaction, a saturated aqueous solution of sodium hydrogen carbonate was added and was subjected to extraction with dichloromethane and methanol, the separated organic layer was dried with anhydrous magnesium sulfate, and vacuum distilled to obtain the title compound (395 mg, 71.69 %).

### [Process 3] Preparation of 5-bromo-2-ethoxy-N-(2-methylpyridin-3-yl)pyrimidin-4-amine

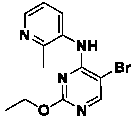

5-bromo-*N*-(2-methylpyridin-3-yl)-2-(methylsulfonyl)pyrimidin-4-amine (395 mg, 1.15 mmol) synthesized in [Process 2] above was dissolved in tetrahydrofuran (11.5 mL) and then a 21 wt% sodium ethoxide ethanol solution (447 mg, 1.38 mmol) was added dropwise. The mixed solution was stirred at room temperature for 2 hours. After completion of the reaction, the mixed solution was vacuum distilled and the obtained residue was purified by silica gel column chromatography to obtain the title compound (245 mg, 68.8 %).

### [Process 4] Preparation of 2-ethoxy-N-(2-methylpyridin-3-yl)-5-(4,4,5,5-tetramethyl-1,3,2-dioxaborolan-2-yl)pyrimidin-4-amine

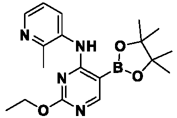

The same reaction was carried out using 5-bromo-*N*-(2-methylpyridin-3-yl)-2-(trifluoromethyl)pyrimidin-4-amine instead of 5-bromo-2-ethoxy-*N*-(2-methylpyridin-3-yl)pyrimidin-4-amine, in [Process 3] of Example 61, to obtain the title compound (57 mg, 20.19 %).

### [Process 5] Preparation of 7-ethoxy-2-(4-methoxybenzyl)-1-methyl-5-(2-methylpyridin-3-yl)-1,5-dihydro-4H-imidazo[4',5':4,5]pyrido[2,3-d]pyrimidin-4-one

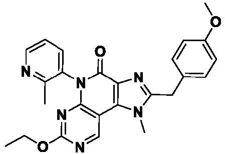

The same reaction was carried out using 2-ethoxy-*N*-(2-methylpyridin-3-yl)-5-(4,4,5,5-tetramethyl-1,3,2-dioxaborolan-2-yl)pyrimidin-4-amine and ethyl 5-bromo-2-(4-methoxybenzyl)-1-methyl-1*H*-imidazole-4-carboxylate obtained from [Intermediate 6-2] instead of *N*-(2-methylpyridin-3-yl)-5-(4,4,5,5-tetramethyl-1,3,2-dioxaborolan-2-yl)-2-(trifluoromethyl)pyrimidin-4-amine and ethyl 5-bromo-1-methyl-1*H*-imidazole-4-carboxylate, in [Process 4] of Example 61, to obtain the title compound (12.5 mg, 17.11 %).

### <Synthesis Example 9>

### Example 63: 2-(4-(difluoromethoxy)benzyl)-7-methoxy-1-methyl-5-(2-methylpyridin-3-yl)-1,5-dihydro-4H-imidazo[4,5-c][1,8]naphthyridin-4-one

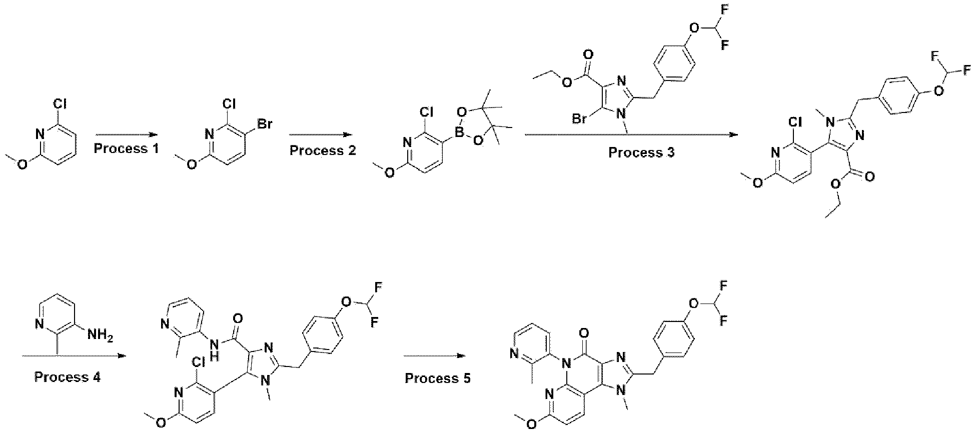

### [Process 1] Preparation of 3-bromo-2-chloro-6-methoxypyridine

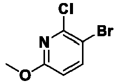

2-chloro-6-methoxypyridine (4.1 mL, 34.1 mmol) and benzoyl peroxide (551 mg, 1.7 mmol) were added to acetonitrile (20 mL). *N*-bromosuccinimide (9.2 g, 51.2 mmol) was added to the above mixture and the reactant was refluxed at 100 °C for 3 hours. The reaction product was cooled to room temperature, followed by adding water then was subjected to extraction with ethyl acetate. The separated organic layer was dried with anhydrous sodium sulfate and concentrated by vacuum distillation. Purification was performed using silica gel column chromatography to obtain the title compound (5.4 g, 71.52 %).

### [Process 2] Preparation of 2-chloro-6-methoxy-3-(4,4,5,5-tetramethyl-1,3,2-dioxaborolan-2-yl)pyridine

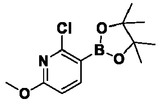

3-bromo-2-chloro-6-methoxypyridine (6.3 g, 28.3 mmol) synthesized in [Process 1] above and bis(pinacolato)diboron (11 g, 42.4 mmol), potassium acetate (5.6 g, 56.5 mmol), [1,1'-bis(diphenylphosphino)ferrocene]dichloropalladium(II) (4.4 g, 5.65 mmol) were added to anhydrous 1,4-dioxane (57 mL), and after nitrogen gas substitution, the mixture was refluxed at 100 °C for 20 hours. After the reaction product was cooled to room temperature, the solid was filtered and washed with dichloromethane:methanol= 10:1 (volume ratio). The filtrate was concentrated and purified by silica gel column chromatography to obtain the title compound (4.5 g, 58.95 %).

### [Process 3] Preparation of ethyl 5-(2-chloro-6-methoxypyridin-3-yl)-2-(4-(difluoromethoxy)benzyl)-1-methyl-1H-imidazole-4-carboxylate

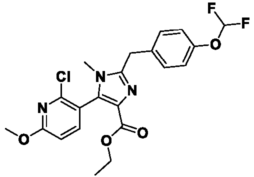

2-chloro-6-methoxy-3-(4,4,5,5-tetramethyl-1,3,2-dioxaborolan-2-yl)pyridine (2.9 g, 10.6 mmol) synthesized in [Process 2] above and ethyl 5-bromo-2-[[4-(difluoromethoxy)phenyl]methyl]-1-methyl-imidazole-4-carboxylate (2.1 g, 5.31 mmol), cesium fluoride (4.2 g, 26.6 mmol), RuPhos (253 mg, 0.53 mmol), RuPhos Pd G3 (468 mg, 0.53 mmol) were added to 1,4-dioxane (20 mL) and water (2 mL), and after nitrogen gas substitution, the mixture was refluxed at 120 °C for 17 hours. After the reaction product was cooled to room temperature, 6 M potassium carbonate solution (2 mL) was added, followed by adding water and was subjected to extraction with ethyl acetate. The separated organic layer was dried with anhydrous sodium sulfate and concentrated by vacuum distillation. The filtrate was concentrated and purified by silica gel column chromatography to obtain the title compound (1.4 g, 58.73 %).

### [Process 4] Preparation of 5-(2-chloro-6-methoxypyridin-3-yl)-2-(4-(difluoromethoxy)benzyl)-1-methyl-N-(2-methylpyridin-3-yl)-1H-imidazole-4-carboxamide

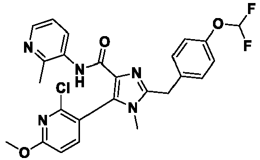

Ethyl 5-(2-chloro-6-methoxypyridin-3-yl)-2-(4-(difluoromethoxy)benzyl)-1-methyl-1H-imidazole-4-carboxylate (1.4 g, 3.12 mmol) and 3-aminopicolin (379 mg, 3.43 mmol) synthesized in [Process 3] above were added to anhydrous tetrahydrofuran (31 mL) and was fitted with an argon balloon. The reaction solution was cooled to -78 °C and lithium bis(trimethylsilyl)amide 1 M tetrahydrofuran solution (6.9 mL, 6.87 mmol) was slowly added dropwise. The reaction solution was stirred at room temperature for 1 hour, and was subjected to extraction with ethyl acetate by adding water. The separated organic layer was dried with anhydrous sodium sulfate and concentrated by vacuum distillation. Purification was performed using silica gel column chromatography to obtain the title compound (1.46 g, 90.94 %).

### [Process 5] preparation of 2-(4-(difluoromethoxy)benzyl)-7-methoxy-1-methyl-5-(2-methylpyridin-3-yl)-1,5-dihydro-4H-imidazo[4,5-c][1,8]naphthyridin-4-one

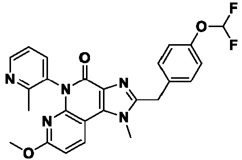

5-(2-chloro-6-methoxypyridin-3-yl)-2-(4-(difluoromethoxy)benzyl)-1-methyl-*N*-(2-methylpyridin-3-yl)-1*H*-imidazole-4-carboxamide (1.3 g, 2.49 mmol) synthesized in [Process 4] above and sodium hydride (199 mg, 4.98 mmol) were added to *N,N-*dimethylformamide (12 mL) at 0 °C, and the reaction solution was stirred at room temperature for 30 minutes. Water and a saturated aqueous solution of ammonium chloride were added to the reaction solution and was subjected to extraction with ethyl acetate. The separated organic layer was dried with anhydrous sodium sulfate and concentrated by vacuum distillation. Purification was performed using silica gel column chromatography to obtain the title compound (633 mg, 53.17 %).

### Example 64: 7-methoxy-2-(4-methoxybenzyl)-1-methyl-5-(2-methylpyridin-3-yl)-1,5-dihydro-4H-imidazo[4,5-c][1,8]naphthyridin-4-one

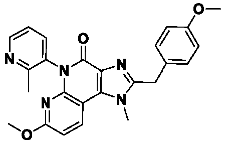

The same reaction was carried out, except for the use of ethyl 5-bromo-2-[[4-(difluoromethoxy)phenyl]methyl]-1-methyl-imidazole-4-carboxylate [Intermediate 6-2 of Synthesis Example 6] instead of ethyl 5-bromo-2-(4-methoxybenzyl)-1-methyl-1*H-*imidazole-4-carboxylate, in Example 63, to obtain the title compound (223 mg, 67.82 %).

### <Synthesis Example 10>

### Example 65: 2-(4-(difluoromethoxy)benzyl)-7-ethyl-1-methyl-5-(2-methylpyridin-3-yl)-1,5-dihydro-4H-imidazo[4,5-c][1,8]naphthyridin-4-one

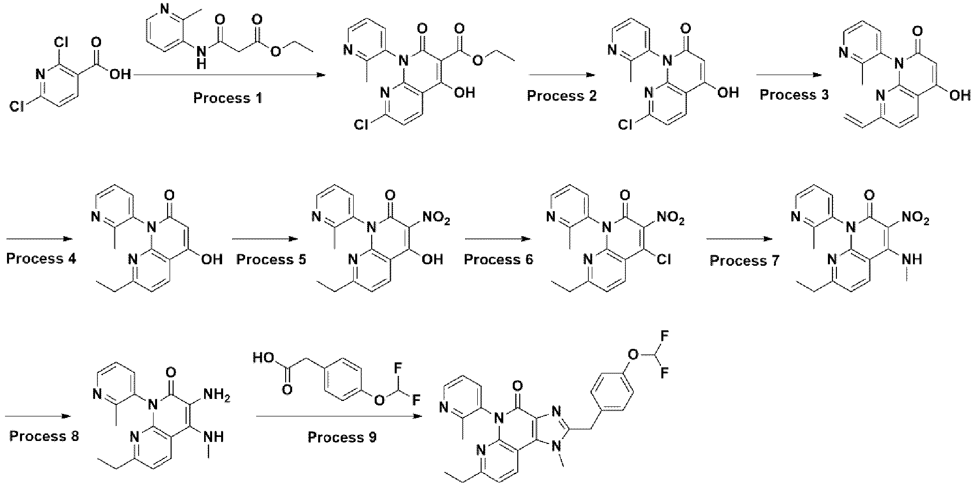

### [Process 1] Preparation of ethyl 7-chloro-4-hydroxy-1-(2-methylpyridin-3-yl)-2-oxo-1,2-dihydro-1,8-naphthyridine-3-carboxylate

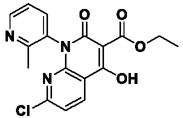

2-3 drops of *N,N*-dimethylformamide were added to the reaction solution of 2,6-dichloronicotinic acid (4.63 g, 23.6 mmol) dissolved in dichloromethane (75 mL), and oxalyl chloride (5.79 mL, 67.50 mmol) was slowly added dropwise, and the mixture was stirred at room temperature for 3 hours. The reaction product was immediately used in the next reaction by removing the solvent by distillation and adding tetrahydrofuran (30 mL). (Reaction solution A).

Ethyl 3-((2-methylpyridin-3-yl)amino)-3-oxopropanoate (5.0 g, 22.50 mmol) was dissolved in tetrahydrofuran (120 mL), and sodium hydride (3.6 g, 90.00 mmol) was slowly added dropwise, stirred at room temperature for 30 minutes and cooled to 0 °C. The Reaction solution A above was added dropwise to the cooled reaction solution, and the temperature was slowly raised to room temperature, stirred for 2 hours, and then stirred and refluxed at 80 °C for 12 hours. The reaction product was cooled to room temperature, followed by adding 3 N aqueous hydrochloric acid solution to adjust the pH to acidic, and was subjected to extraction with ethyl acetate. The extracted organic layer was washed with water, dried with anhydrous sodium sulfate, followed by concentration under decompression. The obtained residue (6.5 g, 80.3 %) was used in the following reaction without a purification procedure.

### [Process 2] Preparation of 7-chloro-4-hydroxy-1-(2-methylpyridin-3-yl)-1,8-naphthyridin-2(1H)-one

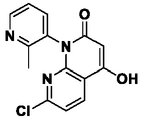

Ethyl 7-chloro-4-hydroxy-1-(2-methylpyridin-3-yl)-2-oxo-1,2-dihydro-1,8-naphthyridin-3-carboxylate (8.94 g, 24.8 mmol) synthesized in [Process 1] was added to trifluoroacetic acid (40 mL)/35 % hydrochloric acid (40 mL) and stirred refluxed at 100 °C for 4 hours. The reaction product was cooled to room temperature, vacuum concentrated, neutralized with a saturated aqueous solution of sodium carbonate (pH=6) and was subjected to extraction with ethyl acetate. The separated organic layer was dried with anhydrous sodium sulfate, filtered and concentrated. Ethyl acetate (50 mL) was again added to the concentrate, and the resulting solid was vacuum filtered to obtain the title compound (3.44 g, 48 %).

### [Process 3] Preparation of 4-hydroxy-1-(2-methylpyridin-3-yl)-7-vinyl-1,8-naphthyridin-2(1H)-one

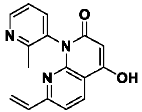

7-chloro-4-hydroxy-1-(2-methylpyridin-3-yl)-1,8-naphthyridin-2(1*H*)-one (900 mg, 3.13 mmol) synthesized in [Process 2] above and tributyl(vinyl)tin (1.0 mL, 3.44 mmol), [1,1'-bis(diphenylphosphino)ferrocene]dichloropalladium(II) dichloromethane complex (264 mg, 0.31 mmol) were added to 1,4-dioxane (31 mL), and after nitrogen gas substitution, the mixture was refluxed at 110 °C for 15 hours. A saturated aqueous solution of potassium fluoride was added to the reaction product, and the mixture was stirred at room temperature for 2 hours. The reaction mixture was filtered through celite and, followed by adding water and was subjected to extraction with ethyl acetate. The separated organic layer was dried with anhydrous sodium sulfate and concentrated by vacuum distillation. Purification was performed using silica gel column chromatography to obtain the title compound (358 mg, 40.94 %).

### [Process 4] Preparation of 7-ethyl-4-hydroxy-1-(2-methylpyridin-3-yl)-1,8-naphthyridin-2(1H)-one

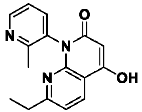

Palladium (Pd/C) (164 mg, 0.15 mmol) in 10 % activated carbon, methanol (26 mL) were added to 4-hydroxy-1-(2-methylpyridin-3-yl)-7-vinyl-1,8-naphthyridin-2(1*H*)-one (358 mg, 1.28 mmol) synthesized in [Process 3] above, and was fitted with a hydrogen balloon. The reaction solution was stirred at room temperature for 16 hours, filtered through celite, and purified by silica gel column chromatography to obtain the title compound (224 mg, 62.09 %).

### [Process 5] Preparation of 7-ethyl-4-hydroxy-1-(2-methylpyridin-3-yl)-3-nitro-1,8-naphthyridin-2(1H)-one

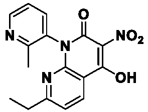

7-ethyl-4-hydroxy-1-(2-methylpyridin-3-yl)-1,8-naphthyridin-2(1*H*)-one (606 mg, 2.15 mmol) synthesized in [Process 4] above and sodium nitrite (30 mg, 0.43 mmol) were added to acetic acid (4 mL), and then fuming nitric acid (0.85 mL, 18.3 mmol) was slowly added dropwise to the reaction solution. The mixture was stirred at 50 °C for 20 minutes, cooled to room temperature, poured into ice water and stirred for 30 minutes. The resulting solid was collected by filtration, washed with water, and dried to obtain the title compound (340 mg, 48.35 %).

### [Process 6] Preparation of 4-chloro-7-ethyl-1-(2-methylpyridin-3-yl)-3-nitro-1,8-naphthyridin-2(1H)-one

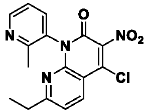

7-ethyl-4-hydroxy-1-(2-methylpyridin-3-yl)-3-nitro-1,8-naphthyridin-2(1*H*)-one (347 mg, 1.06 mmol) synthesized in [Process 5] above, phosphoryl chloride (0.61 mL, 6.38 mmol) and *N,N*-diisopropylethylamine (0.56 mL, 3.19 mmol) were added to toluene (11 mL) and the reaction solution was stirred at 100 °C for 2 hours. The reaction product was concentrated by vacuum distillation and dried to obtain the title compound (367 mg, 99.99 %).

### [Process 7] Preparation of 7-ethyl-4-(methylamino)-1-(2-methylpyridin-3-yl)-3-nitro-1,8-naphthyridin-2(1H)-one

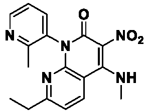

4-chloro-7-ethyl-1-(2-methylpyridin-3-yl)-3-nitro-1,8-naphthyridin-2(1*H*)-one (367 mg, 1.06 mmol) synthesized in [Process 6] above, methylamine hydrochloride (87.97 mg, 1.28 mmol) and *N,N*-diisopropylethylamine (1.68 mL, 9.58 mmol) were added to dichloromethane (11 mL), and the reaction solution was stirred at room temperature for 16 hours. After drying the reaction product and adding water, the resulting solid was collected by filtration, washed with water, and dried to obtain the title compound (330 mg, 91.26 %).

### [Process 8] Preparation of 3-amino-7-ethyl-4-(methylamino)-1-(2-methylpyridin-3-yl)-1,8-naphthyridin-2(1H)-one

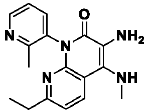

Raney-nickel (114 mg, 0.97 mmol), dichloromethane (9 mL)/methanol (3 mL) was added to 7-ethyl-4-(methylamino)-1-(2-methylpyridin-3-yl)-3-nitro-1,8-naphthyridin-2(1*H*)-one (330 mg, 0.97 mmol) synthesized in [Process 7] above, and was fitted with a hydrogen balloon. The reaction solution was stirred at room temperature for 16 hours, filtered through celite, and purified by silica gel column chromatography to obtain the title compound (228 mg, 75.74 %).

### [Process 9] Preparation of 2-(4-(difluoromethoxy)benzyl)-7-ethyl-1-methyl-5-(2-methylpyridin-3-yl)-1,5-dihydro-4H-imidazo[4,5-c][1,8]naphthyridin-4-one

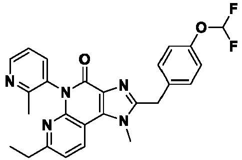

3-amino-7-ethyl-4-(methylamino)-1-(2-methylpyridin-3-yl)-1,8-naphthyridin-2(1H)-one (134 mg, 0.43 mmol) synthesized in [Process 8] above, 2-[4-(difluoromethoxy)phenyl]acetic acid (87.6 mg, 0.43 mmol), 1-[bis(dimethylamino)methylene]-1*H*-1,2,3-triazolo[4,5-b]pyridinium 3-oxide hexafluorophosphate (HATU) (181 mg, 0.48 mmol) and triethylamine (0.18 mL, 1.30 mmol) were sequentially added to *N,N*-dimethylformamide (4.3 mL) and the reaction solution was stirred at room temperature for 1 hour. The reaction solution was subjected to extraction with ethyl acetate with the addition of water. The separated organic layer was dried with anhydrous sodium sulfate and concentrated by vacuum distillation. Acetic acid (1 mL) was added to the concentrated mixture, and the reaction solution was stirred at 100 °C for 16 hours. The reaction product was dried by vacuum distillation and purified by silica gel column chromatography to obtain the title compound (164 mg, 79.48 %).

### Example 66: 7-cyclopropyl-2-(4-(difluoromethoxy)benzyl)-1-methyl-5-(2-methylpyridin-3-yl)-1,5-dihydro-4H-imidazo[4,5-c][1,8]naphthyridin-4-one

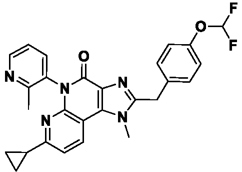

The same reaction was carried out, using 3-amino-7-cyclopropyl-4-(methylamino)-1-(2-methylpyridin-3-yl)-1,8-naphthyridin-2(1*H*)-one and 2-(4-(difluoromethoxy)phenyl)acetic acid instead of 3-amino-7-ethyl-4-(methylamino)-1-(2-methylpyridin-3-yl)-1,8-naphthyridin-2(1*H*)-one and 2-(4-methoxyphenyl)acetic acid, in [Process 9] of Example 65, to obtain the title compound (184 mg, 52.28 %).

According to an embodiment of the present disclosure, the compound of Formula 1 may be a compound selected from the group consisting of the compounds listed in [Table 5] below.

**[Table 5]**

| Compound No. | Structure | Nomenclature | MS[M+H]⁺ |
|---|---|---|---|
| | ¹H-NMR spectrum (300MHz) | | |
| 56 | 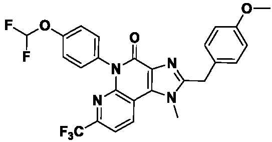 | 5-(4-(Difluoromethoxy)phenyl)-2-(4-methoxybenzyl)-1-methyl-7-(trifluoromethyl)-1,5-dihydro-4*H-*imidazo[4,5-*c*][1,8]naphthyridin-4-one | 531.14 |
| | ¹H-NMR spectrum (300 MHz, CDCl₃) *δ* 8.40(d, 1H), 7.53(d, 1H), 7.27-7.26(m, 4H), 7.18(d, 2H), 6.86(d, 2H), 6.61(t, 1H), 4.37(s, 2H), 3.97(s, 3H), 3.79(s, 3H) | | |
| 57 | 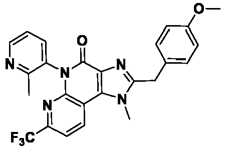 | 2-(4-methoxybenzyl)-1-methyl-5-(2-methylpyridin-3-yl)-7-(trifluoromethyl)-1,5-dihydro-4*H*-imidazo[4,5-*c*][1,8]naphthyridin-4-one | 480.16 |
| | ¹H-NMR spectrum (300 MHz, CDCl₃) *δ* 8.60(dd, 1H), 8.44(d, 1H), 7.54(d, 1H), 7.48(dd, 1H), 7.32-28(m, 1H), 7.17(d, 2H), 6.84(d, 2H), 4.35(s, 2H), 3.98(s, 3H), 3.76(s, 3H), 2.20(s, 3H) | | |
| 58 | 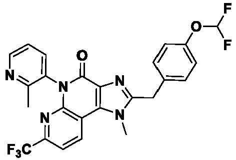 | 2-(4-(Difluoromethoxy)benzyl)-1-methyl-5-(2-methylpyridin-3-yl)-7-(trifluoromethyl)-1,5-dihydro-4*H-*imidazo[4,5-*c*][1,8]naphthyridin-4-one | 516.14 |
| | ¹H-NMR spectrum (300 MHz, CDCl₃) *δ* 8.64(dd, 1H), 8.44(d, 1H), 7.57(d, 1H), 7.50(dd, 1H), 7.33(dd, 1H), 7.30(d, 2H), 7.11(d, 2H), 6.50(t, 1H), 4.43(s, 2H), 4.01(s, 3H), 2.23(s, 3H) | | |
| 59 | 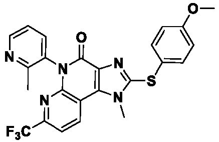 | 2-((4-methoxyphenyl)thio)-1-methyl-5-(2-methylpyridin-3-yl)-7-(trifluoromethyl)-1,5-dihydro-4*H-*imidazo[4,5-*c*][1,8]naphthyridin-4-one | 498.11 |
| | ¹H-NMR spectrum (300 MHz, CDCl₃) *δ* 8.62(dd, 1H), 8.49(d, 1H), 7.58(d, 1H), 7.52(td, 2H), 7.46(dd, 1H), 7.31 (dd, 1H), 6.89(td, 2H), 4.22(s, 3H), 3.80(s, 3H), 2.20(s, 3H) | | |
| 60 | 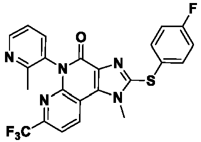 | 2-((4-fluorophenyl)thio)-1-methyl-5-(2-methylpyridin-3-yl)-7-(trifluoromethyl)-1,5-dihydro-4*H-*imidazo[4,5-*c*][1,8]naphthyridin-4-one | 486.09 |
| | ¹H-NMR spectrum (300 MHz, CDCl₃) *δ* 8.62(d, 1H), 8.51(d, 1H), 7.61-7.53(m, 3H), 7.46(dd, 1H), 7.32(dd, 1H), 7.10-7.04(m, 2H), 4.24(s, 3H), 2.21(s, 3H) | | |
| 61 | 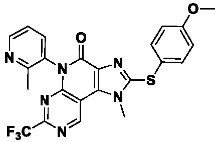 | 2-((4-methoxyphenyl)thio)-1-methyl-5-(2-methylpyridin-3-yl)-7-(trifluoromethyl)-1,5-dihydro-4*H-*imidazo[4',5':4,5]pyrido[2,3-*d*]pyrimidin-4-one | 499.11 |
| | ¹H-NMR spectrum (300 MHz, CDCl₃) *δ* 9.44(s, 1H), 8.66(dd, 1H), 7.53(td, 2H), 7.44(dd, 1H), 7.34(dd, 1H), 6.90(td, 2H), 4.23(s, 3H), 3.80(s, 3H), 2.22(s, 3H) | | |
| 62 | 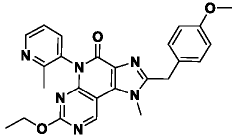 | 7-ethoxy-2-(4-methoxybenzyl)-1-methyl-5-(2-methylpyridin-3-yl)-1,5-dihydro-4*H-*imidazo[4',5':4,5]pyrido[2,3-*d*]pyrimidin-4-one | 457.19 |
| | ¹H-NMR spectrum (300 MHz, CDCl₃) *δ* 9.09(s, 1H), 8.63(dd, 1H), 7.49(dd, 1H), 7.33(dd, 1H), 7.20(d, 2H), 6.87(d, 2H), 4.34(s, 2H), 4.04(dd, 2H), 3.93(s, 3H), 3.79(s, 3H), 2.28(s, 3H), 1.21(t, 3H) | | |
| 63 | 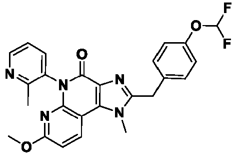 | 2-(4-(difluoromethoxy)benzyl)-7-methoxy-1-methyl-5-(2-methylpyridin-3-yl)-1,5-dihydro-4*H*-imidazo[4,5-*c*][1,8]naphthyridin-4-one | 478.16 |
| | ¹H-NMR spectrum (300 MHz, CDCl₃) *δ* 8.45(d, 1H), 8.18(d, 1H), 7.42(d, 1 H), 7.21-7.18(m, 3H), 6.97(d, 2H), 6.58(d, 1H), 6.42(t, 1H), 4.26(s, 2H), 3.84(s, 3H), 3.37(s, 3H), 2.17(s, 3H) | | |
| 64 | 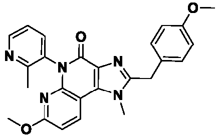 | 7-methoxy-2-(4-methoxybenzyl)-1-methyl-5-(2-methylpyridin-3-yl)-1,5-dihydro-4*H*-imidazo[4,5-*c*][1,8]naphthyridin-4-one | 442.18 |
| | ¹H-NMR spectrum (300 MHz, CDCl₃) *δ* 8.57 (d, 1H), 7.53-7.45(m, 2H), 7.33-7.28(d, 1H), 7.20(d, 2H), 6.85(d, 2H), 6.65(d, 1H), 4.34(s, 2H), 3.90(s, 3H), 3.79(s, 3H), 3.45(s, 3H), 2.27(s, 3H) | | |
| 65 | 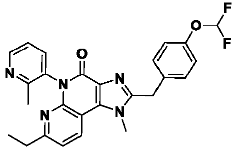 | 2-(4-(difluoromethoxy)benzyl)-7-ethyl-1-methyl-5-(2-methylpyridin-3-yl)-1,5-dihydro-4*H*-imidazo[4,5-*c*][1,8]naphthyridin-4-one | 476.18 |
| | ¹H-NMR spectrum (300 MHz, CDCl₃) *δ* 8.55(d, 1H), 8.20(d, 1H), 7.47(dd, 1H), 7.29-7.24(m, 3H), 7.05(d, 3H), 6.47(t, 1H), 4.35(s, 2H), 3.93(s, 3H), 2.62(q, 2H), 2.19(s, 3H), 1.01(t, 3H) | | |
| 66 | 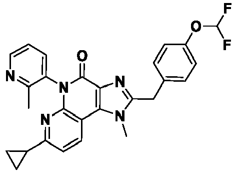 | 7-cyclopropyl-2-(4-(difluoromethoxy)benzyl)-1-methyl-5-(2-methylpyridin-3-yl)-1,5-dihydro-4*H-*imidazo[4,5-*c*][1,8]naphthyridin-4-one | 488.18 |
| | ¹H-NMR spectrum (300 MHz, CDCl₃) *δ* 8.60(dd, 1H), 8.11(d, 1H), 7.45(dd, 1H), 7.30(m, 3H), 7.10(m, 3H), 6.49(t, 1H), 4.38(s, 2H), 3.94(s, 3H), 2.20(s, 3H), 1.89(m, 1H), 0.80(m, 2H), 0.56(m, 2H) | | |

### Experimental Example 1: Cell growth inhibition test

The synthetic compounds above were tested for cell growth inhibition against HCT116 cells.

HCT116 MTAP-/- (homozygous knockout of MTAP gene in HCT116 MTAP-WT cells by CRISPER/Cas9 method) cells were maintained in RPMI1640 medium containing 10 % FBS and 1 % penicillin-streptomycin. Approximately 5×10² cells were seeded in a 96-well plate and treated with a test compound. (0.64 nM - 2,000 nM, 6 dose points for HCT116 MTAP-/- cells for 5x and 6 days) cell growth was measured by SRB staining and absorbance was detected at 540 nm on a Synergy^{™} NEO microplate reader. The 50 % growth inhibition value (GI₅₀) of the cell line was calculated using GraphPad Prism V9 software.

The results of Experimental Example 1 are shown in [Table 6] below.

GI₅₀ values less than 100 nM were labeled as A, at least 100 nM but less than 500 nM were labeled as B, at least 500 nM but less than 1 µM were labeled as C, and 1 µM or more were labeled as D.

**[Table 6]**

| Compound No. | HCT116 MTAP-/- cell growth inhibition (GI₅₀, nM) |
|---|---|
| 1 | B |
| 2 | A |
| 3 | D |
| 4 | A |
| 5 | B |
| 6 | B |
| 7 | D |
| 8 | B |
| 9 | D |
| 10 | A |
| 11 | C |
| 12 | D |
| 15 | D |
| 16 | D |
| 17 | D |
| 18 | A |
| 19 | C |
| 20 | C |
| 21 | A |
| 22 | C |
| 23 | B |
| 24 | B |
| 25 | B |
| 26 | B |
| 27 | A |
| 28 | A |
| 29 | A |
| 30 | A |
| 31 | A |
| 32 | D |
| 33 | A |
| 34 | D |
| 35 | A |
| 36 | A |
| 37 | A |
| 38 | A |
| 39 | A |
| 40 | D |
| 41 | D |
| 42 | D |
| 43 | D |
| 44 | A |
| 45 | A |
| 46 | A |
| 47 | A |
| 48 | B |
| 49 | B |
| 50 | D |
| 51 | A |
| 52 | C |
| 53 | A |
| 54 | A |
| 55 | A |
| 56 | D |
| 57 | A |
| 58 | A |
| 59 | A |
| 60 | B |
| 61 | A |
| 62 | D |
| 63 | A |
| 64 | A |
| 65 | A |
| 66 | A |

So far, the present disclosure has been examined focusing on its specific embodiments. A person skilled in the art to which the present disclosure pertains may understand that the present disclosure may be implemented in a modified form without departing from the essential characteristics of the present disclosure. Therefore, the disclosed embodiments should be considered from an illustrative perspective rather than a limiting one. The scope of the present disclosure is indicated in the claims rather than the foregoing description, and all differences within the equivalent scope should be construed as being included in the present disclosure.

## Claims

1. A compound selected from compounds of Formula 1 below and enantiomers, diastereomers, solvates and hydrates thereof, and pharmaceutically acceptable salts thereof: 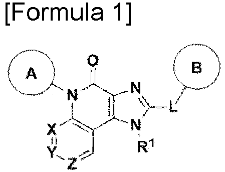
wherein, in Formula 1 above,
X and Z are each independently N or CH;
Y is CR³;
R³ is H, alkyl, alkenyl, alkynyl, alkoxy, alkylthio, alkylsulfonyl, hydroxy, halogen, haloalkyl, haloalkoxy, cycloalkyl, cyano, amino, alkylamino, haloalkylamino, dialkylamino, alkylcarbonyl, alkoxycarbonyl, aminocarbonyl, alkylaminocarbonyl, dialkylaminocarbonyl, hydroxyalkyl, hydroxyalkoxy, hydroxyalkylamino, alkoxyalkyl, alkoxyalkoxy, alkoxyalkylamino, aminoalkyl, aminoalkoxy, aminoalkylamino, aryl, heteroaryl, heteroaryloxy, heteroarylamino, heterocyclyl, heterocyclyloxy, heterocyclylamino, heterocyclyloxyalkoxy, or heterocyclyloxyalkylamino,
wherein each of the aryl, heteroaryl, or heterocyclyl is
unsubstituted or substituted with 1 to 3 members selected from alkyl, cycloalkyl, haloalkyl, haloalkoxy, alkoxy, hydroxy, halo, cyano, hydroxyalkyl, alkoxyalkyl, and aminoalkyl; 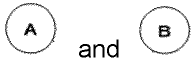 are each independently H, cycloalkyl, aryl, or heteroaryl, heterocyclyl,
each of the cycloalkyl, aryl, heteroaryl, or heterocyclyl is monocyclic, bicyclic, or polycyclic,
wherein the cycloalkyl, aryl, heteroaryl, or heterocyclyl is unsubstituted or substituted with 1 to 3 members independently selected from R² below;
L is C₁₋₃alkylene, O, NH, N(C₁₋₃alkyl), S, SO, SO₂, or a bond;
R² is alkyl, cycloalkyl, haloalkyl, haloalkoxy, alkoxy, hydroxy, alkylsulfonyl, halogen, cyano, carboxy, alkoxycarbonyl, hydroxyalkyl, alkoxyalkyl, aminoalkyl, aminosulfonyl, alkylaminosulfonyl, dialkylaminosulfonyl, sulfonylamino, aminocarbonyl, alkylaminocarbonyl, dialkylaminocarbonyl, heterocyclylcarbonyl, or ureido; and
R¹ is H, alkyl, haloalkyl, hydroxyalkyl, alkoxyalkyl, aminoalkyl, substituted aminoalkyl, aminocarbonylalkyl, or aminosulfonylalkyl.

2. The compound of claim 1, wherein 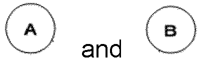 are each independently H, C₃₋₁₀cycloalkyl, bridged C₈₋₁₆cycloalkyl, fused C₃₋₁₀cycloalkyl, C₈₋₁₆spirocycloalkyl, C₆₋₁₀aryl, C₄₋₁₀heteroaryl, C₁₋₁₀heterocyclyl, bridged C₆₋₁₄heterocyclyl, fused C₁₋₁₀heterocyclyl, or C₆₋₁₄spiroheterocyclyl.

3. The compound of claim 1, wherein
X and Z are each independently N or CH;
Y is CR^{3a};
R^{3a} is H, C₁₋₆alkyl, C₂₋₆alkenyl, C₂₋₆alkynyl, C₁₋₆alkoxy, C₁₋₆alkylthio, C₁₋₆alkylsulfonyl, hydroxy, halogen, haloC₁₋₆alkyl, haloC₁₋₆alkoxy, C₃₋₁₀cycloalkyl, cyano, amino, C₁₋₆alkylamino, haloC₁₋₆alkylamino, C₂₋₁₂dialkylamino, C₁₋₆alkylcarbonyl, C₁₋₆alkoxycarbonyl, aminocarbonyl, C₁₋₆alkylaminocarbonyl, C₂₋₁₂dialkylaminocarbonyl, hydroxyC₁₋₆alkyl, hydroxyC₁₋₆alkoxy, hydroxyC₁₋₆alkylamino, C₁₋₆alkoxyC₁₋₆alkyl, C₁₋₆alkoxyC₁₋₆alkoxy, C₁₋₆alkoxyC₁₋₆alkylamino, aminoC₁₋₆alkyl, aminoC₁₋₆alkoxy, aminoC₁₋₆alkylamino, C₆₋₁₀aryl, C₅₋₁₀heteroaryl, C₅₋₁₀heteroaryloxy, C₄₋₁₀heteroarylamino, C₁₋₁₀heterocyclyl, C₁₋₁₀heterocyclyloxy, C₁₋₁₀heterocyclylamino, C₁₋₁₀heterocyclyloxyalkoxy, or C₁₋₁₀heterocyclyloxyalkylamino,
wherein each of C₆₋₁₀aryl, C₄₋₁₀heteroaryl, or C₁₋₁₀heterocyclyl is
unsubstituted or substituted with 1 to 3 members selected from C₁₋₆alkyl, C₃₋₁₀cycloalkyl, haloC₁₋₆alkyl, haloC₁₋₆alkoxy, C₁₋₆alkoxy, hydroxy, halogen, cyano, hydroxyC₁₋₆alkyl, C₁₋₆alkoxyC₁₋₆alkyl, and aminoC₁₋₆alkyl; 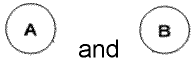
are each independently H, C₃₋₁₀ cycloalkyl, bridged C₈₋₁₆ cycloalkyl, fused C₃₋₁₀ cycloalkyl, C₈₋₁₆ spirocycloalkyl, C₆₋₁₀ aryl, C₄₋₁₀ heteroaryl, C₁₋₁₀ heterocyclyl, bridged C₆₋₁₄ heterocyclyl, fused C₁₋₁₀heterocyclyl, or C₆₋₁₄spiroheterocyclyl, wherein C₆₋₁₀aryl, C₄₋₁₀heteroaryl, or C₁₋₁₀heterocyclyl is unsubstituted or substituted with 1 to 3 members independently selected from R^{2a} below;
L is C₁₋₃alkylene, O, NH, NCH₃, S, SO, SO₂, or a bond;
R^{2a} is C₁₋₆alkyl, C₃₋₁₀cycloalkyl, haloC₁₋₆alkyl, haloC₁₋₆alkylamino, haloC₁₋₆alkoxy, C₁₋₆alkoxy, hydroxy, C₁₋₆alkylsulfonyl, halogen, cyano, carboxy, C₁₋₆alkoxycarbonyl, hydroxyC₁₋₆alkyl, C₁₋₆alkoxyalkyl, aminoC₁₋₆alkyl, aminosulfonyl, C₁₋₆alkylaminosulfonyl, (C₁₋₆alkyl)₂aminosulfonyl, sulfonylamino, aminocarbonyl, C₁₋₆alkylaminocarbonyl, (C₁₋₆alkyl)₂aminocarbonyl, C₁₋₁₀heterocyclylcarbonyl, or ureido; and
R' is H, C₁₋₆alkyl, haloC₁₋₆alkyl, hydroxyC₁₋₆alkyl, C₁₋₆alkoxyalkyl, aminoC₁₋₆alkyl, substituted aminoC₁₋₆alkyl, aminocarbonylC₁₋₆alkyl, or aminosulfonylC₁₋₆alkyl.

4. The compound of claim 1, wherein
X and Z are each independently N or CH;
Y is CR^{3b};
R^{3b} is H, C₂₋₄alkenyl, C₂₋₄alkynyl, C₁₋₃alkoxy, hydroxy, halogen, haloC₁₋₆alkyl, C₃₋₆cycloalkyl, amino, C₁₋₃alkylamino, haloC₁₋₆alkylamino, or C₂₋₆heterocyclyl; 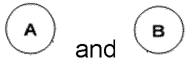
are each independently H, C₆₋₁₀aryl, C₄₋₁₀heteroaryl, or C₁₋₁₀heterocyclyl, wherein C₆₋₁₀aryl, C₄₋₁₀heteroaryl, or fused C₁₋₁₀heterocyclyl is unsubstituted or substituted with 1 to 3 members independently selected from R^{2b} below;
L is C₁₋₆alkylene, O, NH, NCH₃, S, SO, SO₂, or a bond;
R^{2b} is C₁₋₆alkyl, haloC₁₋₃alkyl, haloC₁₋₃alkoxy, C₁₋₆alkoxy, hydroxy, or halogen; and
R' is H or C₁₋₆alkyl.

5. The compound of claim 1, wherein
X and Z are each independently N or CH;
Y is CR^{3c};
R^{3c} is H, C₂₋₄alkenyl, C₂₋₄alkynyl, C₁₋₃alkoxy, hydroxy, halogen, haloC₁₋₃alkyl, C₃₋₆cycloalkyl, amino, C₁₋₃alkylamino, haloC₁₋₃alkylamino, or C₂₋₆heterocyclyl; 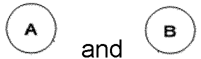 are each independently H, phenyl, pyridinyl, imidazolyl, pyrrolidinyl, indolyl, naphthalenyl, benzodioxynyl, or benzodioxolyl, wherein 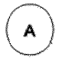 and 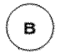 are unsubstituted or substituted with 1 to 3 members independently selected from C₁₋₃alkyl, haloC₁₋₃alkyl, haloC₁₋₃alkoxy, C₁₋₃alkoxy, hydroxy, and halogen;
L is C₁₋₃alkylene, O, NH, NCH₃, S, SO, SO₂, or a bond; and
R' is H or C₁₋₃alkyl.

6. The compound of claim 1, wherein
X and Z are each independently N or CH;
Y is CR^{3c}, wherein R^{3c} is H, C₂₋₄alkenyl, C₂₋₄alkynyl, C₁₋₃alkoxy, hydroxy, halogen, haloC₁₋₃alkyl, C₃₋₆cycloalkyl, amino, C₁₋₃alkylamino, haloC₁₋₃alkylamino, or C₂₋₆heterocyclyl; 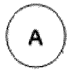
is phenyl or pyridinyl; 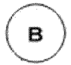
is H, phenyl, pyridinyl, imidazolyl, pyrrolidinyl, or 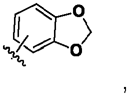
wherein 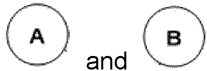 are each independently
unsubstituted or substituted with 1 to 3 members independently selected from C₁₋₃alkyl, haloC₁₋₃alkyl, haloC₁₋₃alkoxy, C₁₋₃alkoxy, hydroxy, and halogen;
L is methylene, ethylene, O, NH, NCH₃, S, SO, or SO₂; and
R' is H or methyl.

7. The compound of claim 1, wherein the compound is selected from the group consisting of the following compounds and enantiomers, diastereomers, solvates and hydrates thereof, and pharmaceutically acceptable salts thereof:
1) 2-benzyl-7-chloro-1-methyl-5-phenyl-1,5-dihydro-4*H*-imidazo[4,5-*c*]quinolin-4-one
2) 7-chloro-2-(4-methoxybenzyl)-1-methyl-5-phenyl-1,5-dihydro-4*H*-imidazo[4,5-*c*]quinolin-4-one;
3) 7-chloro-2-(4-hydroxybenzyl)-1-methyl-5-phenyl-1,5-dihydro-4*H*-imidazo[4,5-c*]*quinolin-4-one;
4) 7-chloro-2-(3-methoxybenzyl)-1-methyl-5-phenyl-1,5-dihydro-4*H*-imidazo[4,5-*c*]quinolin-4-one;
5) 7-chloro-2-(2-methoxybenzyl)-1-methyl-5-phenyl-1,5-dihydro-4*H*-imidazo[4,5-*c*]quinolin-4-one;
6) 7-chloro-2-(4-chlorobenzyl)-1-methyl-5-phenyl-1,5-dihydro-4*H*-imidazo[4,5-*c*]quinolin-4-one;
7) 7-chloro-1-methyl-5-phenyl-2-(pyridin-4-ylmethyl)-1,5-dihydro-4*H*-imidazo[4,5-*c*]quinolin-4-one;
8) 7-chloro-2-(3,4-dimethoxybenzyl)-1-methyl-5-phenyl-1,5-dihydro-4*H-*imidazo[4,5-*c*]quinolin-4-one;
9) 7-chloro-1-methyl-2-phenethyl-5-phenyl-1,5-dihydro-4*H*-imidazo[4,5-*c*]quinolin-4-one;
10) 7-chloro-2-(4-methoxyphenyl)-1-methyl-5-(o-tolyl)-1,5-dihydro-4*H-*imidazo[4,5-*c*]quinolin-4-one;
11) 2-benzyl-7-chloro-5-phenyl-1,5-dihydro-4*H*-imidazo[4,5-*c*]quinolin-4-one;
12) 2-((1*H*-imidazol-1-yl)methyl)-7-chloro-1-methyl-5-phenyl-1,5-dihydro-4*H-*imidazo[4,5-*c*]quinolin-4-one;
13) 7-chloro-2-((3-methoxypyrrolidin-1-yl)methyl)-1-methyl-5-phenyl-1,5-dihydro-4*H*-imidazo[4,5-*c*]quinolin-4-one;
14) 7-chloro-2-((3-hydroxypyrrolidin-1-yl)methyl)-1-methyl-5-phenyl-1,5-dihydro-4*H*-imidazo[4,5-*c*]quinolin-4-one;
15) 2-(4-methoxybenzyl)-1-methyl-5-phenyl-7-((2,2,2-trifluoroethyl)amino)-1,5-dihydro-4*H*-imidazo[4,5-*c*][1,8]naphthyridin-4-one;
16) 7-chloro-5-(4-chloro-3-fluorophenyl)-2-(4-methoxybenzyl)-1-methyl-1,5-dihydro-4*H*-imidazo[4,5-*c*]quinolin-4-one;
17) 7-chloro-5-(3-chloro-4-fluorophenyl)-2-(4-methoxybenzyl)-1-methyl-1,5-dihydro-4*H*-imidazo[4,5-*c*]quinolin-4-one;
18) 7-chloro-2-((4-methoxyphenyl)thio)-1-methyl-5-phenyl-1,5-dihydro-4*H-*imidazo[4,5-*c*]quinolin-4-one ;
19) 7-chloro-2-((4-methoxyphenyl)sulfonyl)-1-methyl-5-phenyl-1,5-dihydro-4*H-*imidazo[4,5-*c*]quinolin-4-one;
20) 7-chloro-2-((4-methoxyphenyl)sulfinyl)-1-methyl-5-phenyl-1,5-dihydro-4*H-*imidazo[4,5-*c*]quinolin-4-one;
21) 7-chloro-2-(4-methoxybenzyl)-1-methyl-5-(2-methylpyridin-3-yl)-1,5-dihydro-4*H*-imidazo[4,5-*c*]quinolin-4-one;
22) 2-(4-methoxybenzyl)-1-methyl-5-(2-methylpyridin-3-yl)-1,5-dihydro-4*H-*imidazo[4,5-*c*]quinolin-4-one;
23) 7-chloro-2-(4-methoxybenzyl)-1-methyl-5-(2-(trifluoromethyl)pyridin-3-yl)-1,5-dihydro-4*H*-imidazo[4,5-*c*]quinolin-4-one;
24) 7-chloro-5-(2-cyclopropylpyridin-3-yl)-2-(4-methoxybenzyl)-1-methyl-1,5-dihydro-4*H*-imidazo[4,5-*c*]quinolin-4-one;
25) 7-chloro-2-(4-methoxybenzyl)-1-methyl-5-(2-(trifluoromethyl)phenyl)-1,5-dihydro-4*H*-imidazo[4,5-*c*]quinolin-4-one;
26) 7-chloro-5-(2-chlorophenyl)-2-(4-methoxybenzyl)-1-methyl-1,5-dihydro-4*H-*imidazo[4,5-*c*]quinolin-4-one;
27) 2-(4-methoxybenzyl)-1-methyl-5-(2-methylpyridin-3-yl)-7-(trifluoromethyl)-1,5-dihydro-4*H*-imidazo[4,5-*c*]quinolin-4-one;
28) 7-chloro-2-(4-fluorobenzyl)-1-methyl-5-(2-methylpyridin-3-yl)-1,5-dihydro-4*H-*imidazo[4,5-*c*]quinolin-4-one;
29) 2-(benzo[*d*][1,3]dioxol-5-ylmethyl)-7-chloro-1-methyl-5-(2-methylpyridin-3-yl)-1,5-dihydro-4*H*-imidazo[4,5-*c*]quinolin-4-one;
30) 7-chloro-2-(3,4-difluorobenzyl)-1-methyl-5-(2-methylpyridin-3-yl)-1,5-dihydro-4*H*-imidazo[4,5-*c*]quinolin-4-one;
31) 7-chloro-2-(4-methoxybenzyl)-1-methyl-5-(pyridin-3-yl)-1,5-dihydro-4*H-*imidazo[4,5-*c*]quinolin-4-one;
32) 7-chloro-5-(4-fluorophenyl)-2-(4-methoxybenzyl)-1-methyl-1,5-dihydro-4*H-*imidazo[4,5-*c*]quinolin-4-one;
33) 7-chloro-2-(4-(difluoromethoxy)benzyl)-1-methyl-5-(2-methylpyridin-3-yl)-1,5-dihydro-4*H*-imidazo[4,5-*c*]quinolin-4-one;
34) 7-chloro-5-(4-methoxyphenyl)-2-((4-methoxyphenyl)thio)-1-methyl-1,5-dihydro-4*H*-imidazo[4,5-*c*]quinolin-4-one;
35) 7-chloro-2-((4-methoxyphenyl)thio)-1-methyl-5-(2-methylpyridin-3-yl)-1,5-dihydro-4*H*-imidazo[4,5-*c*]quinolin-4-one;
36) 7-chloro-2-((4-fluorophenyl)thio)-1-methyl-5-(2-methylpyridin-3-yl)-1,5-dihydro-4*H*-imidazo[4,5-*c*]quinolin-4-one;
37) 7-chloro-2-((3,4-difluorophenyl)thio)-1-methyl-5-(2-methylpyridin-3-yl)-1,5-dihydro-4*H*-imidazo[4,5-*c*]quinolin-4-one;
38) 2-(benzo[*d*][1,3]dioxol-5-ylthio)-7-chloro-1-methyl-5-(2-methylpyridin-3-yl)-1,5-dihydro-4*H*-imidazo[4,5-*c*]quinolin-4-one;
39) 7-chloro-2-((2,4-difluorophenyl)thio)-1-methyl-5-(2-methylpyridin-3-yl)-1,5-dihydro-4*H*-imidazo[4,5-*c*]quinolin-4-one;
40) 7-chloro-2-((4-methoxyphenyl)amino)-1-methyl-5-phenyl-1,5-dihydro-4*H-*imidazo[4,5-*c*]quinolin-4-one;
41) 7-chloro-2-((4-methoxyphenyl)(methyl)amino)-1-methyl-5-phenyl-1,5-dihydro-4*H*-imidazo[4,5-*c*]quinolin-4-one;
42) 7-chloro-2-(4-methoxyphenoxy)-1-methyl-5-phenyl-1,5-dihydro-4*H-*imidazo[4,5-*c*]quinolin-4-one;
43) 7-hydroxy-2-(4-methoxybenzyl)-1-methyl-5-(2-methylpyridin-3-yl)-1,5-dihydro-4*H*-imidazo[4,5-*c*]quinolin-4-one;
44) 2-(4-methoxybenzyl)-1-methyl-5-(2-methylpyridin-3-yl)-7-vinyl-1,5-dihydro-4*H*-imidazo[4,5-*c*]quinolin-4-one;
45) 7-ethyl-2-(4-methoxybenzyl)-1-methyl-5-(2-methylpyridin-3-yl)-1,5-dihydro-4*H*-imidazo[4,5-*c*]quinolin-4-one;
46) 7-cyclopropyl-2-(4-methoxybenzyl)-1-methyl-5-(2-methylpyridin-3-yl)-1,5-dihydro-4*H*-imidazo[4,5-*c*]quinolin-4-one;
47) 7-ethynyl-2-(4-methoxybenzyl)-1-methyl-5-(2-methylpyridin-3-yl)-1,5-dihydro-4*H*-imidazo[4,5-*c*]quinolin-4-one;
48) 7-isopropyl-2-(4-methoxybenzyl)-1-methyl-5-(2-methylpyridin-3-yl)-1,5-dihydro-4*H*-imidazo[4,5-*c*]quinolin-4-one;
49) 2-(4-methoxybenzyl)-1,7-dimethyl-5-(2-methylpyridin-3-yl)-1,5-dihydro-4*H-*imidazo[4,5-*c*]quinolin-4-one;
50) 2-(4-methoxybenzyl)-1-methyl-5-(2-methylpyridin-3-yl)-7-(pyrrolidin-1-yl)-1,5-dihydro-4*H*-imidazo[4,5-*c*]quinolin-4-one;
51) 7-methoxy-2-(4-methoxybenzyl)-1-methyl-5-(2-methylpyridin-3-yl)-1,5-dihydro-4*H*-imidazo[4,5-*c*]quinolin-4-one;
52) 7-amino-2-(4-methoxybenzyl)-1-methyl-5-(2-methylpyridin-3-yl)-1,5-dihydro-4*H*-imidazo[4,5-*c*]quinolin-4-one;
53) 2-(4-methoxybenzyl)-1-methyl-7-(methylamino)-5-(2-methylpyridin-3-yl)-1,5-dihydro-4*H*-imidazo[4,5-*c*]quinolin-4-one;
54) 2-(4-(difluoromethoxy)benzyl)-7-ethyl-1-methyl-5-(2-methylpyridin-3-yl)-1,5-dihydro-4*H*-imidazo[4,5-*c*]quinolin-4-one;
55) 2-(4-(difluoromethoxy)benzyl)-7-methoxy-1-methyl-5-(2-methylpyridin-3-yl)-1,5-dihydro-4*H*-imidazo[4,5-*c*]quinolin-4-one;
56) 5-(4-(difluoromethoxy)phenyl)-2-(4-methoxybenzyl)-1-methyl-7-(trifluoromethyl)-1,5-dihydro-4*H*-imidazo[4,5-*c*][1,8]naphthyridin-4-one;
57) 2-(4-methoxybenzyl)-1-methyl-5-(2-methylpyridin-3-yl)-7-(trifluoromethyl)-1,5-dihydro-4*H*-imidazo[4,5-*c*][1,8]naphthyridin-4-one;
58) 2-(4-(difluoromethoxy)benzyl)-1-methyl-5-(2-methylpyridin-3-yl)-7-(trifluoromethyl)-1,5-dihydro-4*H*-imidazo[4,5-*c*][1,8]naphthyridin-4-one;
59) 2-((4-methoxyphenyl)thio)-1-methyl-5-(2-methylpyridin-3-yl)-7-(trifluoromethyl)-1,5-dihydro-4*H*-imidazo[4,5-*c*][1,8]naphthyridin-4-one;
60) 2-((4-fluorophenyl)thio)-1-methyl-5-(2-methylpyridin-3-yl)-7-(trifluoromethyl)-1,5-dihydro-4H-imidazo[4,5-c][1,8]naphthyridin-4-one;
61) 2-((4-methoxyphenyl)thio)-1-methyl-5-(2-methylpyridin-3-yl)-7-(trifluoromethyl)-1,5-dihydro-4*H*-imidazo[4',5':4,5]pyrido[2,3-*d*]pyrimidin-4-one;
62) 7-ethoxy-2-(4-methoxybenzyl)-1-methyl-5-(2-methylpyridin-3-yl)-1,5-dihydro-4*H*-imidazo[4',5':4,5]pyrido[2,3-d]pyrimidin-4-one;
63) 2-(4-(difluoromethoxy)benzyl)-7-methoxy-1-methyl-5-(2-methylpyridin-3-yl)-1,5-dihydro-4*H*-imidazo[4,5-*c*][1,8]naphthyridin-4-one⁻,
64) 7-methoxy-2-(4-methoxybenzyl)-1-methyl-5-(2-methylpyridin-3-yl)-1,5-dihydro-4*H*-imidazo[4,5-*c*][1,8]naphthyridin-4-one⁻,
65) 2-(4-(difluoromethoxy)benzyl)-7-ethyl-1-methyl-5-(2-methylpyridin-3-yl)-1,5-dihydro-4*H*-imidazo[4,5-*c*][1,8]naphthyridin-4-one; and
66) 7-cyclopropyl-2-(4-(difluoromethoxy)benzyl)-1-methyl-5-(2-methylpyridin-3-yl)-1,5-dihydro-4*H*-imidazo[4,5-*c*][1,8]naphthyridin-4-one.

8. A pharmaceutical composition for treating or preventing a methionine adenosyltransferase 2A-related disease or disorder, comprising, as an active ingredient, a compound selected from the group consisting of the compounds of any one of claims 1 to 7 and enantiomers, diastereomers, solvates and hydrates thereof, and pharmaceutically acceptable salts thereof.

9. The pharmaceutical composition of claim 8, wherein the pharmaceutical composition exhibits MATA2A inhibitory activity.

10. The pharmaceutical composition of claim 8, wherein the pharmaceutical composition is for treating a cancer.

11. The pharmaceutical composition of claim 8, wherein the pharmaceutical composition is for treating a methylthioadenosine phosphorylase deficient cancer.
